# EUROPEAN PATENT APPLICATION

(11) **EP 3 660 511 A1**
(43) Date of publication of application: **03.06.2020**
(21) Application number: 19200537.9
(22) Date of filing: 02.01.2014
(51) Int. Cl.: G01N 33/68, G01N 33/53, G01N 33/58

(54) **ASSAY PANELS**

(30) Priority: 03.01.2013 US 201361748626 P
(62) Divisional of application: 14735123.3
(71) Applicant: Meso Scale Technologies, LLC, Rockville, MD 20850 (US)
(72) Inventor: JOERN, John, Rockville, MD 20850 (US); MANIMALA, Joseph, Silver Spring, MD 20902 (US); MCCLARY, Keith, Rockville, MD 20850 (US); OBEROI, Pankaj, Rockville, MD 20850 (US); SPIELES, Gisbert, Bethesda, MD 20816 (US); STEWART, David, Monrovia, MD 21770 (US); WILBUR, James, Germantown, MD 20874 (US)
(74) Representative: Latscha Schöllhorn Partner AG

(57) **Abstract**

Described herein are kits and components thereof used for a multiplexed analysis of a set of cytokines.

## Description

### Cross Reference to Related Applications

The present application claims benefit of U.S. Provisional Application No. 61/748,626 filed on January 3, 2013, the entire contents of which are incorporated herein by reference.

### Field of the Invention

This application relates to kits used for the detection of cytokines using electrochemiluminescent technology.

### Background of the Invention

Cytokines are the soluble factors that mediate acute and chronic inflammatory responses, and are involved in many physiological events from wound healing to autoimmune disorders. They are important regulators of cell-mediated and humoral immune responses and their differential expression has been associated with a wide array of immune disorders. They function on a variety of cell types, having stimulatory or inhibitory effects on proliferation, differentiation, and maturation. Therefore, measuring the level of only a single cytokine in any biological system provides only partial information relevant to the response on a systemic level. Comprehensive tests for cytokine levels generally aim to measure the concentrations of a large set of cytokines to gain a better understanding of the underlying physiology.

The enzyme-linked immunosorbent assay (ELISA) is the most commonly used and reported method for the quantitation of secreted cytokines. However, ELISA can only detect one analyte per reaction in individual assay wells. This leads to high reagent cost, excessive technician time, and the need to use large sample volumes to generate each results. The ability to detect and quantitate many cytokines simultaneously in the same sample via a robust multiplexed assay would reduce costs and improve efficiency. The advantages of multiplex technology over conventional assay methods include simultaneous analyte detection, reduced reagent handling, high assay throughput, and decreased sample and reagent volumes.

### Summary of the Invention

The invention provides a kit for the analysis of a cytokine panel comprising:
i. (a) a multi-well assay plate comprising a plurality of wells, each well comprising ten discrete binding domains to which capture antibodies to the following human analytes are bound: IFN-gamma, IL-1beta, IL-2, IL-4, IL-6, IL-8, IL-10, IL-12p70, IL-13, TNFalpha; (b) in one or more vials, containers, or compartments, a set of labeled detection antibodies specific for said human analytes; and (c) in one or more vials, containers, or compartments, a set of calibrator proteins.
ii. (a) a multi-well assay plate comprising a plurality of wells, each well comprising ten discrete binding domains to which capture antibodies to the following human analytes are bound: GM-CSF, IL-1alpha, IL-5, IL-7, IL-12/IL-23 p40, IL-15, IL-16, IL-17A, TNF-beta, VEGF-A; (b) in one or more vials, containers, or compartments, a set of labeled detection antibodies specific for said human analytes; and (c) in one or more vials, containers, or compartments, a set of calibrator proteins.
iii. (a) a multi-well assay plate comprising a plurality of wells, each well comprising ten discrete binding domains to which capture antibodies to the following human analytes are bound: Eotaxin, MIP-1 alpha,'Eotaxin-3, TARC, IP-10, MIP-1 beta, IL-8, MCP-1, MDC, MCP-4; (b) in one or more vials, containers, or compartments, a set of labeled detection antibodies specific for said human analytes; and (c) in one or more vials, containers, or compartments, a set of calibrator proteins;
iv. (a) a multi-well assay plate comprising a plurality of wells, each well comprising ten discrete binding domains to which capture antibodies to the following rat analytes are bound: IFN-gamma, IL-2, IL-4, IL-1 beta, IL-5, IL-6, KC/GRO, IL-10, IL-13, TNF-alpha; (b) in one or more vials, containers, or compartments, a set of labeled detection antibodies specific for said human analytes; and (c) in one or more vials, containers, or compartments, a set of calibrator proteins; or
v. (a) a multi-well assay plate comprising a plurality of wells, each well comprising ten discrete binding domains to which capture antibodies to the following mouse analytes are bound: IFN-gamma, IL-1-beta, IL-2, IL-4, IL-5, IL-6, KC/GRO, IL-10, IL-12p70, TNF-alpha; (b) in one or more vials, containers, or compartments, a set of labeled detection antibodies specific for said human analytes; and (c) in one or more vials, containers, or compartments, a set of calibrator proteins.

Also provided is a method of manufacturing a kit or a lot of kit such as those described herein that includes: (a) subjecting a preliminary set of detection antibodies specific for said human analytes to CIEF, DLS, and Experion; (b) selecting qualified detection antibodies from said preliminary set of detection antibodies based on said CIEF, DLS, and Experion testing; (c) subjecting a preliminary set of capture antibodies specific for said human analytes to CIEF, DLS, and Experion; and (b) selecting qualified capture antibodies from said preliminary set of capture antibodies based on said CIEF, DLS, and Experion testing. In a preferred embodiment, a lot of kits is manufacturing using this protocol and meets one or more of the following specifications: (a) average intraplate CV of ≤ 10%; (b) maximum intraplate CV of ≤ 13%; (c) average uniformity metric of ≤ 25%; (d) maximum uniformity metric of ≤ 37%; (e) CV of intraplate averages of ≤ 18%; (f) lower signal boundary of > 1500; and (g) upper signal boundary of < 10⁶.

In a preferred embodiment, the invention provides a kit for the analysis of two or more cytokine panels comprising: (a) two or more multi-well assay plates each comprising a plurality of wells, each well comprising ten discrete binding domains to which capture antibodies to a set of analytes are bound, wherein said set of analytes is selected from the group consisting of:
(i) human analytes: IFN-gamma, IL-1beta, IL-2, IL-4, IL-6, IL-8, IL-10, IL-12p70, IL-13, and TNFalpha;
(ii) human analytes: GM-CSF, IL-1alpha, IL-5, IL-7, IL-12/IL-23 p40, IL-15, IL-16, IL-17A, TNF-beta, and VEGF-A;
(iii) human analytes: Eotaxin, MIP-1 alpha, Eotaxin-3, TARC, IP-10, MIP-1 beta, IL-8, MCP-1, MDC, and MCP-4;
(iv) rat analytes: IFN-gamma, IL-2, IL-4, IL-1 beta, IL-5, IL-6, KC/GRO, IL-10, IL-13, and TNF-alpha; or
(v) mouse analytes: IFN-gamma, IL-1-beta, IL-2, IL-4, IL-5, IL-6, KC/GRO, IL-10, IL-12p70, and TNF-alpha;
   (b) in one or more vials, containers, or compartments, a set of labeled detection antibodies specific for said analytes; and (c) in one or more vials, containers, or compartments, a set of calibrator proteins.

An additional embodiment of the invention is a 10-spot 96-well multi-well plate, wherein each plate comprises a plate top, a plate bottom, an x- and y-axis of the plate top and bottom, and each well comprises a spot pattern, wherein the plate meets the following specifications: Δx ≤ 0.2 mm, Δy ≤ 0.2 mm, and α ≤ 0.1°, wherein (a) Δx is the difference between a center of the spot pattern and a center of a well along the x axis of the plate; (b) Δy is the difference between the center of a spot pattern and a center of the well along the y axis of the plate; and (c) α is a counter-clockwise angle between the x axis of the plate bottom and the x axis of the plate top.

Moreover, the invention contemplates a 10-spot 96-well multi-well plate, wherein each plate comprises a plate top, a plate bottom, and each well comprises a spot pattern, wherein the plate meets the following specifications: (a) a length range of 3.8904-3.9004 inches; (b) a width range of 2.4736-2.4836 inches; and (c) well to well spacing of 0.3513-0.3573 inches.

### Brief Description of the Figures

Fig. 1(a)-(c) illustrate a 10-spot pattern in a well of a multi-well plate (panel (a)), its placement in a 96-well 10-spot plate (panel (b)), and the principles of an immunoassay conducted using a multi-well assay plate such as those described herein.
Figs. 2(a)-(e) are standard curves for each of the five cytokine panels.

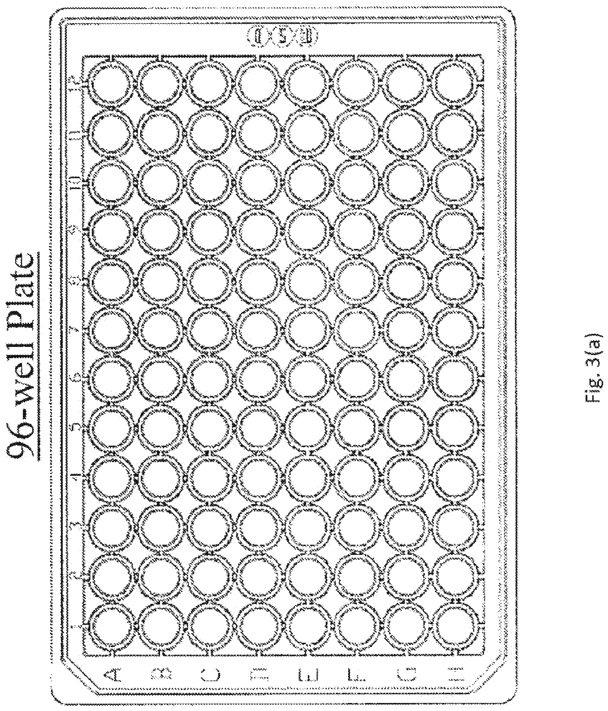

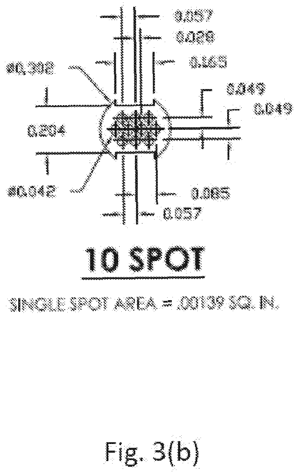

Figs. 3(a)-(b) shows the configuration of a 96 well multi-well assay plate.

### Detailed Description of the Invention

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The articles "a" and "an" are used herein to refer to one or to more than one (i.e., to at least one) of the grammatical object of the article. By way of example, "an element" means one element or more than one element.

As used herein, the term "sample" is intended to mean any biological fluid, cell, tissue, organ or combinations or portions thereof, which includes or potentially includes a biomarker of a disease of interest. For example, a sample can be a histologic section of a specimen obtained by biopsy, or cells that are placed in or adapted to tissue culture. A sample further can be a subcellular fraction or extract, or a crude or substantially pure nucleic acid molecule or protein preparation. In one embodiment, the samples that are analyzed in the assays of the present invention are blood, peripheral blood mononuclear cells (PBMC), isolated blood cells, serum and plasma. Other suitable samples include biopsy tissue, intestinal mucosa, saliva, cerebral spinal fluid, and urine.

The present invention relates to a kit for the analysis of a cytokine panel. At least five assay panels are contemplated and each kit is configured to analyze one of the following panels:

**Table 1. Cytokine Assay Panels**

| Panel | Species | Analytes |
|---|---|---|
| 1 | Human | IFN-gamma, IL-1beta, IL-2, IL-4, IL-6, IL-8, IL-10, IL-12p70, IL-13, TNFalpha |
| 2 | Human | GM-CSF, IL-1alpha, IL-5, IL-7, IL-12/IL-23 p40, IL-15, IL-16, IL-17A, TNF-beta, VEGF-A |
| 3 | Human | Eotaxin, MIP-1 alpha, Eotaxin-3, TARC, IP-10, MIP-1 beta, IL-8, MCP-1, MDC, MCP-4 |
| 4 | Rat | IFN-gamma, IL-2, IL-4, IL-1 beta, IL-5, IL-6, KC/GRO, IL-10, IL-13, TNF-alpha |
| 5 | Mouse | IFN-gamma, IL-1-beta, IL-2, IL-4, IL-5, IL-6, KC/GRO, IL-10, IL-12p70 TNF-alpha |

The kits can include (a) a single panel arrayed on a multi-well plate which is configured to be used in an electrochemiluminescence assay, as well as (b) associated consumables, e.g., detection antibodies, calibrators, and optional diluents and/or buffers. Alternatively, the multi-well plates and associated consumables can be provided separately. Still further, a kit can include two or more multi-well plates with panels arrayed thereon, i.e., panels 1-5, and the associated consumables can be provided in the kit or separately.

Panels 1, 2, 4, and 5 include inflammation-related and/or growth factor biomarkers that are important for inflammation response, immunity, and regulation of numerous biological processes. These secreted biomarkers can be detected in a variety of tissues and bodily fluids and their over- or under-expression can indicate a shift in biological equilibrium of the body. These panels also consist of many of the Th1/Th2 pathway biomarkers. The biomarkers in these panels are involved in numerous disorders such as rheumatoid arthritis, Alzheimer's disease, asthma, atherosclerosis, allergies, systematic lupus erythematosus, obesity, cancer, depression, multiple sclerosis, diabetes, psoriasis, and Crohn's disease, among others.

Panel 3 consists of eight CC chemokine assays (MCP-1, MIP-1a, MIP-1b, Eotaxin, MCP-4, TARC, MDC, and Eotaxin-3) and two CXC chemokine assays (IL-8 and IP-10). Chemokines are small chemotactic cytokines with molecular weights around 8-10 kDa that are capable of inducing directed chemotaxis. The four cysteine residues in conserved locations result in their compact 3-dimensional structure. Based on the spacing of the first two cysteine residues, they are divided into four families of chemokines - CC chemokines, CXC chemokines, C chemokines, and CX3C chemokines, where C represents cysteine and X represents any other amino acids. Chemokines function by activating specific G protein-coupled receptors resulting in migration of inflammatory and non-inflammatory cells. The pro-inflammatory chemokines are responsible for migration of immune cells to the infection site while the homeostatic chemokines are responsible for the migration of cells for the purpose of tissue maintenance and development. Chemokines are associated with number of diseases.

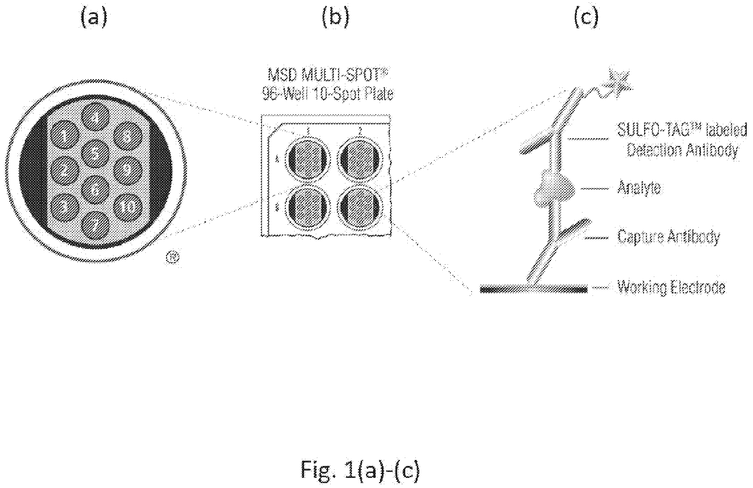

Panels 1-5 are configured in a multi-well assay plate including a plurality of wells, each well having an array with 10 "spots" or discrete binding domains. An example of a 10-spot well is shown in Fig. 1(a) and the incorporation of that well into a multi-well plate is shown in Fig. 1(b). A capture antibody to each analyte is immobilized on a binding domain in the well and that capture antibody is used to detect the presence of the target analyte in an immunoassay as illustrated in Fig. 1(c). Briefly, a sample suspected of containing that analyte is added to the well and if present, the analyte binds to the capture antibody at the designated binding domain. The presence bound analyte on the binding domain is detected by adding labeled detection antibody. The detection antibody also binds to the analyte forming a "sandwich" complex (capture antibody - analyte - detection antibody) on the binding domain. The location of each analyte in Panels 1-5 in this 10-spot pattern is identified in Table 2.

**Table 2. Spot Pattern Configuration Per Panel**

| Panel | Species | Spot Location | Analytes |
|---|---|---|---|
| 1 | Human | 1 | IFN-gamma |
| | | 2 | IL-1beta |
| | | 3 | IL-2 |
| | | 4 | IL-4 |
| | | 5 | IL-6 |
| | | 6 | IL-8 |
| | | 7 | IL-10 |
| | | 8 | IL-12p70 |
| | | 9 | IL-13 |
| | | 10 | TNFalpha |
| 2 | Human | 1 | GM-CSF |
| | | 2 | IL-1alpha |
| | | 3 | IL-5 |
| | | 4 | IL-7 |
| | | 5 | IL-12/IL-23 p40 |
| | | 6 | IL-15 |
| | | 7 | IL-16 |
| | | 8 | IL-17A |
| | | 9 | TNF-beta |
| | | 10 | VEGF-A |
| 3 | Human | 1 | Eotaxin, |
| | | 2 | MIP-1 alpha, |
| | | 3 | Eotaxin-3 |
| | | 4 | TARC |
| | | 5 | IP-10 |
| | | 6 | MIP-1 beta |
| | | 7 | IL-8 |
| | | 8 | MCP-1 |
| | | 9 | MDC |
| | | 10 | MCP-4 |
| 4 | Rat | 1 | IFN-gamma |
| | | 2 | IL-2 |
| | | 3 | IL-4 |
| | | 4 | IL-1 beta |
| | | 5 | IL-5 |
| | | 6 | IL-6 |
| | | 7 | KC/GRO |
| | | 8 | IL-10 |
| | | 9 | IL-13 |
| | | 10 | TNF-alpha |
| 5 | Mouse | 1 | IFN-gamma |
| | | 2 | IL-1-beta |
| | | 3 | IL-2 |
| | | 4 | IL-4 |
| | | 5 | IL-5 |
| | | 6 | IL-6 |
| | | 7 | KC/GRO |
| | | 8 | IL-10 |
| | | 9 | IL-12p70 |
| | | 10 | TNF-alpha |

The multiplexed immunoassay kits described herein allow a user to simultaneously quantify multiple biomarkers. The panels are selected and optimized such that the individual assays function well together. The sample may require dilution prior to being assayed. Sample dilutions for specific sample matrices of interest are optimized for a given panel to minimize sample matrix effects and to maximize the likelihood that all the analytes in the panel will be within the dynamic range of the assay. In a preferred embodiment, all of the analytes in the panel are analyzed with the same sample dilution in at least one sample type. In another preferred embodiment, all of the analytes in a panel are measured using the same dilution for most sample types.

For a given panel, the detection antibody concentration and the number of labels per protein (L/P ratio) for the detection antibody are adjusted to bring the expected levels of all analytes into a quantifiable range at the same sample dilution. If one wants to increase the high end of the quantifiable range for a given analyte, then the L/P can be decreased and/or the detection antibody concentration is decreased. On the other hand, if one wants to increase the lower end of the quantifiable range, the L/P can be increased, the detection antibody concentration can be increased if it is not at the saturation level, and/or the background signal can be lowered.

Calibration standards for use with the assay panels are selected to provide the appropriate quantifiable range with the recommended sample dilution for the panel. The calibration standards have known concentrations of one of more of the analytes in the panel. Concentrations of the analytes in unknown samples are determined by comparison to these standards. In one embodiment, calibration standards comprise mixtures of the different analytes measured by an assay panel. Preferably, the analyte levels in a combined calibrator are selected such that the assay signals for each analyte are comparable, e.g., within a factor of two, a factor of five or a factor of 10. In another embodiment, calibration standards include mixtures of analytes from multiple different assay panels.

A calibration curve may be fit to the assay signals measured with calibration standards using, e.g., curve fits known in the art such as linear fits, 4-parameter logistic (4-PL) and 5-parameter (5-PL) fits. Using such fits, the concentration of analytes in an unknown sample may be determined by backfitting the measured assay signals to the calculated fits. Measurements with calibration standards may also be used to determine assay characteristics such as the limit of detection (LOD), limit of quantification (LOQ), dynamic range, and limit of linearity (LOL).

A kit includes the following assay components: a multi-well assay plate configured to conduct an immunoassay for one of the panels described herein, a set of detection antibodies for the analytes in the panel (wherein the set comprises individual detection antibodies and/or a composition comprising a blend of one or more individual detection antibodies), and a set of calibrators for the analytes in the panel (wherein the set comprises individual calibrator protein compositions and/or a composition comprising a blend of one or more individual calibrator proteins). The kit can also include one of more of the following additional components: a blocking buffer (used to block assay plates prior to addition of sample), an antibody diluent (used to dilute stock detection antibody concentrations to the working concentration), an assay diluent (used to dilute samples), a calibrator diluent (used to dilute or reconstitute calibration standards) and a read buffer (used to provide the appropriate environment for detection of assay labels, e.g., by an ECL measurement). The antibody and assay diluents are selected to reduce background, optimize specific signal, and reduce assay interference and matrix effect. The calibrator diluent is optimized to yield the longest shelf life and retention of calibrator activity. The blocking buffer should be optimized to reduce background. The read buffer is selected to yield the appropriate sensitivity, quantifiable range, and slowest off-rate. The reagent components of the kit can be provided as liquid reagents, lyophilized, or combinations thereof, diluted or undiluted, and the kit includes instructions for appropriate preparation of reagents prior to use. In a preferred embodiment, a set of detection antibodies are included in the kit comprising a plurality of individual detection antibody compositions in liquid form. Moreover, the set of calibrators provided in the kit preferably comprise a lyophilized blend of calibrator proteins. Still further, the kit includes a multi-well assay plate that has been pre-coated with capture antibodies and exposed to a stabilizing treatment to ensure the integrity and stability of the immobilized antibodies.

As part of a multiplexed panel development, assays are optimized to reduce calibrator and detection antibody non-specific binding. In sandwich immunoassays, specificity mainly comes from capture antibody binding. Some considerations for evaluating multiplexed panels include: (a) detection antibody non-specific binding to capture antibodies is reduced to lower background of assays in the panel, and this can be achieved by adjusting the concentrations and L/P of the detection antibodies; (b) non-specific binding of detection antibodies to other calibrators in the panel is also undesirable and should be minimized; (c) non-specific binding of other calibrators in the panel and other related analytes should be minimized; if there is calibrator non-specific binding, it can reduce the overall specificity of the assays in the panel and it can also yield unreliable results as there will be calibrator competition to bind the capture antibody.

Different assays in the panel may require different incubation times and sample handling requirements for optimal performance. Therefore, the goal is to select a protocol that's optimized for most assays in the panel. Optimization of the assay protocol includes, but is not limited to, adjusting one or more of the following protocol parameters: timing (incubation time of each step), preparation procedure (calibrators, samples, controls, etc.), and number of wash steps.

The reagents used in the kits, e.g., the detection and capture antibodies and calibrator proteins, are preferably subjected to analytical testing and meet or exceed the specifications for those tests. The analytical tests that can be used to characterize kit materials include but are not limited to, CIEF, DLS, reducing and/or non-reducing EXPERION, denaturing SDS-PAGE, non-denaturing SDS-PAGE, SEC-MALS, and combinations thereof. In a preferred embodiment, the materials are characterized by CIEF, DLS, and reducing and non-reducing EXPERION. One or more additional tests, including but not limited to denaturing SDS-PAGE, non-denaturing SDS-PAGE, SEC-MALS, and combinations thereof, can also be used to characterize the materials. In a preferred embodiment, the materials are also subjected to functional testing, i.e., a binding assay for the target analyte, as well as one or more characterization tests, such as those listed above. If the materials do not meet or exceed the specifications for the functional and/or characterization tests, they can be subjected to additional purification steps and re-tested. Each of these tests and the metrics applied to the analysis of raw materials subjected to these tests are described below:

Capillary Isoelectric Focusing (CIEF) is a technique commonly used to separate peptides and proteins, and it is useful in the detection of aggregates. During a CIEF separation, a capillary is filled with the sample in solution and when voltage is applied, the ions migrate to a region where they become neutral (pH=pl). The anodic end of the capillary sits in acidic solution (low pH), while the cathodic end sits in basic solution (high pH). Compounds of equal isoelectric points (pI) are "focused" into sharp segments and remain in their specific zone, which allows for their distinct detection based on molecular charge and isoelectric point. Each specific antibody solution will have a fingerprint CIEF that can change over time. When a protein solution deteriorates, the nature of the protein and the charge distribution can change. Therefore, CIEF is a particularly useful tool to assess the relative purity of a protein solution and it is a preferred method of characterizing the antibodies and calibrators in the plates and kits described herein. The metrics used in CIEF include pI of the main peak, the pI range of the solution, and the profile shape, and each of these measurements are compared to that of a reference standard.

Dynamic Light Scattering (DLS) is used to probe the diffusion of particulate materials either in solution or in suspension. By determining the rate of diffusion (the diffusion coefficient), information regarding the size of particles, the conformation of macromolecular chains, various interactions among the constituents in the solution or suspension, and even the kinetics of the scatterers can be obtained without the need for calibration. In a DLS experiment, the fluctuations (temporal variation, typically in a µs to ms time scale) of the scattered light from scatterers in a medium are recorded and analyzed in correlation delay time domain. Like CIEF, each protein solution will generate a fingerprint DLS for the particle size and it's ideally suited to detect aggregation. All IgGs, regardless of binding specificity, will exhibit the same DLS particle size. The metrics used to analyze a protein solution using DLS include percentage polydispersity, percentage intensity, percentage mass, and the radius of the protein peak. In a preferred embodiment, an antibody solution meets or exceeds one or more of the following DLS specifications: (a) radius of the antibody peak: 4-8 nm (antibody molecule size); (b) polydispersity of the antibody peak: <40% (measure of size heterogeneity of antibody molecules); (c) intensity of the antibody peak: >50% (if other peaks are present, then the antibody peak is the predominant peak); and (d) mass in the antibody peak: >50%.

Reducing and non-reducing gel electrophoresis are techniques well known in the art. The EXPERION™ (Bio-Rad Laboratories, Inc., www.bio-rad.com) automated electrophoresis station performs all of the steps of gel-based electrophoresis in one unit by automating and combining electrophoresis, staining, destaining, band detection, and imaging into a single step. It can be used to measure purity. Preferably, an antibody preparation is greater 50% pure by Experion, more preferably, greater than 75% pure, and most preferably greater than 80% pure. Metrics that are applied to protein analysis using non-reducing Experion include percentage total mass of protein, and for reducing Experion they include percentage total mass of the heavy and light chains in an antibody solution, and the heavy to light chain ratio.

Multi-Angle Light Scattering (MALS) detection can be used in the stand-alone (batch) mode to measure specific or non-specific protein interactions, as well as in conjunction with a separation system such as flow field flow fractionation (FFF) or size exclusion chromatography (SEC). The combined SEC-MALS method has many applications, such as the confirmation of the oligomeric state of a protein, quantification of protein aggregation, and determination of protein conjugate stoichiometry. Preferably, this method is used to detect molecular weight of the components of a sample.

In a preferred embodiment, an assay is conducted in a single assay chamber, such as a single well of an assay plate or an assay chamber that is an assay chamber of a cartridge. In a preferred embodiment, the kits of the invention include multi-well assay plates that are configured to conduct an electrochemiluminescence measurement as described for example, in US 20040022677; US 20050052646; US 20050142033; US 20040189311, each of which is incorporated herein by reference in their entireties. Assay plates and plate readers are now commercially available (MULTI-SPOT® and MULTI-ARRAY® plates and SECTOR® instruments, Meso Scale Discovery, a division of Meso Scale Diagnostics, LLC, Gaithersburg, MD.).

As used herein, a lot of kits comprise a group of kits comprising kit components that meet a set of kit release specifications. A lot can include at least 10, at least 100, at least 500, at least 1,000, at least 5,000, or at least 10,000 kits and a subset of kits from that lot are subjected to analytical testing to ensure that the lot meets or exceeds the release specifications. In one embodiment, the release specifications include but are not limited to kit processing, reagent stability, and kit component storage condition specifications. Kit processing specifications include the maximum total sample incubation time and the maximum total time to complete an assay using the kit. Reagent stability specifications include the minimum stability of each reagent component of the kit at a specified storage temperature. Kit storage condition specifications include the range of storage temperatures for all components of the kit, the maximum storage temperature for frozen components of the kit, and the maximum storage temperature for non-frozen components of the kit. A subset of kits in a lot are reviewed in relation to these specifications and the size of the subset depends on the lot size. In a preferred embodiment, for a lot of up to 300 kits, a sampling of 4-7 kits are tested; for a lot of 300-950 kits, a sampling of 8-10 kits are tested; and for a lot of greater than 950 kits, a sampling of 10-12 kits are tested. Alternatively or additionally, a sampling of up to 1-5% preferably up to 1-3%, and most preferably up to 2% is tested.

In addition, each lot of multi-well assay plates is preferably subjected to uniformity and functional testing. A subset of plates in a lot are subjected to these testing methods and the size of the subset depends on the lot size. In a preferred embodiment, for a lot of up to 300 plates, a sampling of 4-7 plates are tested; for a lot of 300-950 plates, a sampling of 8-10 plates are tested; and for a lot of greater than 950 plates, a sampling of 10-12 plates are tested. Alternatively or additionally, a sampling of up to 1-5% preferably up to 1-3%, and most preferably up to 2% is tested. The uniformity and functional testing specifications are expressed in terms of %CV, Coefficient of Variability, which is a dimensionless number defined as the standard deviation of a set of measurements, in this case, the relative signal detected from binding domains across a plate, divided by the mean of the set.

One type of uniformity testing is protein A/G testing. Protein A/G binding is used to confirm that all binding domains within a plate are coupled to capture antibody. Protein A/G is a recombinant fusion protein that combines IgG binding domains of Protein A and protein G and it binds to all subclasses of human IgG, as well as IgA, IgE, IgM and, to a lesser extent, IgD. Protein A/G also binds to all subclasses of mouse IgG but not mouse IgA, IgM, or serum albumin, making it particularly well suited to detect mouse monoclonal IgG antibodies without interference from IgA, IgM, and serum albumin that might be present in the sample matrix. Protein A/G can be labeled with a detectable moiety, e.g., a fluorescent, chemiluminescent, or electrochemiluminescent label, preferably an ECL label, to facilitate detection. Therefore, if capture antibody is adhered to a binding domain of a well, it will bind to labeled protein A/G, and the relative amount of capture antibody bound to the surface across a plate can be measured.

In addition to the uniformity testing described above, a uniformity metric for a subset of plates within a lot can be calculated to assess within-plate trending. A uniformity metric is calculated using a matrix of normalized signals from protein A/G and/or other uniformity or functional tests. The raw signal data is smoothed by techniques known in the art, thereby subtracting noise from the raw data, and the uniformity metric is calculated by subtracting the minimum signal in the adjusted data set from the maximum signal.

In a preferred embodiment, a subset of plates in a lot is subjected to protein A/G and functional testing and that subset meet or exceed the following specifications:

**Table 3(a). Plate Metrics**

| Metric | Preferred Specification for a subset of 96 well multi-well plates |
|---|---|
| Average intraplate CV | ≤ 10% |
| Maximum intraplate CV | ≤ 13% |
| Average Uniformity | ≤ 25% |
| Maximum Uniformity | ≤ 37% |
| CV of intraplate averages | ≤ 18% |
| Signal, lower boundary | >1500 |
| Signal, upper boundary | <10⁽⁶⁾ |

As disclosed in U.S. Patent No. 7,842,246 to Wohlstadter et al., the disclosure of which is incorporated herein by reference in its entirety, each plate consists of several elements, e.g., a plate top, a plate bottom, wells, working electrodes, counter electrodes, reference electrodes, dielectric materials, electrical connects, and assay reagents. The wells of the plate are defined by holes/openings in the plate top. The plate bottom can be affixed, manually or by automated means, to the plate top, and the plate bottom can serve as the bottom of the well. Plates may have any number of wells of any size or shape, arranged in any pattern or configuration, and they can be composed of a variety of different materials. Preferred embodiments of the invention use industry standard formats for the number, size, shape, and configuration of the plate and wells. Examples of standard formats include 96, 384, 1536, and 9600 well plates, with the wells configured in two-dimensional arrays. Other formats may include single well plates (preferably having a plurality of assay domains that form spot patterns within each well), 2 well plates, 6 well plates, 24 well plates, and 6144 well plates. Each well of the plate includes a spot pattern of varying density, ranging from one spot within a well to 2, 4, 7, 9, 10, 16, 25, etc. In a preferred embodiment, the plates used in the kits of the invention comprise 10-spot 96-well plates.

Each plate is assembled according to a set of preferred specifications. In a preferred embodiment, a plate bottom meets or exceeds the following specifications:

**Table 3(b). Plate bottom specifications**

| Parameter | 96-well (round well) specifications in inches |
|---|---|
| Length range (C to C)* | 3.8904-3.9004 (A1-A12 and H1-H12)** |
| Width range (C to C) | 2.4736-2.4836 (A1-A12 and H1-H12) |
| Well to well spacing | 0.3513-0.3573 |

| | |
|---|---|
| *C to C well distance is the center of spot to center of spot distance between the outermost wells of a plate. **As shown in Fig. 3, a 96-well multi-well plate includes a set of wells arranged in an 8 x 12 array, wherein the rows on the short side of the plate are identified by A-H, and the columns on the long side of the plate are identified by 1-12. Therefore, length and width can be measured in row A1-A12 and compared to that of row H1-H12. | |

In a further preferred embodiment, the plate also meets or exceeds defined specifications for alignment of a spot pattern within a well of the plate. These specifications include three parameters: (a) Δx, the difference between the center of the spot pattern and the center of the well along the x axis of the plate (column-wise, long axis); (b) Δy, the difference between the center of the spot pattern and the center of the well along the y axis of the plate (row-wise, short axis); and (c) α, the counter-clockwise angle between the long axis of the plate bottom and the long axis of the plate top of a 96-well plate. In a preferred embodiment, the plate meets or exceeds the following specifications: Δx ≤ 0.2 mm, Δy ≤ 0.2 mm, and α ≤ 0.1°.

The following non-limiting examples serve to illustrate rather than limit the present invention.

### Examples

### Example 1. Reagent Preparation

All reagents were brought to room temperature and diluents were thawed in water at room temperature.

### (i) Preparation of Standards

Multi-analyte lyophilized calibrator blends and all diluents for each panel were obtained from Meso Scale Discovery (Rockville, MD) which yield the recommended highest standard upon reconstitution in one mL of diluent. The lyophilized calibrator was reconstituted and kept on ice. Seven (7) standard solutions and a zero calibrator blank were prepared for up to 4 replicates as follows: (x) The highest standard was prepared by adding 1000 µL of diluent to the lyophilized calibrator vial. The solution was mixed by vortexing and keep on wet ice for a minimum of 5 minutes prior to use. (y) The next standard was prepared by transferring 75 µL of the highest standard to 225 µL of diluent. The solution was mixed well and the procedure repeated 4-fold serial dilutions 5 additional times to generate 7 standards. (z) Diluent was used as the blank. Once reconstituted to the recommended highest standard in Diluent 2, the multi-analyte lyophilized calibrator for each kit is stable at 2 - 8 °C for 30 days.

### (ii) Sample Collection & Handling

When preparing serum, samples were allowed to clot for two hours at room temperature. Plasma prepared in heparin tubes commonly display additional clotting following thawing of the sample. Both serum and plasma were centrifuged for 20 minutes at 2000 x g prior to aliquoting. For serum-free medium, the presence of carrier proteins, e.g., 1% BSA, in the solution was used to prevent loss of analyte to the labware. Samples with extremely high levels of cytokines were diluted. Tissue culture supernatant samples were diluted at least 2-fold in diluent. Upon collection, samples were tested immediately or aliquots were frozen at ≤ 20 °C. Samples were centrifuged at 2000 g for three minutes to remove particulates prior to sample preparation.

### (iii) Dilution of Samples

For human serum, plasma, CSF, urine, and cell culture supernates, a minimum of 2-fold dilution in diluent was done.

### (iv) Preparation of Controls

Controls were prepared in non-human animal matrix with spiked recombinant human analytes. The lyophilized controls were reconstituted in 250 uL of diluent and treated as a sample. Once reconstituted in 250 uL of diluent, the controls were stable for 30 days at 2-8 °C.

### (v) Preparation of Detection Antibody Solutions

Detection antibodies were obtained from Meso Scale Discovery (Rockville, MD) as a 50x stock solution and the working detection antibody solution was 1X. Exposure of 1X detection antibody solution to light was avoided to prevent elevated assay background. Once prepared, the 1X detection antibody solution was kept in the dark.

For 1 plate of Panel 1, the following were combined:
1. 60 uL of 50X SULFO-TAG™ Anti-human IFN-gamma antibody
2. 60 uL of 50X SULFO-TAG Anti-human IL-1 beta antibody
3. 60 uL of 50X SULFO-TAG Anti-human IL-2 antibody
4. 60 uL of 50X SULFO-TAG Anti-human IL-4 antibody
5. 60 uL of 50X SULFO-TAG Anti-human IL-6 antibody
6. 60 uL of 50X SULFO-TAG Anti-human IL-8 antibody
7. 60 uL of 50X SULFO-TAG Anti-human IL-10 antibody
8. 60 uL of 50X SULFO-TAG Anti-human IL-12p70 antibody
9. 60 uL of 50X SULFO-TAG Anti-human IL-13 antibody
10. 60 uL of 50X SULFO-TAG Anti-human TNFalpha antibody
11. 2400 uL Diluent 3 from Meso Scale Discovery (Rockville, MD)

For 1 plate of Panel 2, the following were combined:
1. 60 uL of 50X SULFO-TAG Anti-human GM-CSF antibody
2. 60 uL of 50X SULFO-TAG Anti-human IL-1 alpha antibody
3. 60 uL of 50X SULFO-TAG Anti-human IL-5 antibody
4. 60 uL of 50X SULFO-TAG Anti-human IL-7 antibody
5. 60 uL of 50X SULFO-TAG Anti-human IL-12/IL-23p40 antibody
6. 60 uL of 50X SULFO-TAG Anti-human IL-15 antibody
7. 60 uL of 50X SULFO-TAG Anti-human IL-16 antibody
8. 60 uL of 50X SULFO-TAG Anti-human IL-17A antibody
9. 60 uL of 50X SULFO-TAG Anti-human TNFbeta antibody
10. 60 uL of 50X SULFO-TAG Anti-human VEGF-A antibody
11. 2400 uL Diluent 3 from Meso Scale Discovery (Rockville, MD)

For 1 plate of Panel 3, the following were combined:
1. 60 uL of 50X SULFO-TAG Anti-human Eotaxin antibody
2. 60 uL of 50X SULFO-TAG Anti-human MIP-1beta antibody
3. 60 uL of 50X SULFO-TAG Anti-human MCP-4 antibody
4. 60 uL of 50X SULFO-TAG Anti-human Eotaxin-3 antibody
5. 60 uL of 50X SULFO-TAG Anti-human TARC antibody
6. 60 uL of 50X SULFO-TAG Anti-human IP-10 antibody
7. 60 uL of 50X SULFO-TAG Anti-human MIP-1alpha antibody
8. 60 uL of 50X SULFO-TAG Anti-human IL-8 antibody
9. 60 uL of 50X SULFO-TAG Anti-human MCP-1 antibody
10. 60 uL of 50X SULFO-TAG Anti-human MDC antibody
11. 2400 uL Diluent 3 from Meso Scale Discovery (Rockville, MD)

For 1 plate of Panel 4, the following were combined:
1. 60 uL of 50X SULFO-TAG Anti-rat IFN-gamma antibody
2. 60 uL of 50X SULFO-TAG Anti- rat IL-2 antibody
3. 60 uL of 50X SULFO-TAG Anti- rat IL-4 antibody
4. 60 uL of 50X SULFO-TAG Anti- rat IL-1 beta antibody
5. 60 uL of 50X SULFO-TAG Anti- rat IL-5 antibody
6. 60 uL of 50X SULFO-TAG Anti- rat IP-6 antibody
7. 60 uL of 50X SULFO-TAG Anti- rat KC/GRO antibody
8. 60 uL of 50X SULFO-TAG Anti- rat IL-10 antibody
9. 60 uL of 50X SULFO-TAG Anti- rat IL-13 antibody
10. 60 uL of 50X SULFO-TAG Anti- rat TNF alpha antibody
11. 2400 uL Diluent 40 from Meso Scale Discovery (Rockville, MD)

For 1 plate of Panel 5, the following were combined:
1. 60 uL of 50X SULFO-TAG Anti-mouse IFN gamma antibody
2. 60 uL of 50X SULFO-TAG Anti- mouse IL-1 beta antibody
3. 60 uL of 50X SULFO-TAG Anti- mouse IL-2 antibody
4. 60 uL of 50X SULFO-TAG Anti- mouse IL-4 antibody
5. 60 uL of 50X SULFO-TAG Anti- mouse IL-5 antibody
6. 60 uL of 50X SULFO-TAG Anti- mouse IP-6 antibody
7. 60 uL of 50X SULFO-TAG Anti- mouse KC/GRO antibody
8. 60 uL of 50X SULFO-TAG Anti- mouse IL-10 antibody
9. 60 uL of 50X SULFO-TAG Anti- mouse IL-12p70 antibody
10. 60 uL of 50X SULFO-TAG Anti- mouse TNF alpha antibody
11. 2400 uL Diluent 45 from Meso Scale Discovery (Rockville, MD)

### (vi) Preparation of Read Buffer

Read Buffer T (also available from Meso Scale Discovery) is obtained as a 4X stock solution and the working solution was 2X. For 1 plate, equal parts (10 mL) of Read Buffer T (4X) was combined with deionized water (10 mL). A working solution of read buffer was prepared in advance and stored at room temperature in a tightly sealed container (stable for up to three years).

### (vii) Preparation of MSD Plate

Multi-well plates (also available from Meso Scale Discovery) were pre-coated with capture antibodies (Figure 1) and exposed to a proprietary stabilizing treatment to ensure the integrity and stability of the immobilized antibodies. Plates were used as delivered; no additional preparation (e.g., pre-wetting) was required.

### Example 2. Assay Protocol

(i) Fifty (50) uL of diluted sample (standards, controls, or unknowns) per well were added. The plate was sealed with an adhesive plate seal and incubated for 2 hours with vigorous shaking (300-1000 rpm) at room temperature.
(ii) The plate was washed 3 times with 150-300 uL/well of PBS-T. Twenty-five (25) uL of detection antibody solution was added to each well. The plate was sealed with an adhesive plate seal and incubated for 2 hours with vigorous shaking (300-1000 rpm) at room temperature.
(iii) The plate was washed 3 times with 150-300 uL/wel of PBS-T. One hundred fifty (150) uL of 2X Read Buffer T (Meso Scale Discovery, Rockville, MD) was added to each well. The plate was analyzed in a SECTOR® Imager (Meso Scale Discovery, Rockville, MD).

### Example 3. Panel Verification

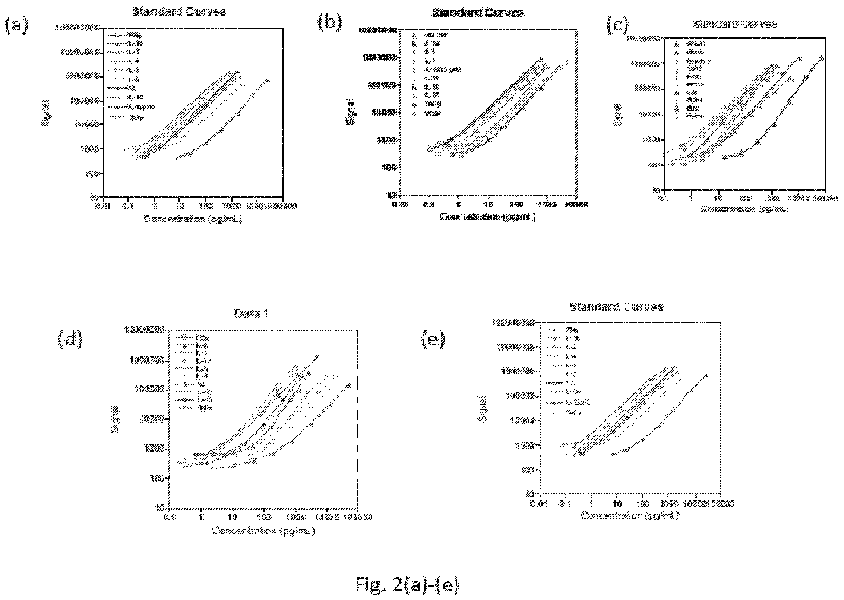

Assay development and evaluation of assay performance was executed utilizing industry and regulatory guidelines. During product development, kit components and protocols were developed and optimized to yield optimum product performance. The robustness of the assay protocol was evaluated to examine the boundaries of selected incubation times. Accelerated stability studies for calibrators, antibodies, and controls were performed during assay development and were augmented with real-time stability studies on complete kits out to 36 months from the date of manufacture. Verification of product design specifications was performed by evaluating standard curves, and a set of controls for each panel (also obtained from Meso Scale Discovery, Rockville, MD) for three days by two independent analysts for a total of eight plates. Each plate was considered as a run. A summary of the standard curve data is shown in Figs. 2(a)-(e) and Tables 4-8.

Intra- and inter-run precision and accuracy for a set of controls for each panel was evaluated for nine runs. Precision and accuracy were verified for each lot as part of the lot verification and quality control release. The typical specification for precision is a concentration CV of less than 20% for controls on both intra-and inter-day runs. As part of product verification, the performance of each panel was evaluated for spike and recovery and dilution linearity in serum, heparin plasma, EDTA plasma, citrate plasma, CSF, urine, and/or cell culture supernates. Native human analyte levels were measured in serum, heparin plasma, EDTA plasma, citrate plasma, CSF, and urine. Native rat and mouse analyte levels were measured in serum, heparin plasma, EDTA plasma, and urine.

Pooled human blood was stimulated in vitro with different stimuli (LPS and Zymosan and Peptidoglycan) and at the end of the stimulation period, plasma was isolated. In addition, for panels 1-3, THP-1 cell line was stimulated with LPS and at the end of stimulation, lysates were prepared. Freshly isolated PBMC were treated with different stimulating agents and supernates were isolated. The plasma, the cell lysates, and PBMC supernates were then evaluated for native human analyte levels using panels 1-3. For panel 4, rat macrophase cell line NR8383 was stimulated with LPS, PHA, and Pokeweed mitogen (PWM) and the cell lysate and cell culture supernates were isolated. The plasma, cell lysates, and cell culture supernates were evaluated for native rat analyte levels using panel 4. For panel 5, RAW cell line was stimulated with LPS and J774A.1 cell line was stimulated with LPS and PWM and at the end of stimulation, lysates were prepared. The plasma and cell lysates were then evaluated for native mouse analyte levels using panel 5.

Fig. 2(a)-(e) shows a standard curve graph that illustrates the dynamic range of each panel (panels 1-5, respectively).

**Table 4. Panel 1 Typical Data**

| **IFNγ** | | | **IL-1β** | | | **IL-2** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **% CV** |
| 0 | 364 | 9.5 | 0 | 979 | 7.6 | 0 | 278 | 12.0 |
| 0.31 | 485 | 5.0 | 0.12 | 1435 | 5.2 | 0.31 | 533 | 9.0 |
| 1.2 | 856 | 5.1 | 0.49 | 2640 | 4.6 | 1.2 | 1257 | 5.4 |
| 4.9 | 2120 | 2.7 | 2.0 | 7149 | 4.0 | 4.9 | 3826 | 3.7 |
| 20 | 7559 | 1.8 | 7.8 | 25 643 | 3.5 | 20 | 13 922 | 3.2 |
| 78 | 29 285 | 1.1 | 31 | 97 601 | 2.3 | 78 | 55 350 | 3.2 |
| 313 | 114 776 | 2.0 | 125 | 385 990 | 4.3 | 313 | 207 898 | 2.8 |
| 1250 | 420 758 | 1.6 | 500 | 1 523 384 | 2.5 | 1250 | 740 647 | 3.8 |

| **IL-4** | | | **IL-6** | | | **IL-8** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 203 | 11.1 | 0 | 219 | 8.0 | 0 | 208 | 12..0 |
| 0.05 | 365 | 10.1 | 016 | 404 | 9.4 | 0.12 | 370 | 5.4 |
| 0.21 | 933 | 8.1 | 0.63 | 982 | 4.8 | 0.49 | 858 | 4.3 |
| 0.82 | 3008 | 6.6 | 2.5 | 3137 | 3.6 | 2.0 | 2724 | 2.7 |
| 3.3 | 12 155 | 2.7 | 10 | 12 068 | 2.7 | 7.8 | 10 113 | 2.5 |
| 13 | 47 521 | 2.0 | 41 | 49 674 | 3.4 | 31 | 41 493 | 2.2 |
| 53 | 178 863 | 2.5 | 163 | 215 238 | 3.7 | 125 | 169 766 | 2.6 |
| 210 | 618 057 | 2.2 | 650 | 930 463 | 2.7 | 500 | 722 931 | 2.2 |

| **IL-10** | | | **IL-12p70** | | | **IL-13** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 232 | 10.7 | 0 | 267 | 15.5 | 0 | 188 | 18.5 |
| 0.08 | 420 | 9.8 | 0.10 | 327 | 13.1 | 0.11 | 241 | 15.1 |
| 0.3 | 937 | 5.4 | 0.41 | 511 | 7.2 | 0.45 | 405 | 9.3 |
| 1.2 | 2970 | 3.1 | 1.6 | 1348 | 6.1 | 1.8 | 1030 | 3.7 |
| 4.8 | 10 699 | 43 | 6.6 | 4787 | 3.6 | 7.3 | 3543 | 2.0 |
| 19 | 43 376 | 3.5 | 26 | 18 351 | 2.3 | 29 | 17 828 | 2.1 |
| 78 | 163 839 | 2.1 | 105 | 69 571 | 43 | 118 | 110 781 | 4.4 |
| 310 | 568 597 | 2.3 | 420 | 250 748 | 2.8 | 470 | 587 447 | 1.7 |

| **TNFα** | | | | | | | | |
|---|---|---|---|---|---|---|---|---|
| **Conc. (pg/mL)** | **Average Signal** | **%CV** | | | | | | |
| 0 | 138 | 19.2 | | | | | | |
| 0.08 | 266 | 12.8 | | | | | | |
| 0.32 | 606 | 9.9 | | | | | | |
| 1.3 | 1960 | 3.7 | | | | | | |
| 5.2 | 7100 | 2.5 | | | | | | |
| 21 | 29 253 | 2.6 | | | | | | |
| 83 | 118 437 | 4.7 | | | | | | |
| 330 | 508 866 | 2.7 | | | | | | |

**Table 5. Panel 2 Typical Data**

| **GM-CSF** | | | **IL-1α** | | | **IL-5** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 221 | 14.3 | 0 | 401 | 11.9 | 0 | 571 | 10.5 |
| 0.24 | 355 | 7.5 | 0.1 | 531 | 7.5 | 0.2 | 833 | 8.7 |
| 0.98 | 781 | 5.5 | 0.4 | 852 | 5.8 | 0.8 | 1438 | 7.4 |
| 3.9 | 2374 | 4.8 | 1.4 | 2167 | 84 | 3.1 | 3961 | 5.8 |
| 16 | 9635 | 43 | 58 | 7368 | 5.1 | 12 | 14 364 | 7.9 |
| 63 | 35 827 | 3.4 | 23 | 27 075 | 5.5 | 49 | 52 918 | 3.4 |
| 250 | 139 828 | 3.4 | 93 | 110 306 | 3.2 | 198 | 198 664 | 3.8 |
| 1000 | 472 016 | 3.2 | 370 | 394 888 | 3.6 | 790 | 639 511 | 5.7 |

| **IL-7** | | | **IL-12/IL-23 p40** | | | **IL-15** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 235 | 12. 6 | 0 | 285 | 8.7 | 0 | 227 | 8.3 |
| 0.2 | 342 | 9.5 | 0.7 | 431 | 7.1 | 0.2 | 338 | 8.6 |
| 0.7 | 712 | 6.1 | 2.9 | 874 | 3.5 | 0.7 | 681 | 6.1 |
| 2.9 | 2107 | 5.1 | 12 | 2531 | 3.8 | 2.7 | 1954 | 4.6 |
| 12 | 8770 | 6.3 | 47 | 10 105 | 6.5 | 11 | 7840 | 7.0 |
| 47 | 32 322 | 3.2 | 188 | 36 783 | 5.9 | 44 | 28 139 | 3.5 |
| 188 | 137 340 | 2.8 | 750 | 144 847 | 2.8 | 175 | 105 824 | 5.3 |
| 750 | 581 986 | 2.1 | 3000 | 512 130 | 5.5 | 700 | 464 580 | 3.0 |

| **IL-16** | | | **IL-17A** | | | **TNFβ** | | |
|---|---|---|---|---|---|---|---|---|
| **Conc. (pg/mL)** | **Average Signal** | **% CV** | **Conc. (pg/mL)** | **Average Signal** | **%CV** | **Conc. (pg/mL)** | **Average Signal** | **%CV** |
| 0 | 264 | 11.3 | 0 | 144 | 28.9 | 0 | 248 | 10.9 |
| 0.61 | 326 | 11.4 | 1.2 | 290 | 103 | 0.1 | 456 | 6.1 |
| 2.4 | 477 | 6.6 | 4.8 | 821 | 8.1 | 0.6 | 1156 | 3.8 |
| 9.8 | 1090 | 4.8 | 19 | 2719 | 4.3 | 2.4 | 3813 | 3.0 |
| 39 | 4481 | 6.4 | 76 | 12 286 | 6.8 | 10 | 15 143 | 2.4 |
| 156 | 15 741 | 2.1 | 304 | 45 447 | 4.4 | 38 | 57 815 | 2.1 |
| 625 | 82 935 | 4.1 | 1218 | 190 122 | 2.5 | 153 | 233 155 | 1.3 |
| 2500 | 436 497 | 4.5 | 4870 | 684 182 | 4.4 | 610 | 890 796 | 3.2 |

| **VEGF** | | | | | | | | |
|---|---|---|---|---|---|---|---|---|
| **Conc. (pg/mL)** | **Average Signal** | **%CV** | | | | | | |
| 0 | 476 | 5.2 | | | | | | |
| 0.3 | 547 | 8.9 | | | | | | |
| 1.0 | 681 | 5.4 | | | | | | |
| 4.2 | 1235 | 3.5 | | | | | | |
| 17 | 4187 | 7.1 | | | | | | |
| 67 | 14 990 | 3.7 | | | | | | |
| 268 | 93 22 7 | 3.4 | | | | | | |
| 1070 | 517 033 | 3.1 | | | | | | |

**Table 6. Panel 3 Typical Data**

| **Eotaxin** | | | **MIP-1β** | | | **Eotaxin-3** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 189 | 13.2 | 0 | 91 | 39.9 | 0 | 114 | 35.7 |
| 0.37 | 215 | 7.6 | 0.24 | 160 | 16.9 | 1.2 | 208 | 10.7 |
| 1.5 | 252 | 14.5 | 1.0 | 313 | 5.9 | 4.9 | 501 | 5.3 |
| 5.9 | 430 | 6.3 | 3.9 | 1033 | 3.9 | 20 | 1610 | 3.4 |
| 23 | 2408 | 5.8 | 16 | 5293 | 3.0 | 78 | 6029 | 1.6 |
| 94 | 21 595 | 1.8 | 63 | 35 020 | 2.3 | 313 | 23 154 | 3.0 |
| 375 | 159 348 | 1.8 | 250 | 210 203 | 3.4 | 1250 | 85 026 | 5.2 |
| 1500 | 813 344 | 3.4 | 1000 | 834 960 | 4.2 | 5000 | 283 990 | 4.9 |

| **TARC** | | | **IP-10** | | | **MIP-1α** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 283 | 16.7 | 0 | 88 | 29.3 | 0 | 141 | 18.3 |
| 0.37 | 546 | 8.5 | 0.6 | 435 | 7.1 | 0.2 | 170 | 16.9 |
| 1.5 | 1409 | 3.5 | 2.4 | 1532 | 4.7 | 1.0 | 185 | 14.5 |
| 5.9 | 4580 | 3.0 | 10 | 5655 | 3.5 | 3.9 | 297 | 14.3 |
| 23 | 18 048 | 4.4 | 39 | 22 406 | 3.8 | 15 | 1023 | 5.1 |
| 94 | 69 091 | 2.7 | 156 | 84 024 | 4.2 | 62 | 7812 | 2.7 |
| 375 | 269 113 | 2.7 | 625 | 264 067 | 3.7 | 248 | 69 584 | 4.2 |
| 1500 | 873 335 | 2.9 | 2500 | 437 795 | 7.1 | 990 | 454 451 | 3.0 |

| **IL-8** | | | **MCP-1** | | | **MDC** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 127 | 21.5 | 0 | 171 | 17.8 | 0 | 152 | 18.3 |
| 18 | 218 | 23.5 | 0.1 | 277 | 11.1 | 2 | 288 | 6.0 |
| 71 | 338 | 10.1 | 0.5 | 654 | 6.6 | 10 | 677 | 3.3 |
| 283 | 888 | 7.6 | 2.0 | 2090 | 6.4 | 39 | 2273 | 4.1 |
| 1131 | 4028 | 4.8 | 7.8 | 8243 | 6.1 | 156 | 9996 | 3.0 |
| 4525 | 31 569 | 7.4 | 31 | 33 293 | 4.1 | 625 | 59 008 | 4.3 |
| 18100 | 314 805 | 4.6 | 125 | 134 498 | 3.3 | 2500 | 406 977 | 4.2 |
| 72400 | 1 784 204 | 3.3 | 500 | 547 716 | 8.3 | 10000 | 1 163 220 | 3.8 |

| **MCP-4** | | | | | | | | |
|---|---|---|---|---|---|---|---|---|
| **Conc. (pg/mL)** | **Average Signal** | **%CV** | | | | | | |
| 0 | 71 | 29.8 | | | | | | |
| 0.2 | 121 | 28.7 | | | | | | |
| 0.6 | 120 | 32.8 | | | | | | |
| 2.4 | 187 | 16.9 | | | | | | |
| 10 | 976 | 8.6 | | | | | | |
| 39 | 9827 | 3.8 | | | | | | |
| 156 | 80 691 | 3.8 | | | | | | |
| 625 | 494 877 | 1.7 | | | | | | |

**Table 7. Panel 4 Typical Data**

| **IFNγ** | | | **IL-2** | | | **IL-4** | | |
|---|---|---|---|---|---|---|---|---|
| **Conc. (pg/mL)** | **Average Signal** | **%CV** | **Conc. (pg/mL)** | **Average Signal** | **% CV** | **Conc. (pg/mL)** | **Average Signal** | **%CV** |
| 0 | 462 | 11 | 0 | 334 | 13 | 0 | 281 | 11 |
| 1.2 | 663 | 6.8 | 12 | 330 | 16 | 0.2 | 348 | 11 |
| 4.9 | 1383 | 6.3 | 49 | 425 | 8.8 | 1.0 | 499 | 7.9 |
| 20 | 4056 | 3.8 | 195 | 747 | 5.9 | 4.0 | 1184 | 6.3 |
| 78 | 15 075 | 2.7 | 781 | 1969 | 6.1 | 16 | 3974 | 6.4 |
| 313 | 65 765 | 3.8 | 3125 | 7571 | 5.7 | 64 | 21 355 | 4.2 |
| 1250 | 318 965 | 2.3 | 1 2500 | 32 383 | 4.3 | 255 | 132 949 | 3.7 |
| 5000 | 1 334 897 | 4.5 | 50 000 | 147 984 | 7.6 | 1020 | 660 873 | 4.9 |

| **IL-1 β** | | | **IL-5** | | | **IL-6** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 523 | 14 | 0 | 218 | 14 | 0 | 491 | 7.4 |
| 2.6 | 506 | 13 | 2.4 | 220 | 15 | 3.0 | 485 | 8.8 |
| 11 | 542 | 9.2 | 98 | 250 | 9.0 | 12 | 542 | 7.2 |
| 42 | 683 | 7.6 | 39 | 400 | 9.0 | 48 | 729 | 9.0 |
| 169 | 1508 | 2.9 | 156 | 1477 | 66 | 194 | 1565 | 5.8 |
| 675 | 4915 | 2.9 | 625 | 11226 | 6.1. | 775 | 5186 | 5.7 |
| 2700 | 20 813 | 2.8 | 2500 | 75 782 | 4.3 | 3100 | 22 199 | 5.6 |
| 10800 | 93 359 | 4.4 | 10 000 | 295 607 | 6.3 | 12 400 | 108 108 | 11 |

| **KC/GRO** | | | **IL-10** | | | **IL-13** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 648 | 9.9 | 0 | 596 | 14 | 0 | 275 | 16 |
| 0.7 | 687 | 5.6 | 4.9 | 583 | 20 | 0.4 | 269 | 17 |
| 2.7 | 714 | 6.0 | 20 | 858 | 16 | 1.6 | 336 | 9.1 |
| 11 | 781 | 5.4 | 78 | 1630 | 7.9 | 63 | 563 | 8.6 |
| 43 | 1228 | 6.8 | 313 | 5131 | 6.7 | 25 | 1544 | 3.6 |
| 174 | 5568 | 5.0 | 1250 | 19 039 | 5.2 | 100 | 7033 | 4.5 |
| 695 | 49356 | 3.9 | 5000 | 73 865 | 43 | 400 | 44 979 | 2.3 |
| 2780 | 387 251 | 6.6 | 20 000 | 278 070 | 6.1 | 1600 | 299 022 | 6.2 |

| **TNFα** | | | | | | | | |
|---|---|---|---|---|---|---|---|---|
| **Conc. (pg/mL)** | **Average Signal** | **%CV** | | | | | | |
| 0 | 208 | 17 | | | | | | |
| 0.3 | 259 | 9.9 | | | | | | |
| 1.1 | 404 | 10 | | | | | | |
| 4.5 | 993 | 6.1 | | | | | | |
| 18 | 3323 | 4.8 | | | | | | |
| 73 | 15 943 | 2.8 | | | | | | |
| 290 | 92 423 | 3.7 | | | | | | |
| 1160 | 535 091 | 4.1 | | | | | | |

**Table 8. Panel 5 Typical Data**

| **IFNγ** | | | **IL-1β** | | | **IL-2** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 311 | 14.5 | 0 | 284 | 12.4 | 0 | 289 | 12.0 |
| 0.2 0 | 821 | 5.6 | 0.34 | 562 | 6.3 | 0.51 | 513 | 8.2 |
| 0.78 | 2443 | 2.4 | 1.4 | 1462 | 3.6 | 2.1 | 1232 | 5.4 |
| 3.1 | 8641 | 2.3 | 5.5 | 4909 | 2.6 | 8.2 | 4094 | 3.6 |
| 13 | 35510 | 1.9 | 22 | 19280 | 2.1 | 33 | 15136 | 3.5 |
| 50 | 137358 | 1.8 | 8.8 | 77541 | 2.2 | 131 | 64 545 | 3.4 |
| 200 | 504 897 | 2.2 | 350 | 307 686 | 1.9 | 525 | 264 160 | 2.3 |
| 800 | 1437412 | 2.2 | 1400 | 1 149 886 | 1.9 2100 | | 939 169 | 1.6 |

| **IL-4** | | | **IL-5** | | | **IL-6** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 495 | 8.9 | 0 | 145 | 21.6 | 0 | 369 | 7.8 |
| 0.34 | 732 | 7.3 | 0.2 | 412 | 10.6 | 1.1 | 544 | 6.0 |
| 1.4 | 1529 | 4.0 | 0.78 | 1298 | 4.2 | 4.4 | 1107 | 5.9 |
| 5.5 | 4351 | 2.9 | 3.1 | 4826 | 2.6 | 18 | 3321 | 2.3 |
| 22 | 17 081 | 1.6 | 13 | 19027 | 3.0 | 70 | 12 162 | 1.8 |
| 88 | 65 254 | 2.7 | 50 | 79 736 | 3.9 | 281 | 50 520 | 2.8 |
| 350 | 245 316 | 2.6 | 200 | 336 576 | 2.9 | 1125 | 225 797 | 2.2 |
| 1400 | 798 882 | 2.1 | 800 | 1 282 609 | 2.9 | 4500 | 1 085 547 | 2.8 |

| **KC/GRO** | | | **IL-10** | | | **IL-12p70** | | |
|---|---|---|---|---|---|---|---|---|
| **Conc. (pg/mL)** | **Average Signal** | **%CV** | **Conc. (pg/mL)** | **Average Signal** | **%CV** | **Conc. (pg/mL)** | **Average Signal** | **%CV** |
| 0 | 307 | 10.3 | 0 | 763 | 10.1 | 0 | 372 | 7.6 |
| 0.39 | 480 | 9.1 | 0.63 | 819 | 5.9 | 6.4 | 453 | 11.1 |
| 1.6 | 1129 | 2.6 | 2.5 | 1125 | 6.2 | 26 | 684 | 5.1 |
| 6.3 | 3695 | 1.9 | 10 | 2349 | 3.1 | 103 | 1754 | 2.9 |
| 25 | 13 975 | 2.8 | 41 | 7758 | 3.4 | 413 | 6261 | 2.6 |
| 100 | 65 482 | 2.6 | 163 | 29289 | 2.2 | 1650 | 30 330 | 2.9 |
| 400 | 335078 | 1.9 | 650 | 121 581 | 3.7 | 6600 | 163 885 | 2.8 |
| 1600 | 1 609 664 | 2.1 | 2600 | 504 589 | 43 | 26400 | 731 703 | 2.9 |

| **TNFα** | | | | | | | | |
|---|---|---|---|---|---|---|---|---|
| **Conc. (pg/mL)** | **Average Signal** | **% CV** | | | | | | |
| 0 | 883 | 5.8 | | | | | | |
| 0.12 | 985 | 5.5 | | | | | | |
| 0.49 | 1291 | 43 | | | | | | |
| 2 | 2592 | 3.1 | | | | | | |
| 7.8 | 7460 | 3.4 | | | | | | |
| 31 | 29 633 | 2.9 | | | | | | |
| 125 | 128 475 | 3.0 | | | | | | |
| 500 | 582 743 | 3.3 | | | | | | |

The lower limit of detection (LLOD) is a calculated concentration based on a signal 2.5 standard deviations above the background (zero calibrator blank). The LLOD shown in Tables 9-13 for each panel was calculated based on 8-9 runs.

**Table 9. Panel 1 LLOD**

| | IFNγ | IL-1β | IL-2 | IL-4 | IL-6 | IL-8 | IL-10 | IL-12p70 | IL-13 | TNFα |
|---|---|---|---|---|---|---|---|---|---|---|
| Median LLOD (gp/mL) | 0.22 | 0.05 | 0.12 | 0.03 | 0.07 | 0.01 | 0.03 | 0.13 | 0.27 | 0.06 |
| LLOD Range (pg/mL) | 0.18-0.37 | 0.02-0.04 | 0.07-0.13 | 0.02-0.04 | 0.46-1.0 | 0.01-0.01 | 0.02-003 | 0.09-0.19 | 0.21-0.43 | 0.05-0.10 |

**Table 10. Panel 2 LLOD**

| | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|
| Median LLOD (pg/mL) | 0.14 | 0.08 | 0.10 | 0.15 | 0.40 | 0.14 | 1.60 | 0.77 | 0.06 | 0.9 |
| LLOD Range (pp/mL) | 0.10-0.34 | 0.05-0.29 | 0.08-0.28 | 0.11-0.22 | 0.30-0.58 | 0.08-0.19 | 0.98-2.77 | 0.50-2.70 | 0.04-0.12 | 0.75-1.39 |

**Table 11. Panel 3 LLOD**

| | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|
| Median LLOD (pg/mL) | 3.2 | 0.34 | 1.2 | 0.13 | 0.13 | 1.4 | 54 | 0.09 | 2.6 | 2.3 |
| LLOD Range (pg/mL) | 2.2-4.9 | 0.42-0.79 | 0.79-3.1 | 0.10-0.22 | 0.09-0.22 | 1.4-2.3 | 31-62 | 0.07-0.20 | 1.7-2.1 | 1.6-2.7 |

**Table 12. Panel 4 LLOD**

| | IFNγ | IL-2 | IL-4 | IL-1 β | IL-5 | IL-6 | KC/GRO | IL-10 | IL-13 | TNFα |
|---|---|---|---|---|---|---|---|---|---|---|
| Median LLOD (pg/mL) | 0.7 | 57 | 0.7 | 35 | 27 | 23 | 21 | 14 | 3.7 | 0.9 |
| LLOD Range (pg/mL) | 0.4-2.7 | 38-126 | 0.4-1.0 | 16-80 | 19-37 | 16-41 | 19-30 | 8.9-20 | 2.7-8.6 | 0.5-1.5 |

**Table 13. Panel 5 LLOD**

| | IFNγ | IL-1β | IL-2 | IL-4 | IL-5 | IL-6 | KC/GRO | IL-10 | IL-12p70 | TNFα |
|---|---|---|---|---|---|---|---|---|---|---|
| Median LLOD (pg/mL) | 0.042 | 0.11 | 0.22 | 0.19 | 0.056 | 0.606 | 0.22 | 1.1 | 8.9 | 0.15 |
| LLOD Range (pg/mL) | 0.025-0.084 | 0.093-0.171 | 0.165-0.338 | 0.099-0.343 | 0.05-0.099 | 0.486-1.075 | 0.185-0.373 | 0.518-3.19 | 7.598-14.254 | 0.109-0.548 |

Controls were made by spiking calibrator into non-human animal matrix for panels 1-3, rat serum for panel 4, and mouse serum for panel 5, at levels throughout the range of the assay. Analyte levels were measured using a minimum of 3 replicates on 3 runs over 3 days. Average intra-run %CV is the average %CV of the control replicates within an individual run. Inter-run %CV is the variability of controls across a selected number of runs. Inter-lot %CV is the variability of controls across a selected number of kit lots.

**Table 14. Panel 1**

| | Control | Runs | Average Conc. (pg/mL) | Average Intra-nm %CV | Inter-run %CV | Inter-lot %CV |
|---|---|---|---|---|---|---|
| IFNγ | High | 9 | 1941 | 1.9 | 6.8 | 4.8 |
| | Mid | 9 | 203 | 1.9 | 8.3 | |
| | Low | 9 | 16 | 4.8 | 7.8 | |
| IL-1β | High | 9 | 107 | 2.6 | 5.3 | |
| | Mid | 9 | 11 | 1.8 | 6.5 | |
| | Low | 9 | 7 | 3.4 | 9.8 | |
| IL-2 | High | 9 | 986 | 2.2 | 3.2 | |
| | Mid | 9 | 99 | 2.2 | 4.8 | |
| | Low | 9 | 9 | 4.9 | 13 | |
| IL-4 | High | 9 | 294 | 1.7 | 6.2 | |
| | Mid | 9 | 32 | 4.4 | 6.7 | |
| | Low | 9 | 4 | 3.5 | 5.8 | |
| IL-6 | High | 9 | 801 | 3.4 | 5.8 | |
| | Mid | 9 | 76 | 3.0 | 4.6 | |
| | Low | 9 | 6 | 4.1 | 7.0 | |
| IL-8 | High | 9 | 613 | 2.3 | 5.2 | |
| | Mid | 9 | 60 | 1.8 | 6.1 | |
| | Low | 9 | 8 | 3.4 | 8.4 | |
| IL-10 | High | 9 | 372 | 2.2 | 2.8 | |
| | Mid | 9 | 39 | 1.5 | 5.5 | |
| | Low | 9 | 4 | 3.3 | 8.6 | |
| IL-12p70 | High | 9 | 467 | 4.4 | 6.4 | |
| | Mid | 9 | 51 | 3.2 | 6.6 | |
| | Low | 9 | 5 | 3.9 | 4.3 | |
| IL-13 | High | 9 | 657 | 3.0 | 8.6 | |
| | Mid | 9 | 74 | 2.7 | 13.8 | |
| | Low | 9 | 5 | 6.3 | 11.6 | |
| TNFα | High | 9 | 270 | 4.0 | 7.2 | |
| | Mid | 9 | 24 | 3.7 | 9.6 | |
| | Low | 9 | 3 | 3.7 | 12.0 | |

**Table 15. Panel 2**

| | Control | Runs | Average Conc. (pg/mL) | Average Intra-run %CV | Inter-run % CV | Inter-lot %CV |
|---|---|---|---|---|---|---|
| GM-CSF | High | 9 | 506 | 4.0 | 22.1 | N/A |
| | Mid | 9 | 53 | 2.6 | 20.8 | N/A |
| | Low | 9 | 5 | 4.8 | 16.8 | N/A |
| IL-1α | High | 9 | 144 | 3.4 | 11.8 | N/A |
| | Mid | 9 | 15 | 3.2 | 12.4 | N/A |
| | Low | 9 | 2 | 5.7 | 13.8 | N/A |
| IL-5 | High | 9 | 450 | 4.2 | 16.6 | N/A |
| | Mid | 9 | 45 | 2.4 | 18.2 | N/A |
| | Low | 9 | 4 | 4.4 | 16.0 | N/A |
| IL-7 | High | 9 | 437 | 3.3 | 9.7 | N/A |
| | Mid | 9 | 44 | 2.9 | 4.8 | N/A |
| | Low | 9 | 5 | 3.8 | 4.6 | N/A |
| IL-12/IL-23p40 | High | 9 | 1631 | 3.7 | 10.9 | N/A |
| | Mid | 9 | 173 | 2.4 | 9.9 | N/A |
| | Low | 9 | 17 | 3.9 | 8.0 | N/A |
| IL-15 | High | 9 | 317 | 3.5 | 26.0 | N/A |
| | Mid | 9 | 35 | 3.8 | 31.1 | N/A |
| | Low | 9 | 4 | 4.7 | 25.6 | N/A |
| IL-16 | High | 9 | 1965 | 2.3 | 19.8 | N/A |
| | Mid | 9 | 166 | 1.7 | 23.7 | N/A |
| | Low | 9 | 19 | 5.2 | 25.8 | N/A |
| IL-17A | High | 9 | 2662 | 5.3 | 20.0 | N/A |
| | Mid | 9 | 256 | 4.3 | 18.1 | N/A |
| | Low | 9 | 25 | 4.3 | 16.2 | N/A |
| TNFβ | High | 9 | 298 | 4.4 | 27.2 | N/A |
| | Mid | 9 | 30 | 3.0 | 27.7 | N/A |
| | Low | 9 | 3 | 3.0 | 25.0 | N/A |
| VEGF | High | 9 | 766 | 2.6 | 18.3 | N/A |
| | Mid | 9 | 64 | 2.7 | 12.1 | N/A |
| | Low | 9 | 8 | 5.9 | 5.4 | N/A |

**Table 16. Panel 3**

| | Control | Runs | Average Conc. (pg/mL) | Average Intra-run %CV | Inter-run %CV | Inter-lot %CV |
|---|---|---|---|---|---|---|
| Eotaxin | High | 9 | 2889 | 2.3 | 5.9 | |
| | Mid | 9 | 312 | 2.3 | 4.5 | |
| | Low | 9 | 34 | 12.8 | 7.4 | |
| MIP-1β | High | 9 | 2071 | 3.0 | 5.3 | |
| | Mid | 9 | 222 | 1.4 | 4.8 | |
| | Low | 9 | 20 | 4.4 | 5.7 | |
| Eotaxin-3 | High | 9 | 13931 | 2.1 | 6.5 | |
| | Mid | 9 | 1025 | 4.4 | 5.0 | |
| | Low | 9 | 131 | 7.0 | 9.5 | |
| TARC | High | 9 | 3240 | 4.6 | 6.2 | |
| | Mid | 9 | 332 | 3.0 | 3.6 | |
| | Low | 9 | 34 | 4.3 | 8.4 | |
| IP-10 | High | 9 | 5858 | 9.3 | 14.4 | |
| | Mid | 9 | 435 | 3.5 | 5.3 | |
| | Low | 9 | 51 | 4.2 | 10.2 | |
| MIP-1α | High | 9 | 2253 | 1.8 | 4.0 | |
| | Mid | 9 | 219 | 1.6 | 54 | |
| | Low | 9 | 26 | 10.4 | 8.8 | |
| IL-8 | High | 9 | 125226 | 2.7 | 10.7 | |
| | Mid | 9 | 44664 | 1.3 | 13.2 | |
| | Low | 9 | 4830 | 2.1 | 18.8 | |
| MCP-1 | High | 9 | 1066 | 5.1 | 8.4 | |
| | Mid | 9 | 113 | 5.0 | 4.4 | |
| | Low | 9 | 11 | 6.3 | 6.2 | |
| MDC | High | 9 | 25521 | 46 | 5.3 | |
| | Mid | 9 | 1548 | 4.1 | 5.2 | |
| | Low | 9 | 197 | 4.1 | 8.3 | |
| MCP-4 | High | 9 | 1349 | 3.0 | 5.3 | |
| | Mid | 9 | 170 | 2.5 | 5.1 | |
| | Low | 9 | 14 | 12.7 | 14.6 | |

**Table 17. Panel 5**

| | Control | Runs | Average Conc. (pg/mL) | Average Intra-run %CV | Inter-run %CV | Inter-lot%CV |
|---|---|---|---|---|---|---|
| IFNγ | High | 9 | 305 | 2.1 | 4.5 | 4.8 |
| | Mid | 9 | 722 | 2.2 | 9.6 | |
| | Low | 9 | 23 | 13 | 6.6 | |
| IL-1β | High | 9 | 826 | 2.0 | 3.4 | |
| | Mid | 9 | 928 | 2.0 | 7.5 | |
| | Low | 9 | 53 | 1.7 | 5.2 | |
| IL-2 | High | 9 | 2092 | 2.3 | 3.8 | |
| | Mid | 9 | 2293 | 2.2 | 7.8 | |
| | Low | 9 | 80 | 2.4 | 5.6 | |
| IL-4 | High | 9 | 759 | 3.8 | 6.0 | |
| | Mid | 9 | 836 | 2.9 | 9.3 | |
| | Low | 9 | 70 | 2.4 | 6.8 | |
| IL-5 | High | 9 | 849 | 2.0 | 4.2 | |
| | Mid | 9 | 981 | 2.8 | 7.0 | |
| | Low | 9 | 36 | 2.2 | 4.8 | |
| IL-6 | High | 9 | 115 | 2.4 | 3.7 | |
| | Mid | 9 | 400 | 3.5 | 11 | |
| | Low | 9 | 26 | 2.5 | 5.4 | |
| KC/GRO | High | 9 | 776 | 3.1 | 3.4 | |
| | Mid | 9 | 752 | 2.7 | 6.2 | |
| | Low | 9 | 106 | 3.4 | 4.8 | |
| IL-10 | High | 9 | 3370 | 3.1 | 4.3 | |
| | Mid | 9 | 4167 | 3.1 | 7.6 | |
| | Low | 9 | 627 | 2.4 | 6.1 | |
| IL-12p70 | High | 9 | 7821 | 4.7 | 7.8 | |
| | Mid | 9 | 26735 | 7.0 | 9.9 | |
| | Low | 9 | 3193 | 4.5 | 12 | |
| TNFα | High | 9 | 448 | 2.5 | 5.0 | |
| | Mid | 9 | 479 | 2.1 | 7.0 | |
| | Low | 9 | 22 | 3.0 | 5.1 | |

To assess linearity in panels 1-3, normal individual human serum, EDTA plasma, heparin plasma, citrate plasma, and CSF samples from a commercial source were spiked with recombinant calibrators and diluted 2-fold, 4-fold, 8-fold, 16-fold, 32-fold, and 64-fold before testing. Normal individual human urine was spiked with recombinant calibrators and diluted 2-fold, 4-fold, 8-fold, and 16-fold. Percent recovery at each dilution was calculated by dividing the dilution adjusted calculated concentration by the expected concentration, i.e., the calculated dilution adjusted concentration at 2-fold dilution for panels 1-2 and a 4-fold dilution for panel 3 (see equation below).

To assess linearity in panel 4, normal rat serum, EDTA plasma, heparin plasma, citrate plasma, and urine samples from a commercial source were spiked with recombinant calibrators and diluted 4-fold, 8-fold, 16-fold, and 32-fold before testing. Percent recovery at each dilution was calculated by dividing the dilution adjusted calculated concentration by the expected concentration, i.e., the calculated dilution adjusted concentration at 4-fold dilution.

To assess linearity in panel 5, normal mouse serum, EDTA plasma, heparin plasma, citrate plasma, and urine samples from a commercial source were spiked with recombinant calibrators and diluted 2-fold, 4-fold, 8-fold, 16-fold, 32-fold, and 64-fold before testing. Percent recovery at each dilution was calculated by dividing the dilution adjusted calculated concentration by the expected concentration, i.e., the calculated dilution adjusted concentration at 2-fold dilution.

The average percent recovery shown below is based on samples within the quantitative range of the assay. $% Recovery = \left(\frac{Measured}{Expected}\right) ∗ 100$

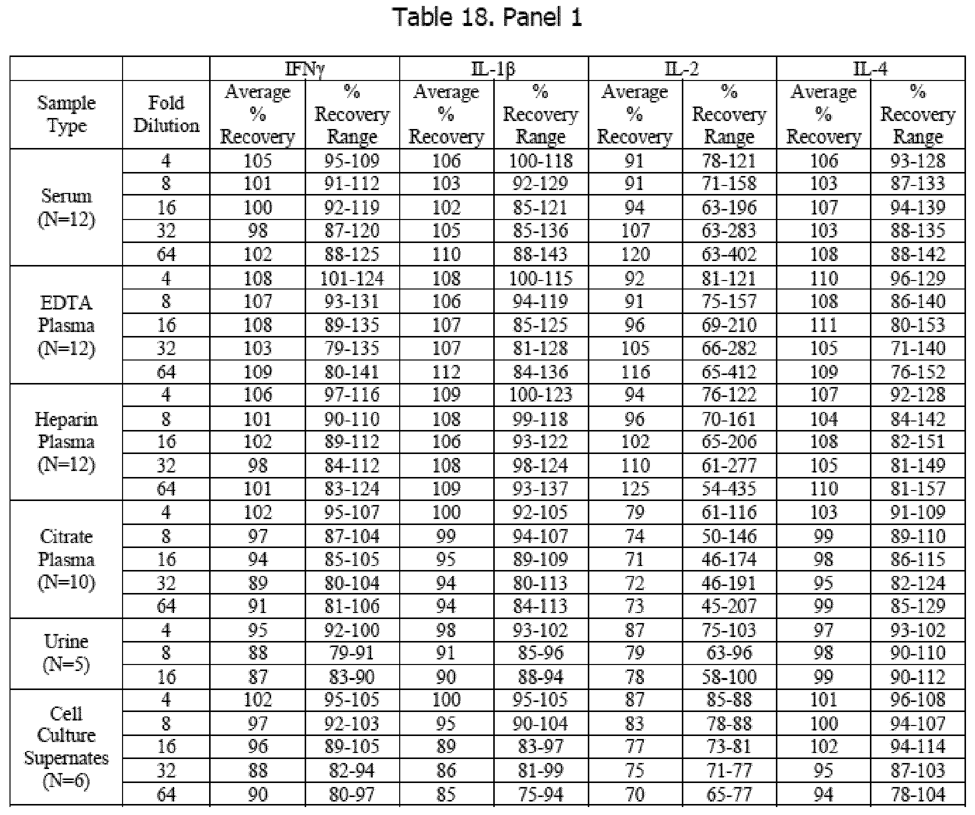

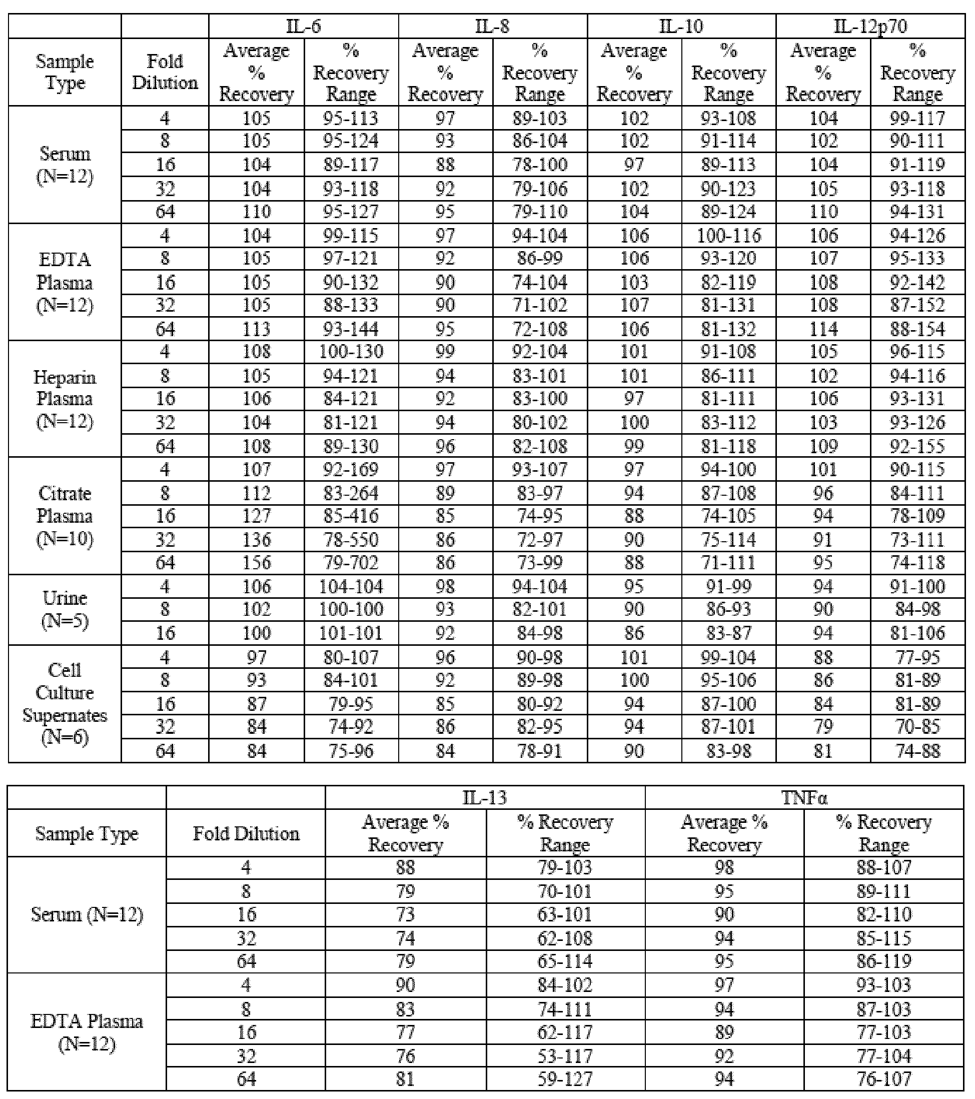

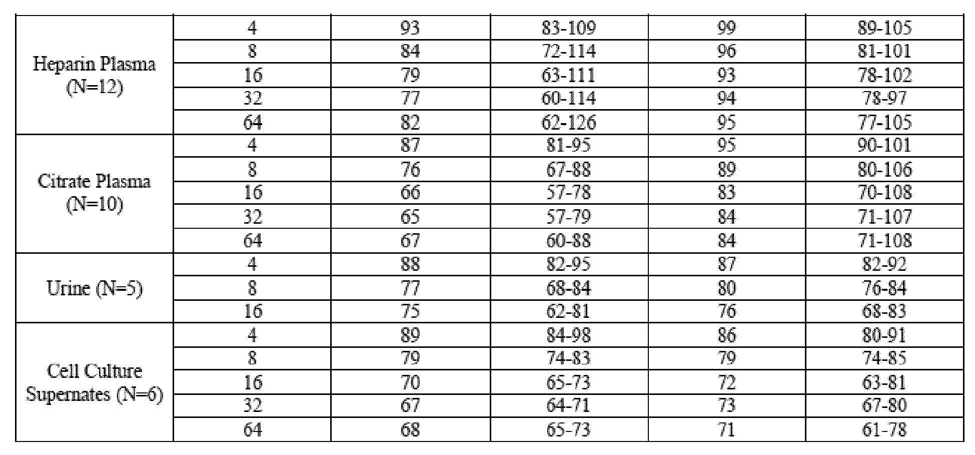

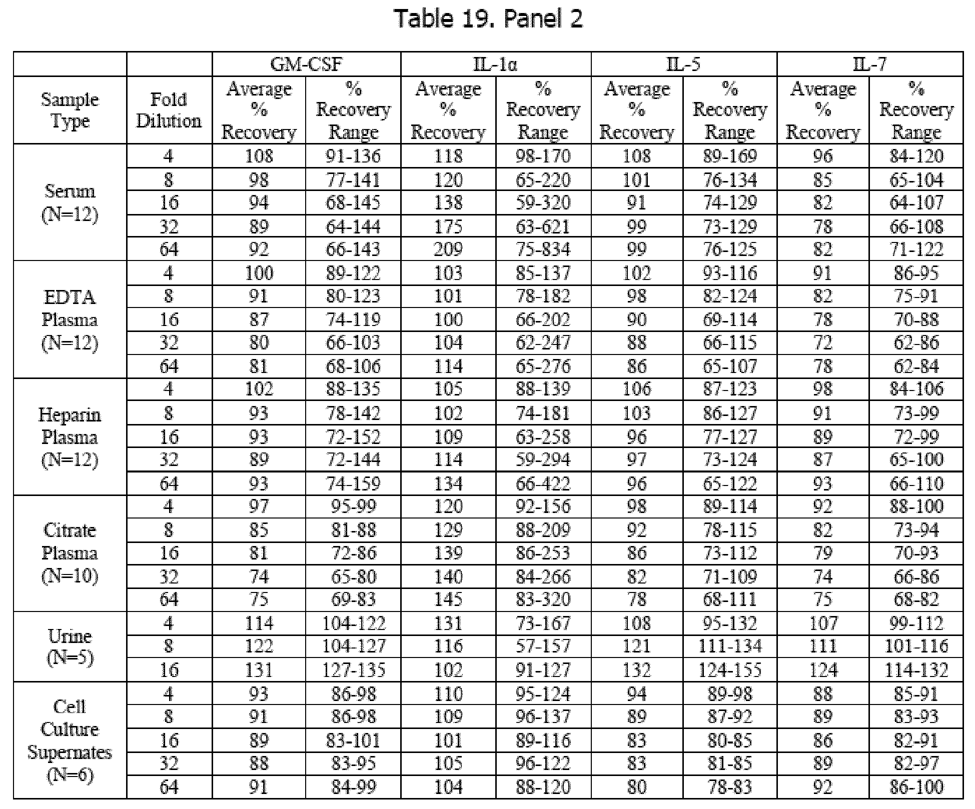

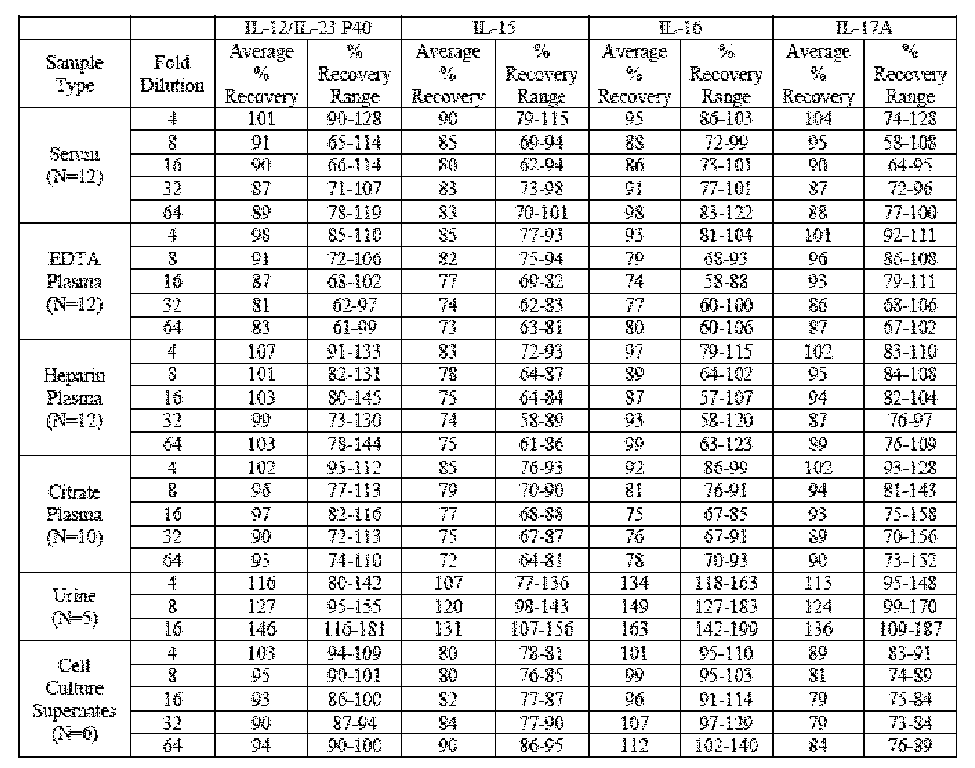

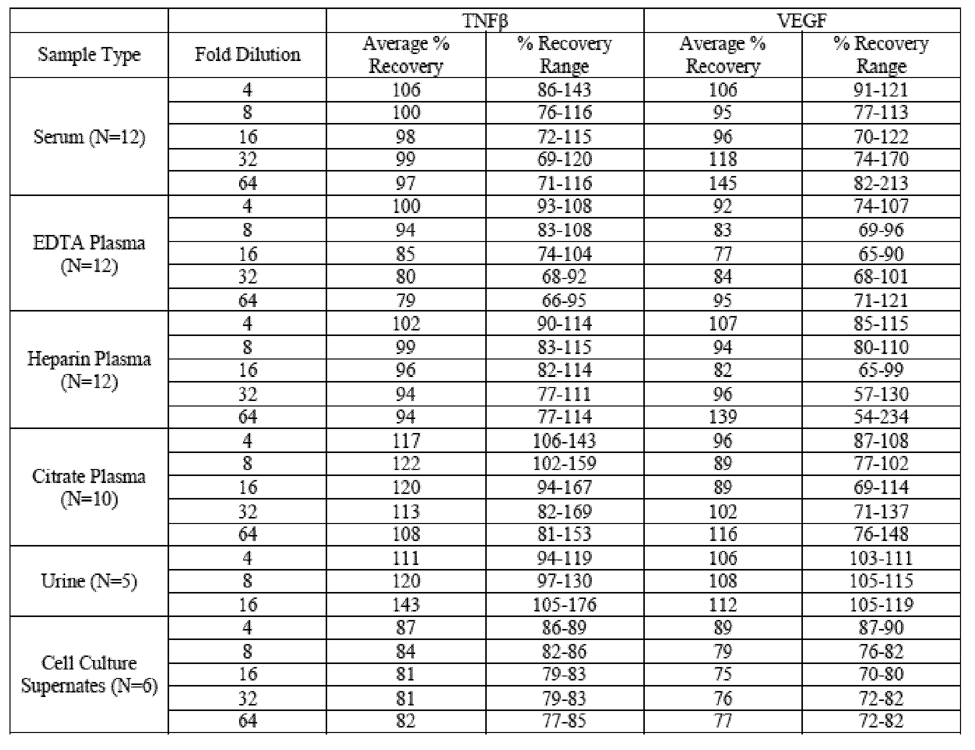

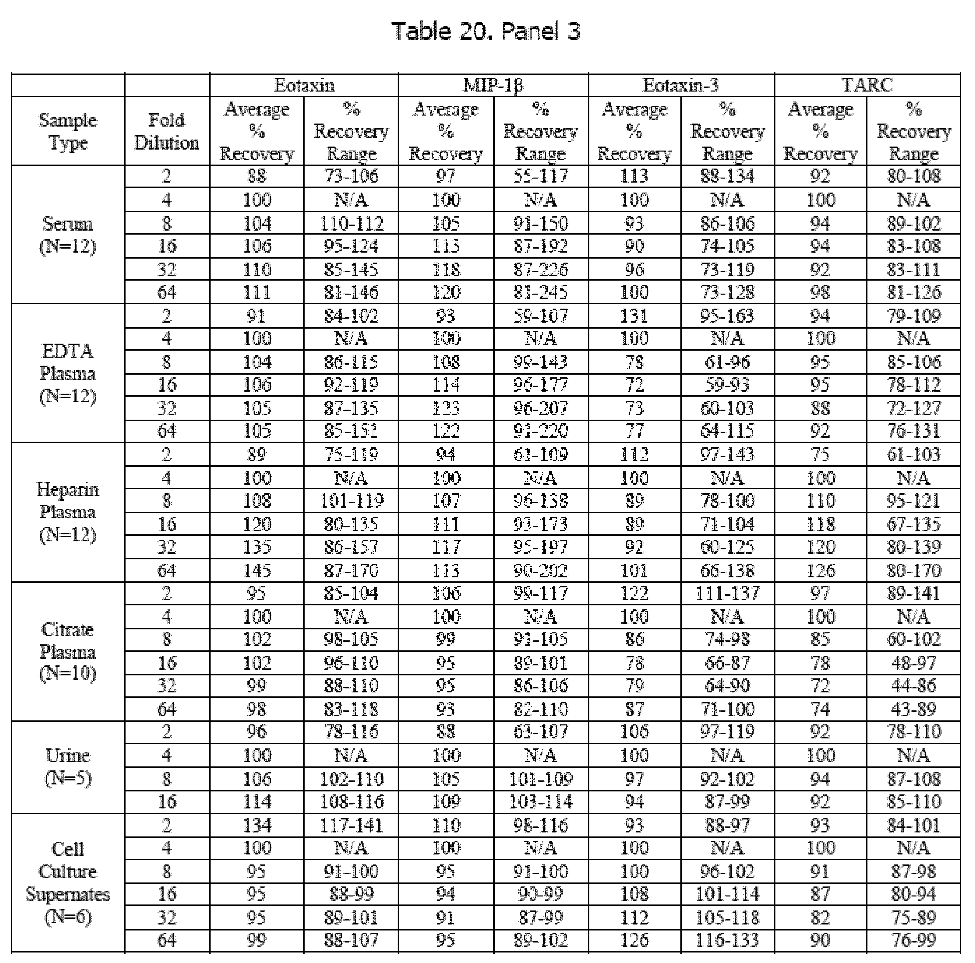

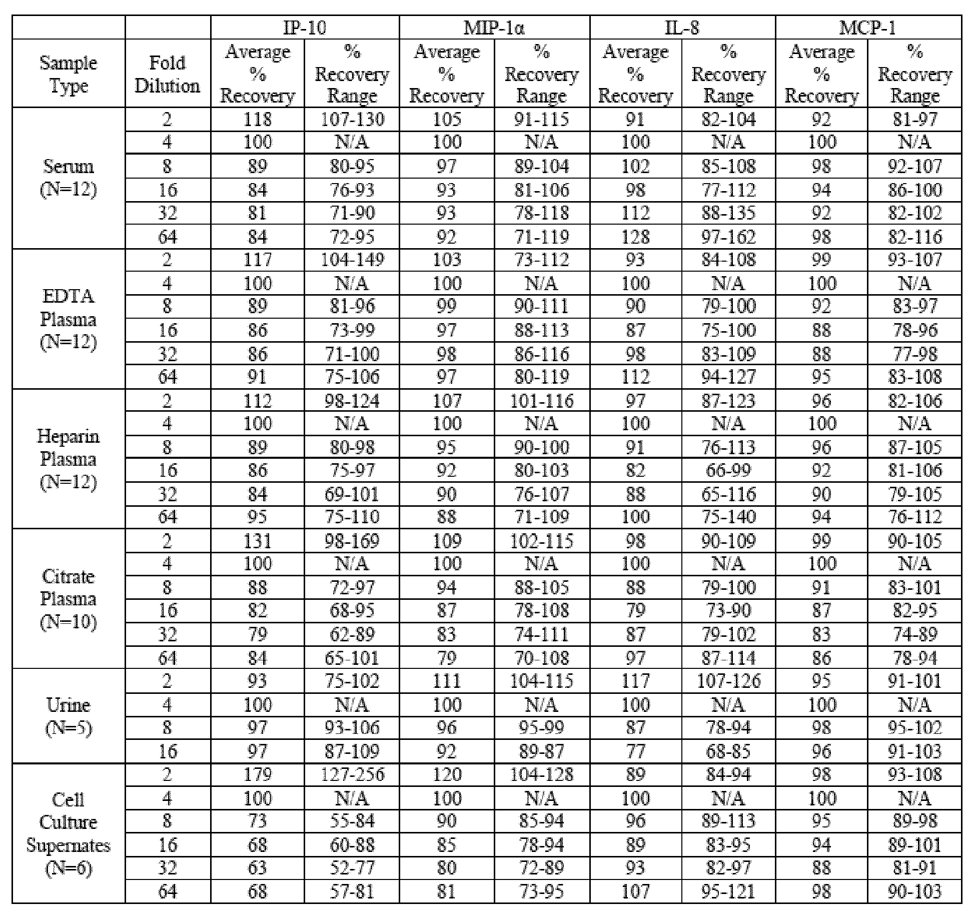

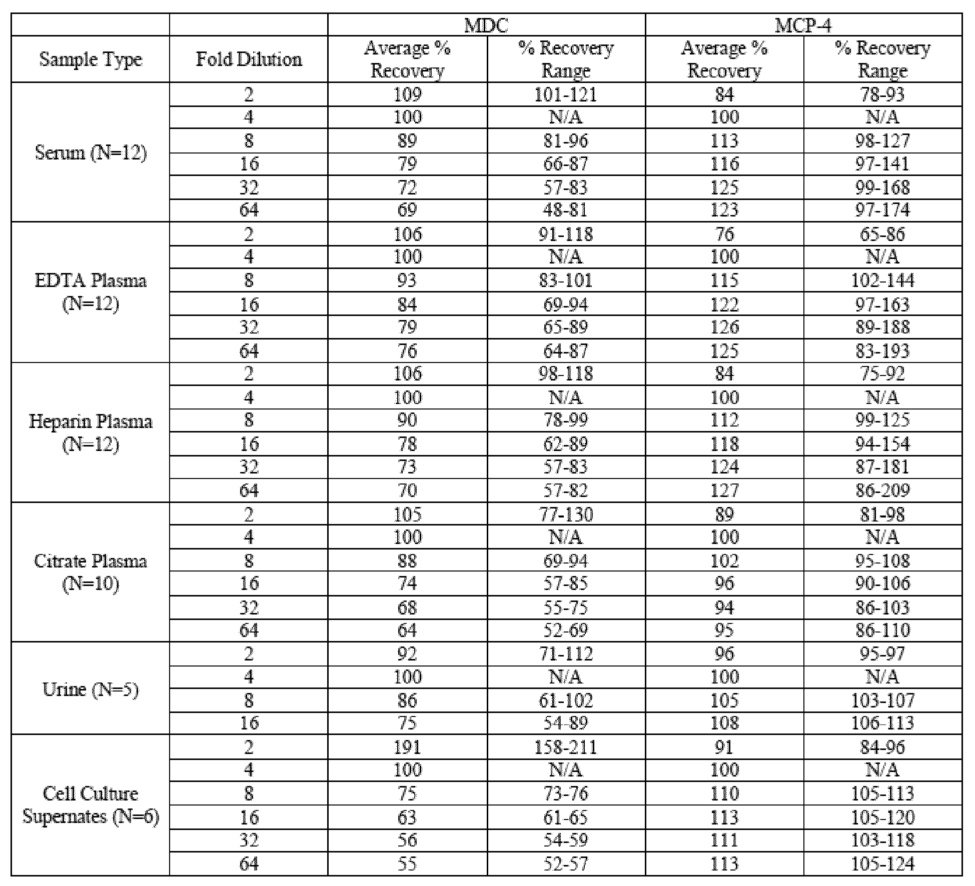

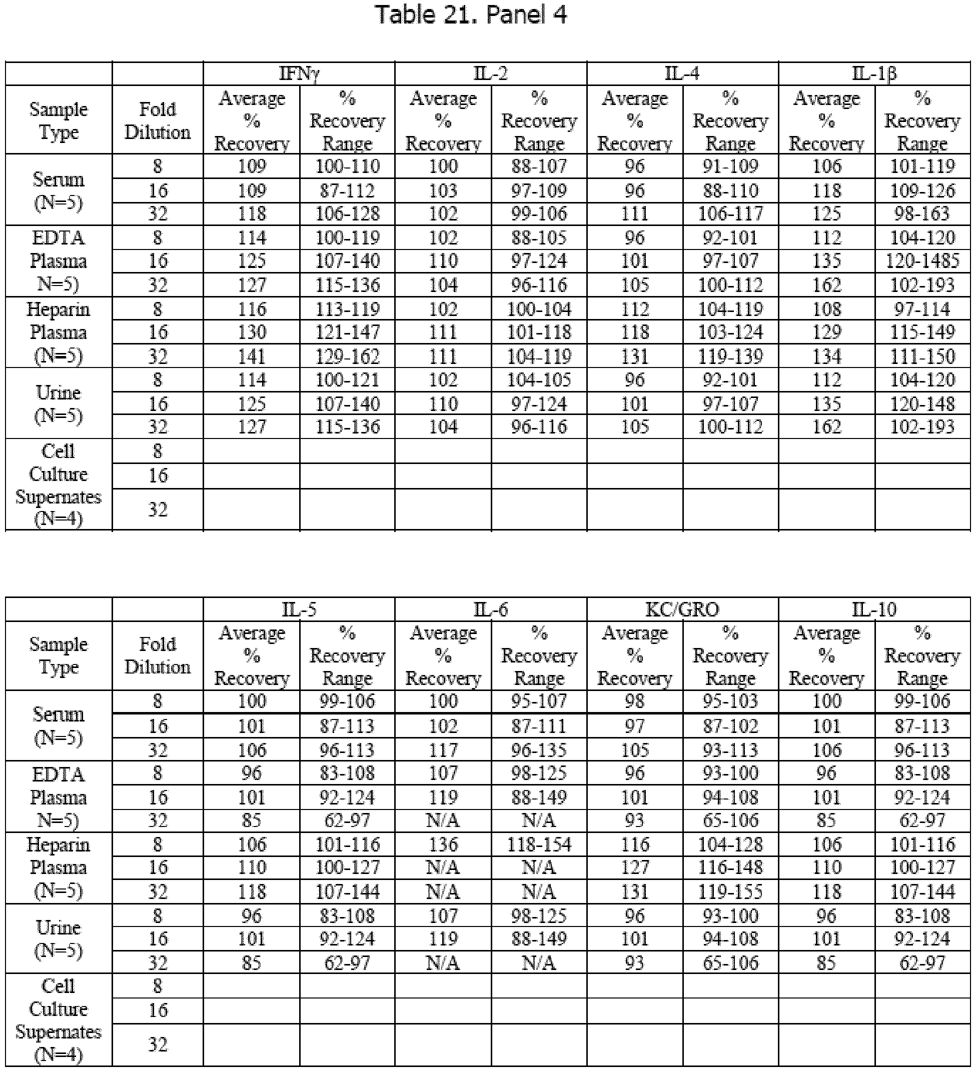

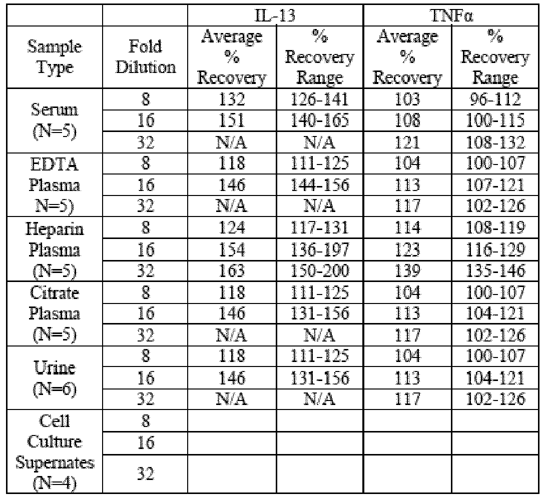

**Table 22. Panel 5**

| | | IFNγ | | IL-1β | | IL-2 | | IL-4 | |
|---|---|---|---|---|---|---|---|---|---|
| Sample Type | Fold Dilution | Average % Recovery | % Recovery Range | Average % Recovery | % Recovery Range | Average % Recovery | % Recovery Range | Average % Recovery | % Recovery Range |
| Serum (N=8) | 4 | 107 | 94-115 | 108 | 99-122 | 106 | 98-115 | 144 | 128-154 |
| | 8 | 99 | 86-107 | 96 | 82-102 | 108 | 88-110 | 156 | 136-169 |
| | 16 | 98 | 86-108 | 98 | 88-103 | 94 | 82-110 | 170 | 145-182 |
| | 32 | 95 | 82-101 | 99 | 87-109 | 96 | 78-110 | 171 | 139-190 |
| | 64 | 98 | 82-109 | 100 | 88-109 | 97 | 83-113 | 184 | 144-204 |
| EDTA Plasma (N=8) | 4 | 101 | 90-112 | 103 | 98-108 | 95 | 92-106 | 123 | 114-129 |
| | 8 | 100 | 80-115 | 101 | 86-111 | 94 | 84-104 | 134 | 127-149 |
| | 16 | 106 | 78-156 | 100 | 85-112 | 90 | 78-105 | 147 | 138-187 |
| | 32 | 103 | 79-124 | 100 | 83-115 | 93 | 82-108 | 149 | 133-177 |
| | 64 | 114 | 80-157 | 104 | 79-125 | 94 | 79-100 | 160 | 143-158 |
| Heparin Plasma (N=8) | 4 | 105 | 82-122 | 106 | 100-112 | 101 | 94-112 | 134 | 124-139 |
| | 8 | 98 | 81-123 | 102 | 97-100 | 95 | 90-104 | 147 | 139-152 |
| | 16 | 107 | 91-171 | 100 | 93-111 | 92 | 83-107 | 162 | 153-179 |
| | 32 | 103 | 86-181 | 100 | 92-111 | 95 | 81-100 | 159 | 129-174 |
| | 64 | 106 | 87-147 | 101 | 94-115 | 93 | 78-108 | 174 | 154-194 |
| Citrate Plasma (N=8) | 4 | 99 | 83-109 | 103 | 95-109 | 102 | 94-111 | 120 | 105-131 |
| | 8 | 91 | 79-100 | 95 | 85-102 | 94 | 89-103 | 125 | 112-137 |
| | 16 | 88 | 71-103 | 92 | 83-101 | 90 | 85-99 | 133 | 112-149 |
| | 32 | 83 | 71-96 | 89 | 75-104 | 97 | 74-97 | 129 | 105-147 |
| | 64 | 86 | 74-101 | 91 | 73-108 | 97 | 74-98 | 134 | 111-155 |
| Urine (N=6) | 4 | 99 | 93-104 | 104 | 93-110 | 105 | 97-115 | 122 | 110-128 |
| | 8 | 96 | 97-104 | 102 | 93-111 | 103 | 95-119 | 136 | 129-143 |
| | 16 | 96 | 99-101 | 99 | 81-107 | 100 | 94-118 | 149 | 145-153 |
| | 32 | 94 | 90-100 | 101 | 94-112 | 103 | 95-124 | 151 | 146-157 |
| | 64 | 97 | 96-103 | 102 | 86-112 | 103 | 91-122 | 166 | 160-169 |
| Cell Culture Supernates (N=4) | 4 | 103 | 100-105 | 105 | 101-108 | 96 | 93-100 | 112 | 108-118 |
| | 8 | 98 | 96-99 | 102 | 99-107 | 92 | 89-95 | 112 | 108-115 |
| | 16 | 100 | 95-104 | 102 | 99-108 | 88 | 87-90 | 115 | 112-122 |
| | 32 | 95 | 94-97 | 101 | 97-106 | 92 | 91-93 | 112 | 109-115 |
| | 64 | 100 | 95-105 | 104 | 101-107 | 93 | 87-96 | 119 | 111-125 |

| | | IL-5 | | IL-6 | | KC/GRO | | IL-10 | |
|---|---|---|---|---|---|---|---|---|---|
| Sample Type | Fold Dilution | Average % Recovery | % Recovery Range | Average % Recovery | % Recovery Range | Average % Recovery | % Recovery Range | Average % Recovery | % Recovery Range |
| Serum (N=8) | 4 | 106 | 92-129 | 98 | 87-109 | 118 | 105-154 | 120 | 105-142 |
| | 8 | 101 | 85-124 | 90 | 72-102 | 110 | 96-146 | 117 | 102-135 |
| | 16 | 99 | 84-126 | 90 | 74-108 | 106 | 95-136 | 119 | 105-136 |
| | 32 | 94 | 83-116 | 94 | 73-112 | 111 | 98-145 | 117 | 101-143 |
| | 64 | 100 | 86-124 | 98 | 74-117 | 118 | 103-147 | 126 | 107-159 |
| EDTA Plasma (N=8) | 4 | 100 | 90-107 | 97 | 83-95 | 91 | 83-95 | 103 | 90-117 |
| | 8 | 98 | 79-110 | 91 | 72-93 | 85 | 72-93 | 101 | 90-₁18 |
| | 16 | 95 | 80-114 | 88 | 64-90 | 82 | 84-90 | 102 | 94-123 |
| | 32 | 99 | 79-120 | 91 | 88-106 | 87 | 88-106 | 99 | 85-121 |
| | 64 | 105 | 82-133 | 95 | 72-112 | 90 | 72-112 | 102 | 88-127 |
| | 4 | 100 | 89-113 | 95 | 76-111 | 93 | 76-111 | 111 | 103-127 |
| | 8 | 95 | 87-106 | 98 | 88-115 | 90 | 88-115 | 107 | 94-122 |
| | | | | | | | | | |
| Heparin Plasma (N=8) | 16 | 93 | 86-110 | 97 | 85-118 | 88 | 85-118 | 109 | 95-124 |
| | 32 | 93 | 78-121 | 101 | 85-122 | 88 | 85-122 | 105 | 90-129 |
| | 64 | 95 | 81-131 | 104 | 84-125 | 88 | 84-125 | 111 | 92-131 |
| Citrate Plasma (N=8) | 4 | 97 | 94-106 | 91 | 86-99 | 90 | 77-99 | 99 | 84-106 |
| | 8 | 87 | 76-97 | 83 | 72-97 | 79 | 64-94 | 89 | 75-99 |
| | 16 | 83 | 70-102 | 79 | 67-94 | 73 | 59-86 | 87 | 73-101 |
| | 32 | 83 | 66-102 | 80 | 65-99 | 71 | 53-91 | 83 | 71-90 |
| | 64 | 83 | 50-107 | 82 | 60-102 | 74 | 54-91 | 88 | 76-103 |
| Urine (N=6) | 4 | 98 | 84-114 | 99 | 95-104 | 85 | 70-95 | 103 | 87-114 |
| | 8 | 97 | 80-115 | 94 | 83-106 | 70 | 59-79 | 98 | 84-109 |
| | 16 | 91 | 75-106 | 93 | 81-101 | 64 | 53-75 | 94 | 74-111 |
| | 32 | 93 | 82-106 | 96 | 85-105 | 63 | 49-77 | 91 | 82-106 |
| | 64 | 95 | 91-105 | 101 | 92-108 | 64 | 50-78 | 95 | 85-108 |
| Cell Culture Supernates (N=4) | 4 | 96 | 94-98 | 93 | 91-94 | 76 | 72-80 | 93 | 87-100 |
| | 8 | 93 | 92-97 | 90 | 88-92 | 69 | 63-72 | 84 | 79-92 |
| | 16 | 92 | 86-96 | 88 | 83-91 | 65 | 60-69 | 84 | 77-94 |
| | 32 | 92 | 85-99 | 93 | 88-97 | 67 | 62-72 | 81 | 74⁻90 |
| | 64 | 95 | 89-100 | 96 | 90-102 | 70 | 66-76 | 85 | 77-101 |

| | | IL-12p70 | | TNFα | | | | | |
|---|---|---|---|---|---|---|---|---|---|
| Sample Type | Fold Dilution | Average % Recovery | % Recovery Range | Average % Recovery | % Recovery Range | | | | |
| Serum (N=5) | 4 | 98 | 84-112 | 111 | 100-124 | | | | |
| | 8 | 87 | 73-100 | 108 | 93-120 | | | | |
| | 16 | 81 | 65-94 | 109 | 97-123 | | | | |
| | 32 | 79 | 62-90 | 117 | 105-142 | | | | |
| | 64 | 81 | 61-93 | 123 | 107-156 | | | | |
| EDTA Plasma N=5) | 4 | 93 | 87-101 | 90 | 90-110 | | | | |
| | 8 | 85 | 78-94 | 100 | 88-111 | | | | |
| | 16 | 81 | 75-89 | 96 | 84-112 | | | | |
| | 32 | 81 | 68-92 | 102 | 85-124 | | | | |
| | 64 | 86 | 69-102 | 105 | 89-136 | | | | |
| Heparin Plasma (N=5) | 4 | 87 | 77-95 | 102 | 95-111 | | | | |
| | 8 | 77 | 86-89 | 100 | 89-109 | | | | |
| | 16 | 75 | 66-84 | 100 | 88-109 | | | | |
| | 32 | 74 | 62-87 | 105 | 89-118 | | | | |
| | 64 | 78 | 63-90 | 107 | 91-119 | | | | |
| Citrate Plasma (N=5) | 4 | 96 | 86-105 | 89 | 92-106 | | | | |
| | 8 | 84 | 68-96 | 94 | 89-97 | | | | |
| | 16 | 79 | 52-94 | 91 | 85-95 | | | | |
| | 32 | 76 | 46-98 | 92 | 89-97 | | | | |
| | 64 | 79 | 44-103 | 93 | 47-98 | | | | |
| Urine (N=6) | 4 | 100 | 96-122 | 90 | 91-107 | | | | |
| | 8 | 107 | 87-133 | 96 | 94-112 | | | | |
| | 16 | 106 | 90-132 | 97 | 95-116 | | | | |
| | 32 | 106 | 91-140 | 102 | 89-122 | | | | |
| | 64 | 113 | 101-148 | 107 | 94-130 | | | | |
| Cell Culture | 4 | 94 | 92-98 | 93 | 92-94 | | | | |
| | 8 | 87 | 85-88 | 89 | 87-93 | | | | |
| | 16 | 83 | 79-88 | 89 | 84-93 | | | | |
| | | | | | | | | | |
| Supernates (N=4) | 32 | 81 | 78-87 | 93 | 88-96 | | | | |
| | 64 | 85 | 79-93 | 96 | 92-100 | | | | |

Spike and recovery measurements of different sample types throughout the quantifiable range of the assays were evaluated. Multiple individual human serum, EDTA plasma, heparin plasma, citrate plasma, urine, and/or CSF samples from a commercial source and cell culture supernates were spiked with calibrators at three levels (high, mid, and low) and subsequently diluted two-fold.

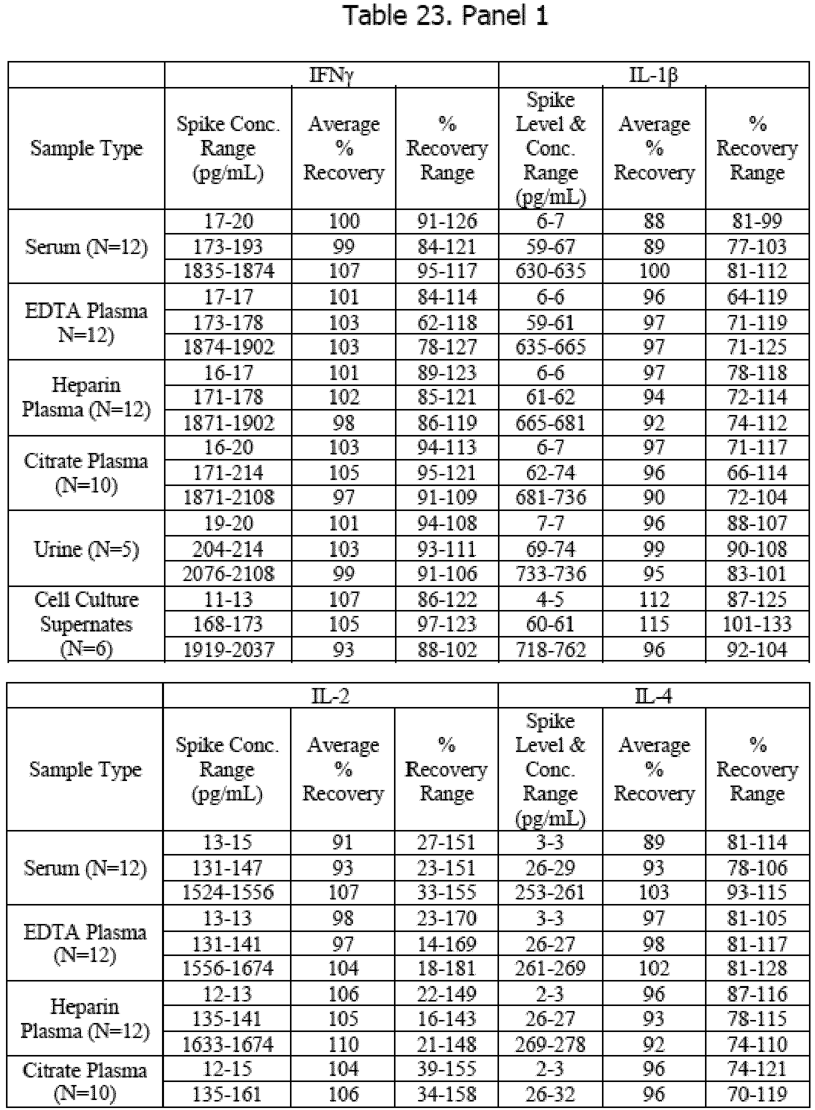

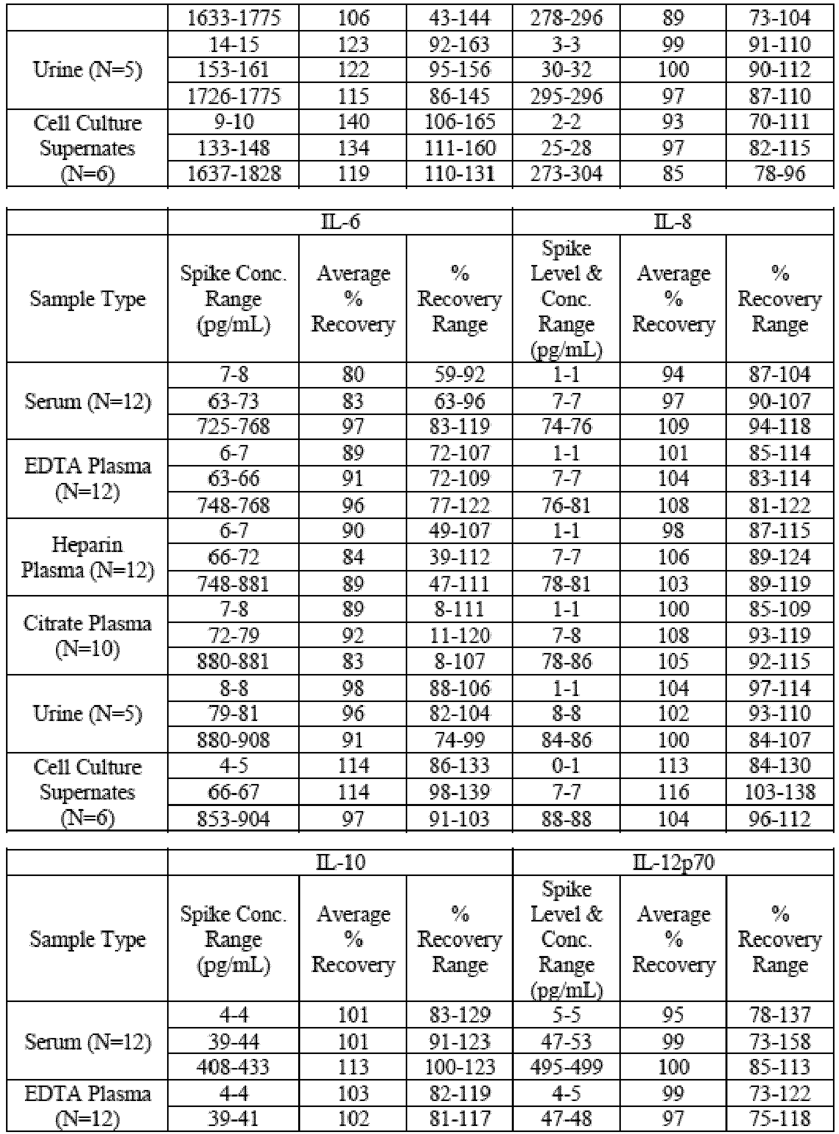

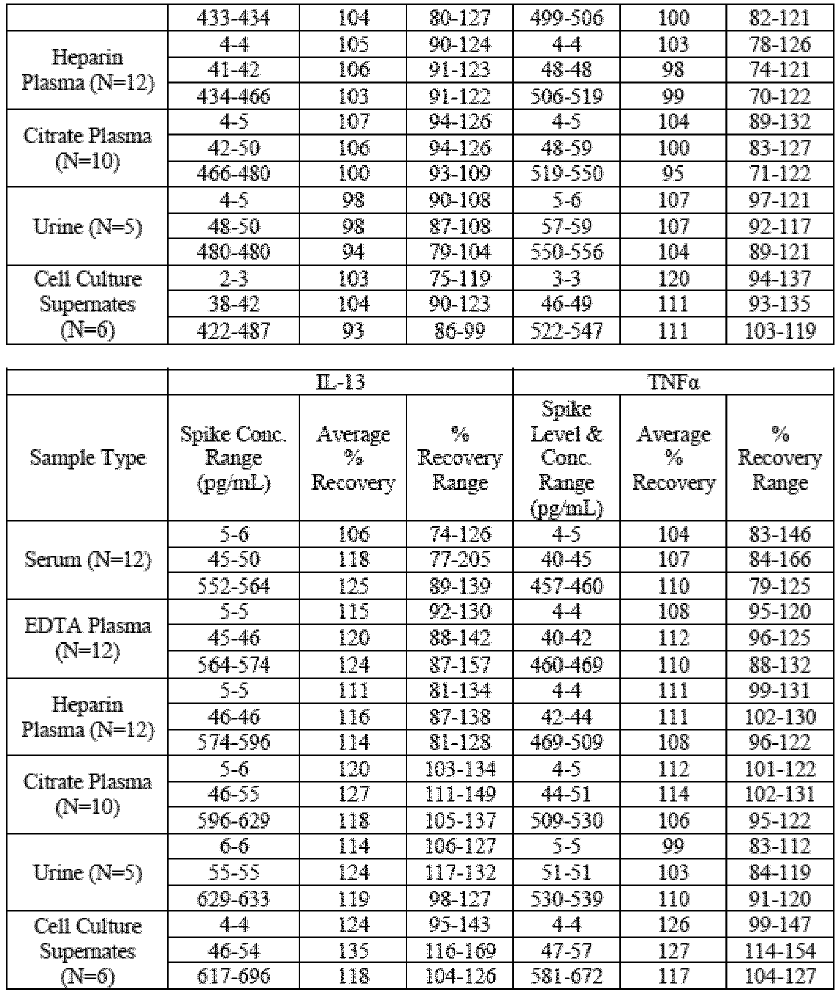

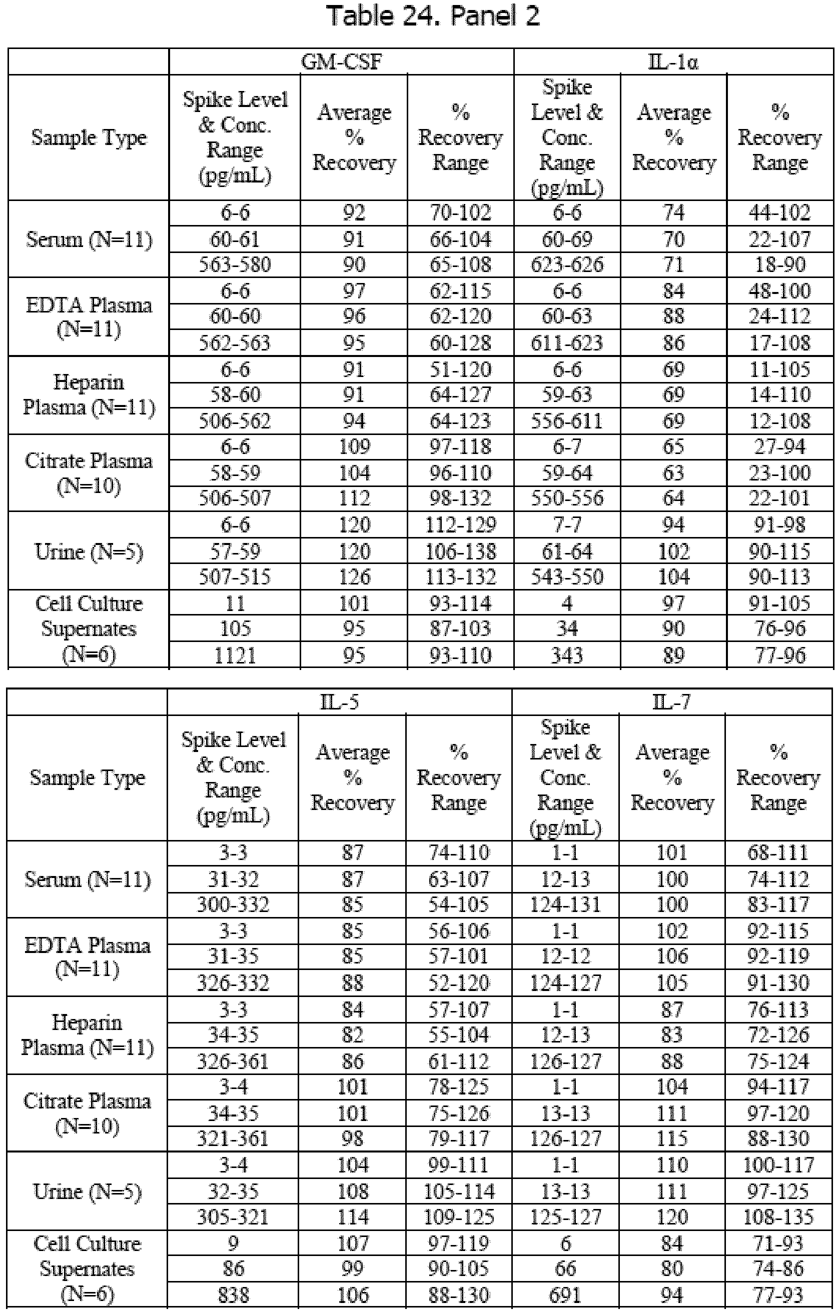

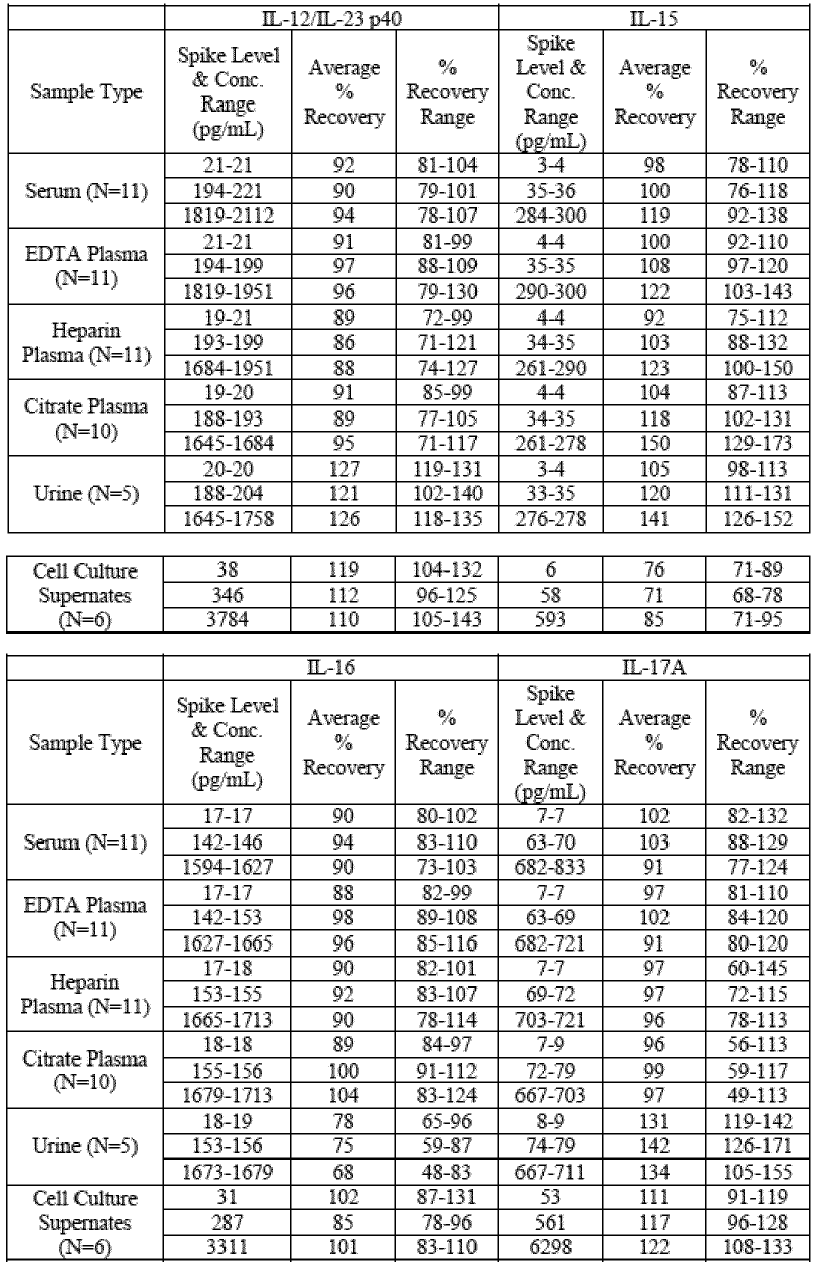

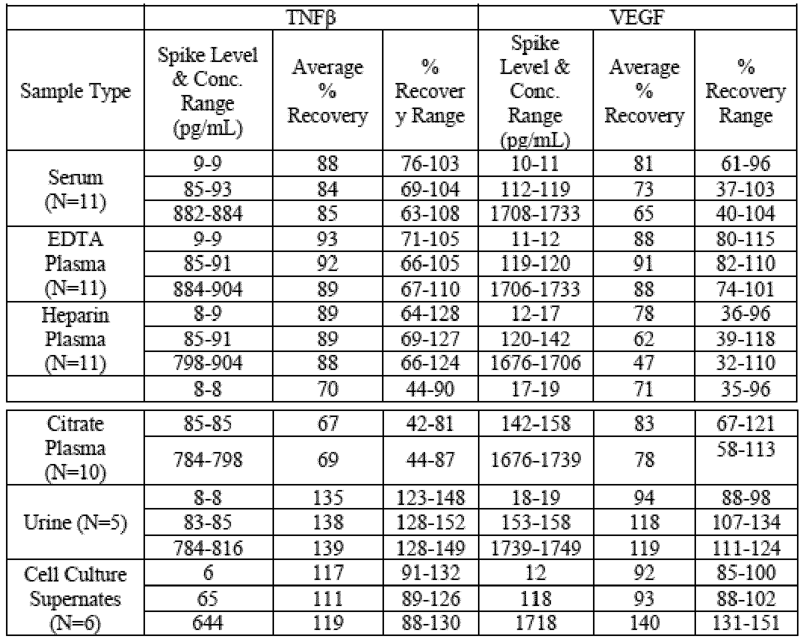

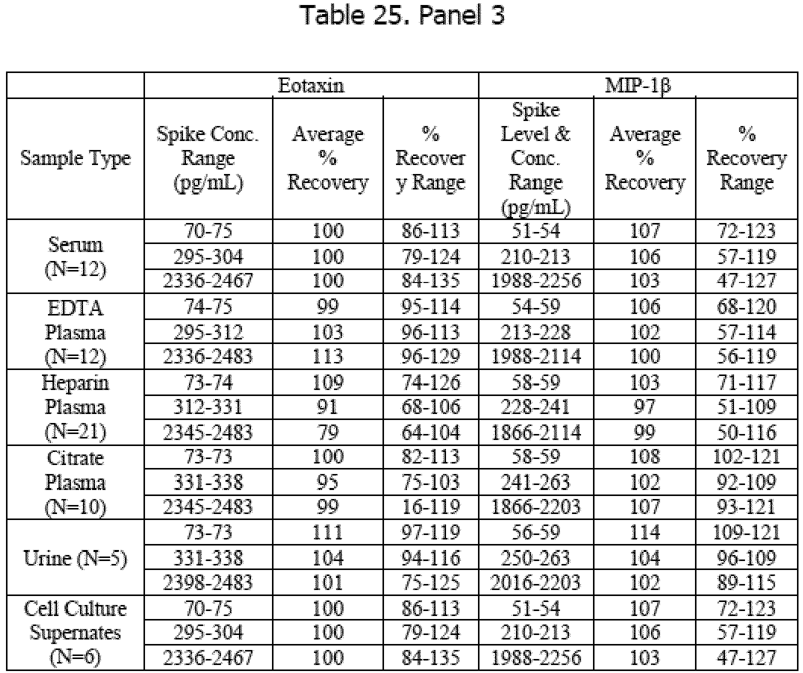

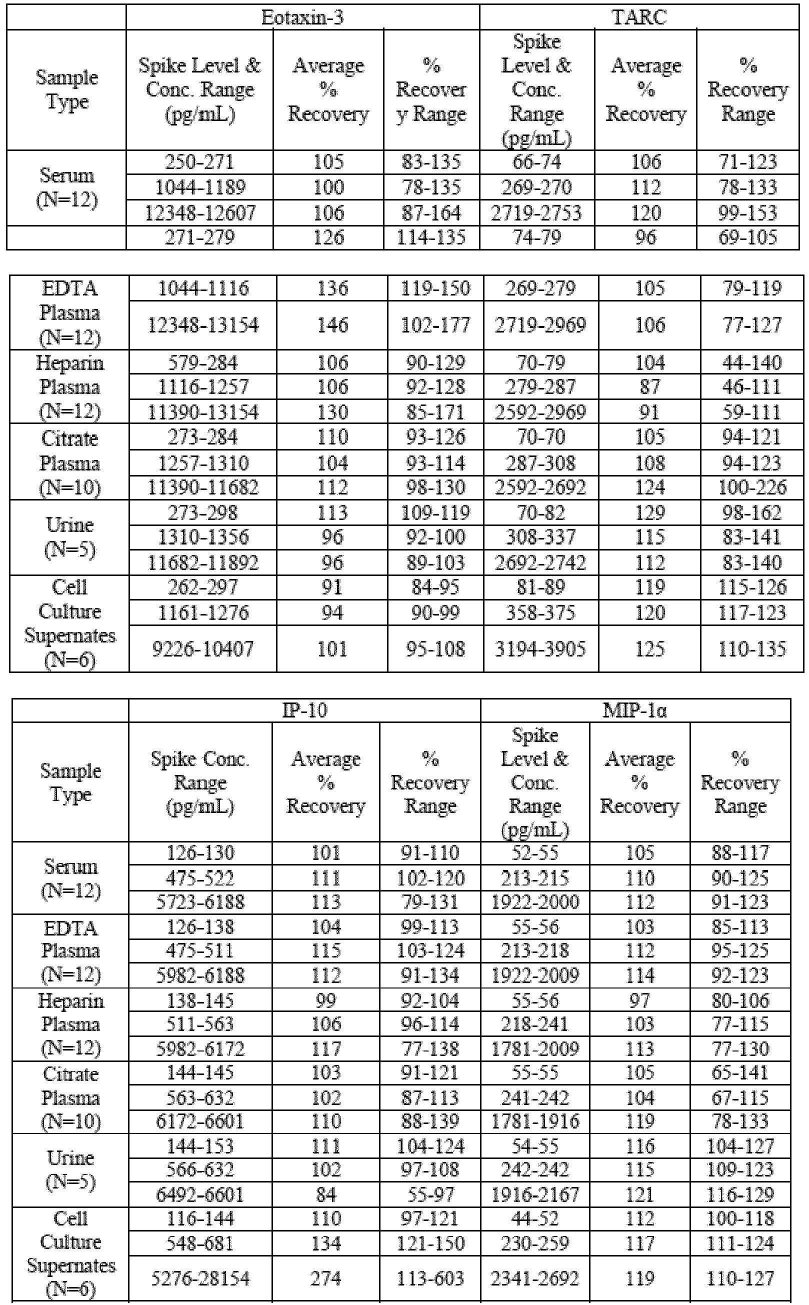

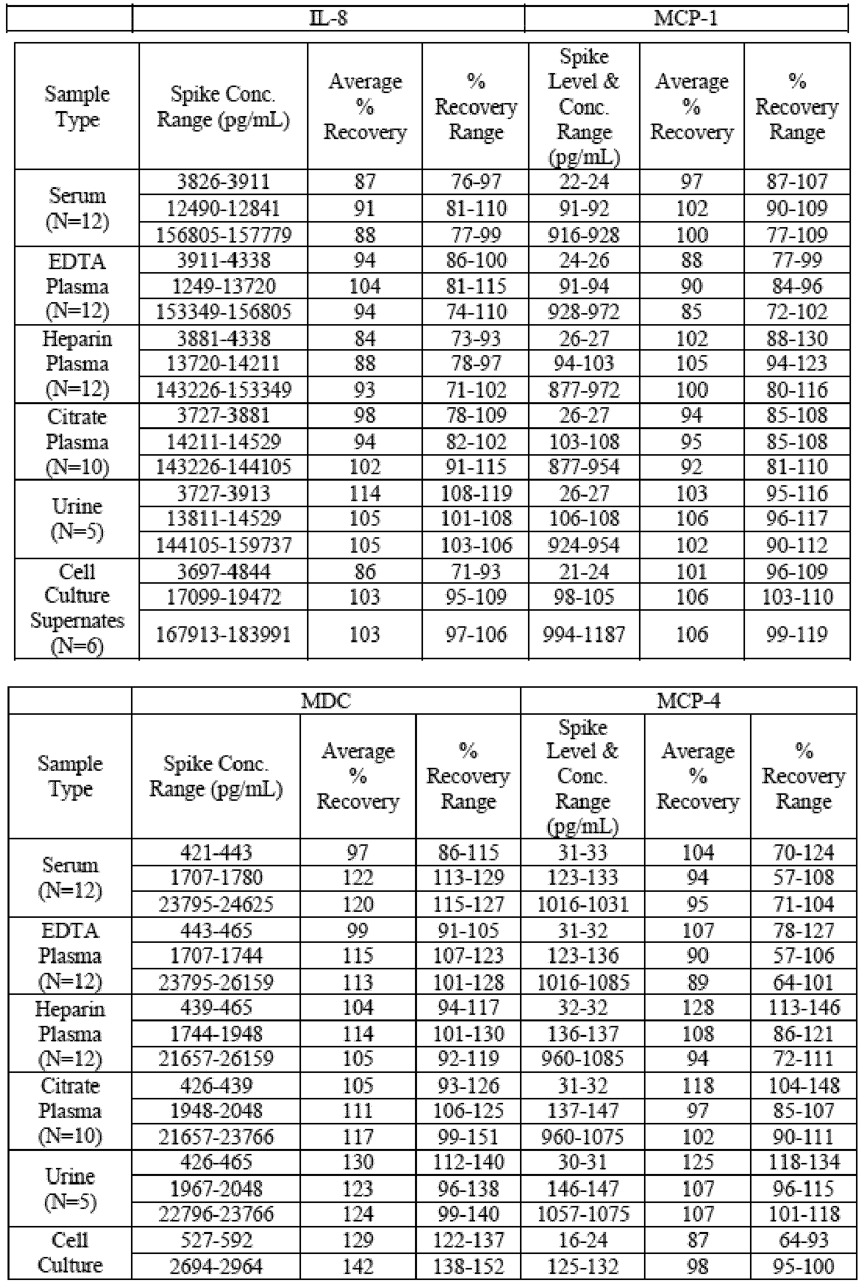

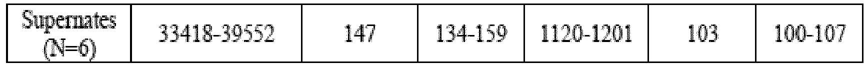

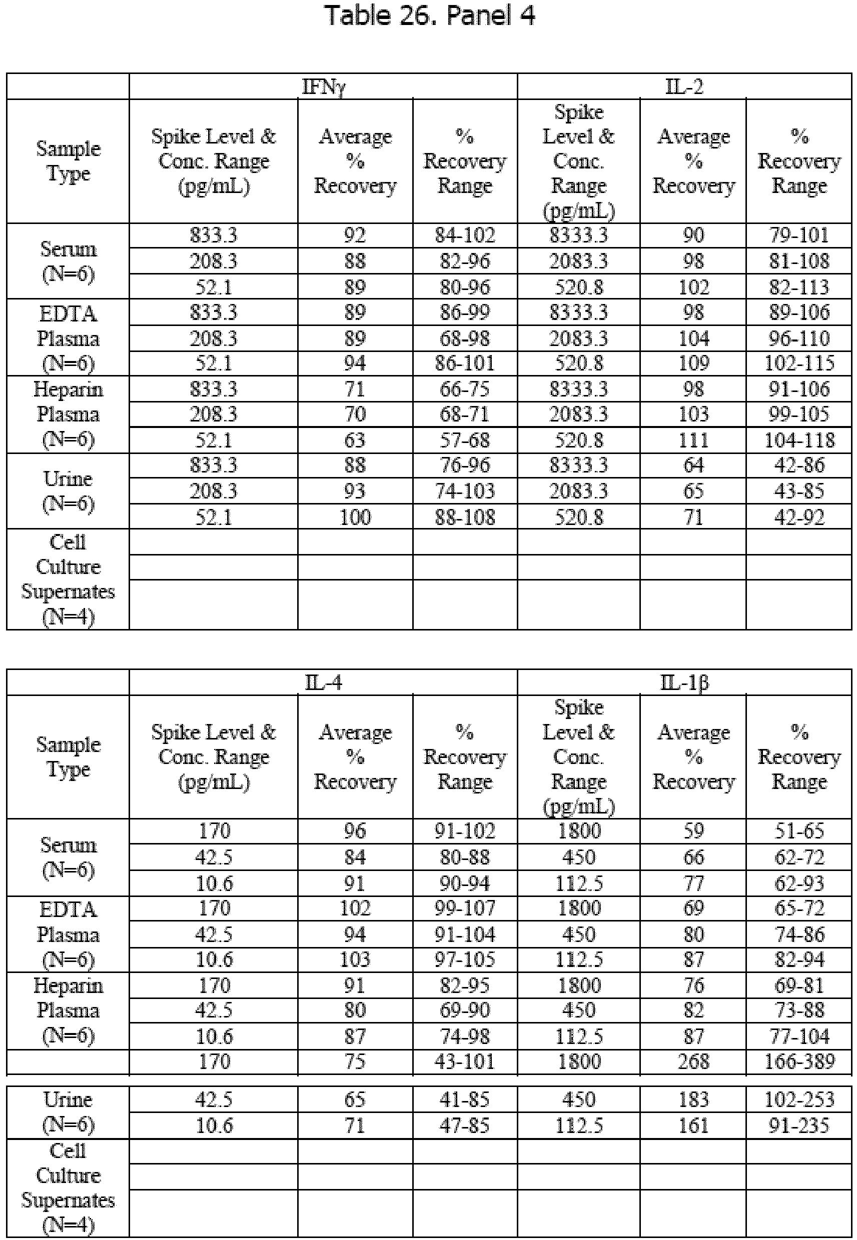

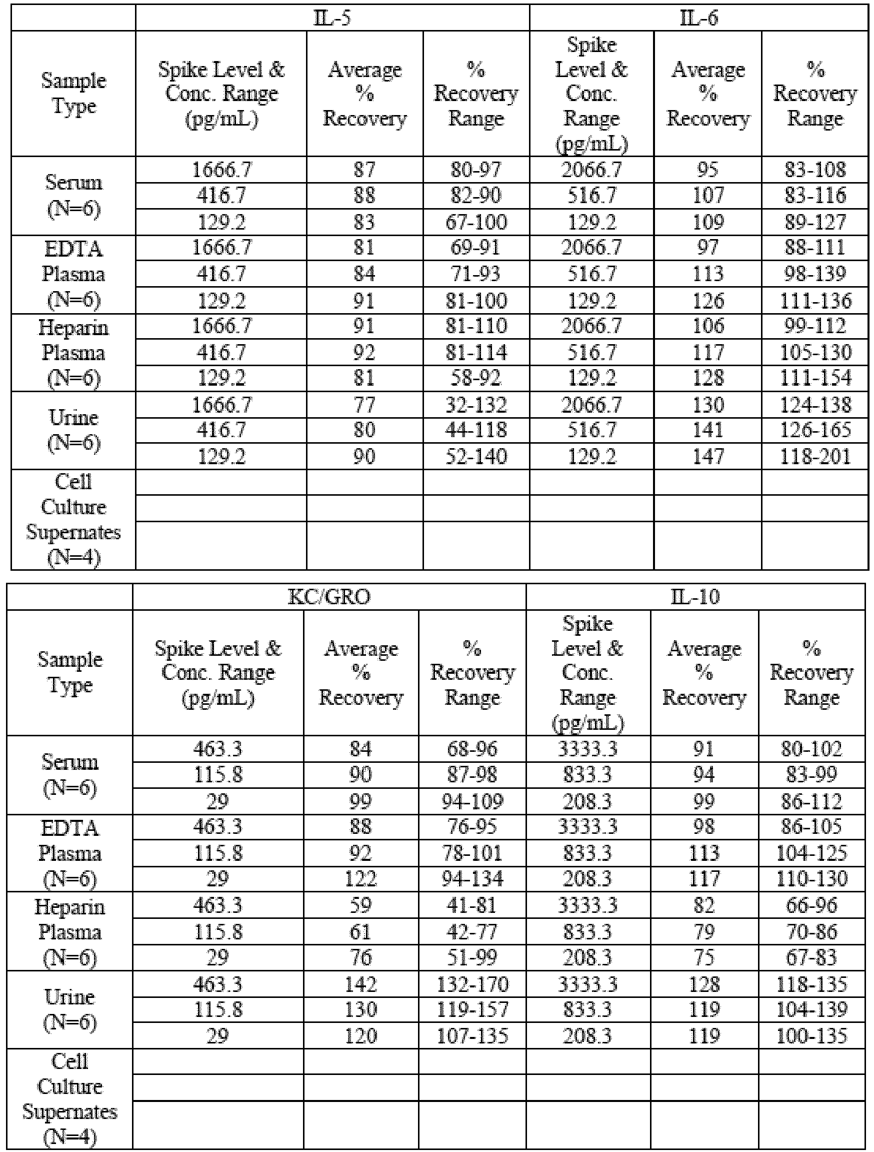

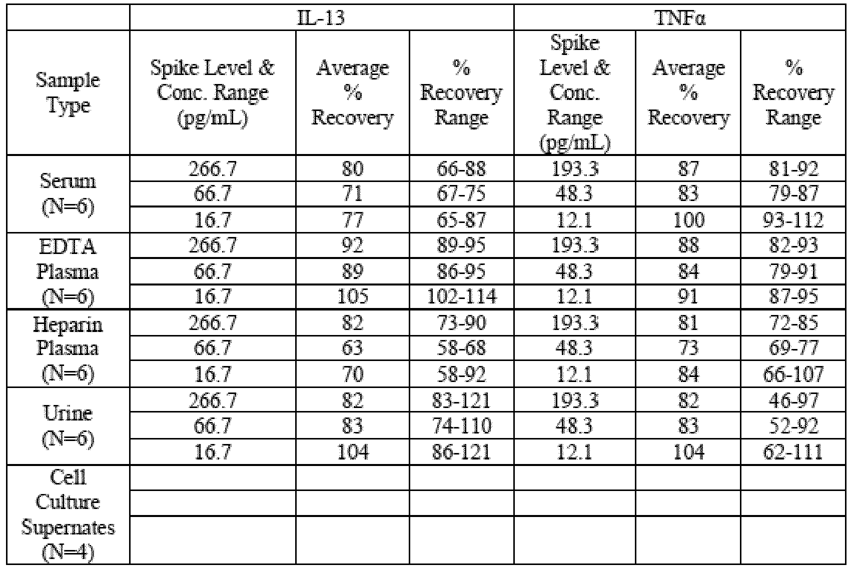

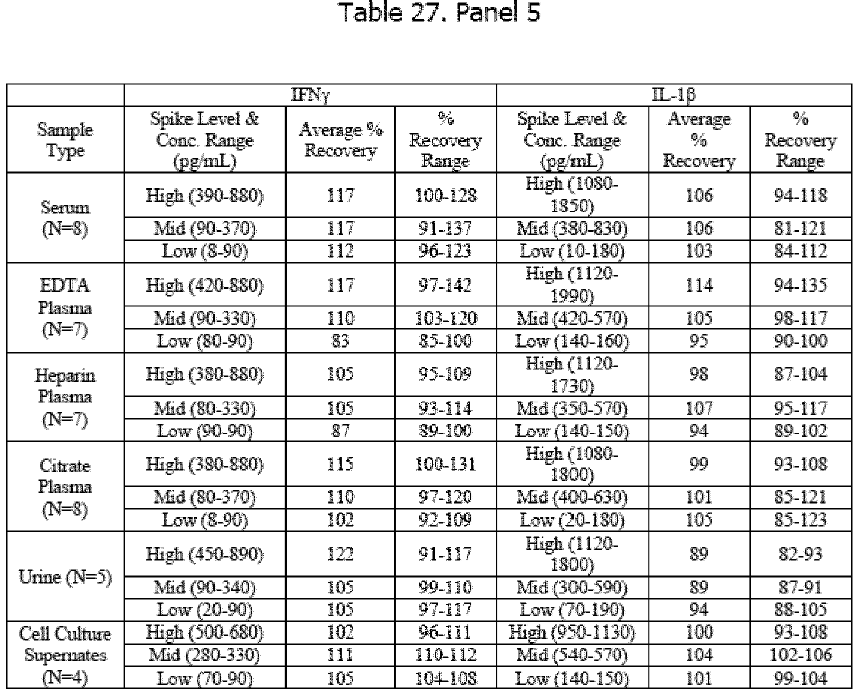

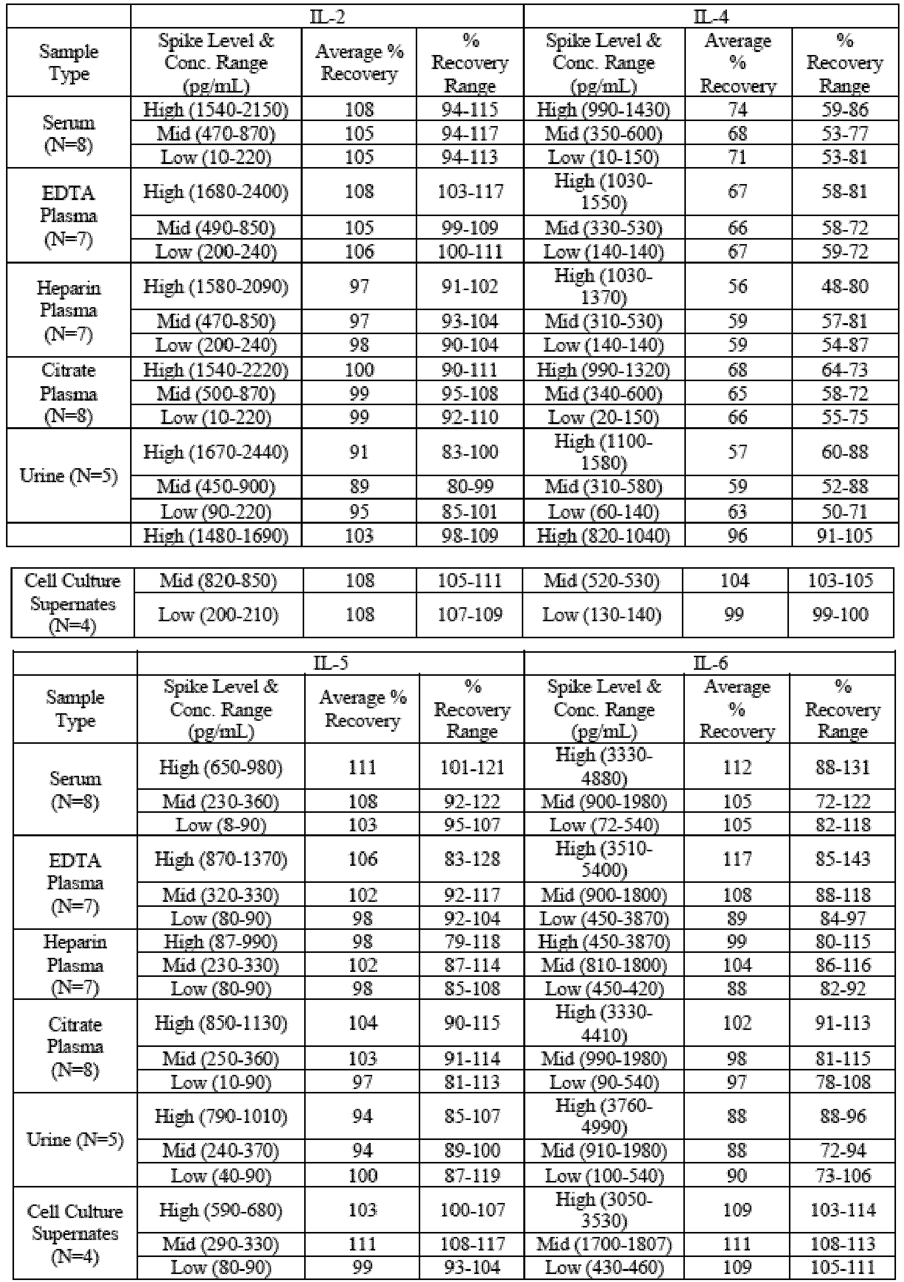

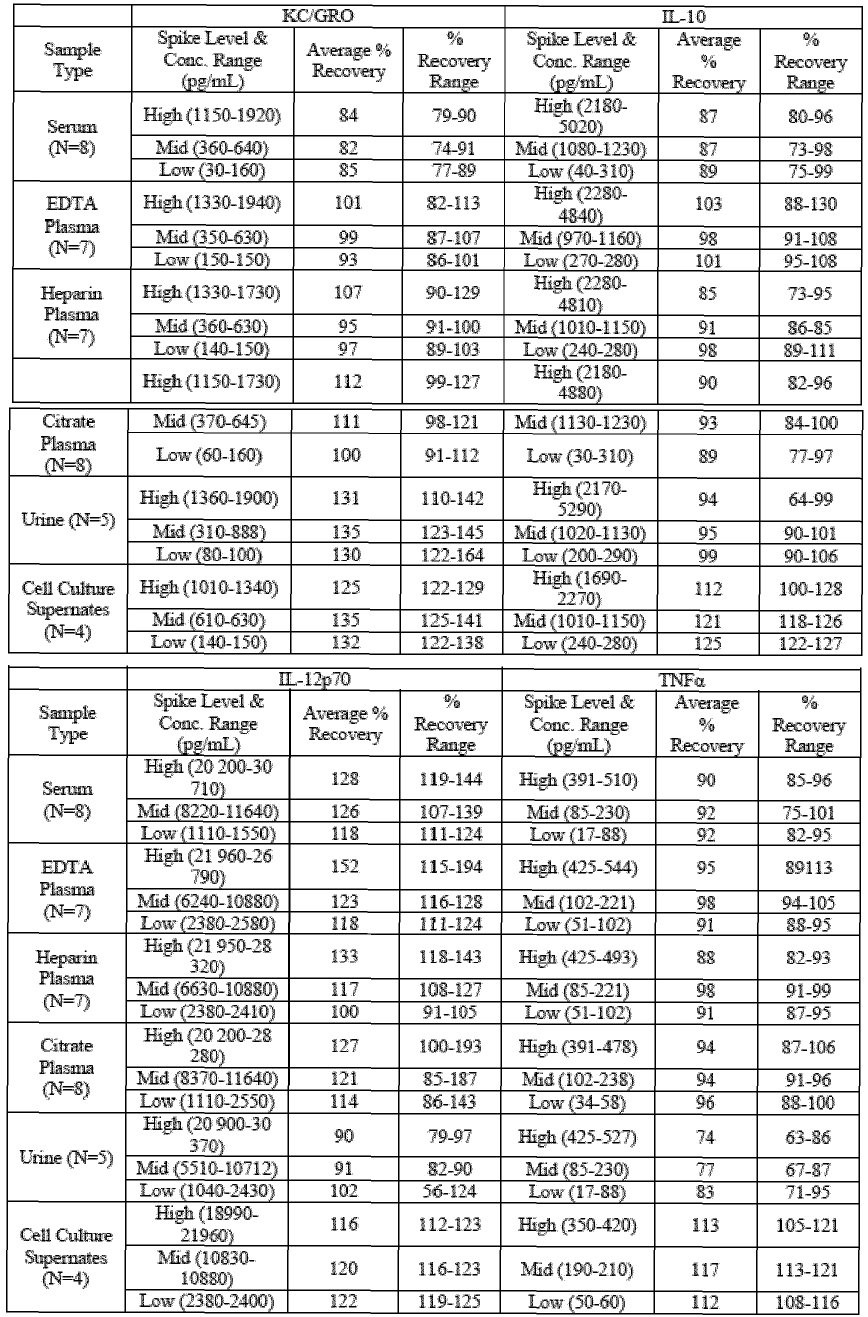

To assess specificity of the individual assays, each panel was run using blended antibodies with individual calibrators at concentration that yield signal around 100,000 counts. $Non - specificity \left(%\right) = \left(\frac{Specific Signal}{Non - specific Signal}\right) ∗ 100$

**Table 28. Panel 1**

| Calibrator | IFNγ | IL-1β | IL-2 | IL-4 | IL-6 | IL-8 | IL-10 | IL-12p70 | IL-23 | TNFα |
|---|---|---|---|---|---|---|---|---|---|---|
| Concentration (pg/mL) | 157 | 34 | 92 | 40 | 120 | 50 | 100 | 313 | 208 | 120 |
| Highest non-specificity (%). | 0.01 | 0.01 | 0.03 | 0.02 | 0.51 | 0.15 | 0.01 | 0.01 | 0.05 | 0.02 |

**Table 29. Panel 2**

| Calibrator | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|
| Concentration (pg/mL) | 125 | 50 | 73 | 92 | 328 | 98 | 469 | 457 | 47 | 108 |
| Highest non-specificity (%) | 0.13 | 0.12 | 0.08 | 0.05 | 0.55 | 0.81 | 0.18 | 0.12 | 0.09 | 0.08 |

**Table 30. Panel 3**

| Calibrator | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|
| Concentration (pg/mL) | 250 | 100 | 1250 | 100 | 290 | 150 | 6000 | 100 | 700 | 156 |
| Highest non-specificity (%) | 0.02 | 0.06 | 0.03 | 0.04 | 0.33 | 0.63 | 0.03 | 0.04 | 0.04 | 0.05 |

**Table 31. Panel 4**

| Calibrator | IFNγ | IL-2 | EL-4 | IL-1β | IL-5 | IL-6 | KC/GRO | IL-10 | IL-13 | TNFα |
|---|---|---|---|---|---|---|---|---|---|---|
| Concentration (pg/mL) | 1250 | 15 000 | 300 | 10 000 | 2000 | 3500 | 600 | 3500 | 750 | 200 |
| Highest non-specificity (%) | 0.05 | 0.08 | 0.03 | 0.09 | 0.14 | 0.35 | 0.34 | 0.47 | 0.81 | 0.81 |

**Table 32. Panel 5**

| Calibrator | IFNγ | IL-1β | IL-2 | IL-4 | IL-5 | IL-6 | KC/GRO | EL-10 | IL-12p70 | TNFα |
|---|---|---|---|---|---|---|---|---|---|---|
| Concentration (pg/mL) | 40 | 100 | 160 | 100 | 60 | 420 | 200 | 500 | 5010 | 83 |
| Highest non-specificity (%) | 0.01 | 0.03 | 0.02 | 0.02 | 0.04 | 0.19 | 0.19 | 0.46 | 0.07 | 0.03 |

To assess the specificity of each antibody, each panel was run using blended calibrators with concentrations listed above and individual antibodies at 1X concentration.

**Table 33. Panel 1**

| Antibody | IFNγ | IL-1β | IL-2 | IL-4 | IL-6 | IL-8 | IL-10 | IL-12p70 | IL-13 | TNFα |
|---|---|---|---|---|---|---|---|---|---|---|
| Highest non-specificity (%) | 0.02 | 0.02 | 0.03 | 0.02 | 0.52 | 0.11 | 0.05 | 0.11 | 0.04 | 0.43 |

**Table 34. Panel 2**

| Antibody | GM-CSF | IL-1α | IL-5 | IL-7 | IL-12/IL-23 p40 | IL-15 | IL-16 | IL17A | TNFβ | VEGF |
|---|---|---|---|---|---|---|---|---|---|---|
| Highest nοn-specificity (%) | 0.40 | 0.10 | 0.05 | 0.06 | 0.19 | 0.27 | 0.47 | 0.12 | 0.06 | 0.02 |

**Table 35. Panel 3**

| Antibody | Eotaxin | MIP-1β | Eotaxin-3 | TARC | IP-10 | MIP-1α | MCP-1 | MDC | MCP-4 |
|---|---|---|---|---|---|---|---|---|---|
| Highest non-specificity (%) | 0.05 | 0.29 | 0.29 | 0.12 | 0.76 | 0.34 | 0.49 | 0.75 | 1.2 |

**Table 36. Panel 4**

| Antibody | IFNγ | IL-2 | IL-4 | IL-1β | IL-5 | IL-6 | KC/GRO | IL-10 | IL-13 | TNFα |
|---|---|---|---|---|---|---|---|---|---|---|
| Highest non-specificity (%) | 0.09 | 0.1 | 0.08 | 0.07 | 0.07 | 0.12 | 0.22 | 0.11 | 0.25 | 0.08 |

**Table 37. Panel 5**

| Antibody | IFNγ | IL-1β | IL-2 | IL-4 | IL-5 | IL-6 | KC/GRO | IL-10 | IL-12p70 | TNFα |
|---|---|---|---|---|---|---|---|---|---|---|
| Highest non-specificity (%) | 0.01 | 0.09 | 0.01 | 0.05 | 0.02 | 0.02 | 0.05 | 0.04 | 0.03 | 0.04 |

To evaluate the specificity of the Panel 1 Kit assays against other biomarkers, each kit was run using blended antibodies with individual recombinant human proteins.

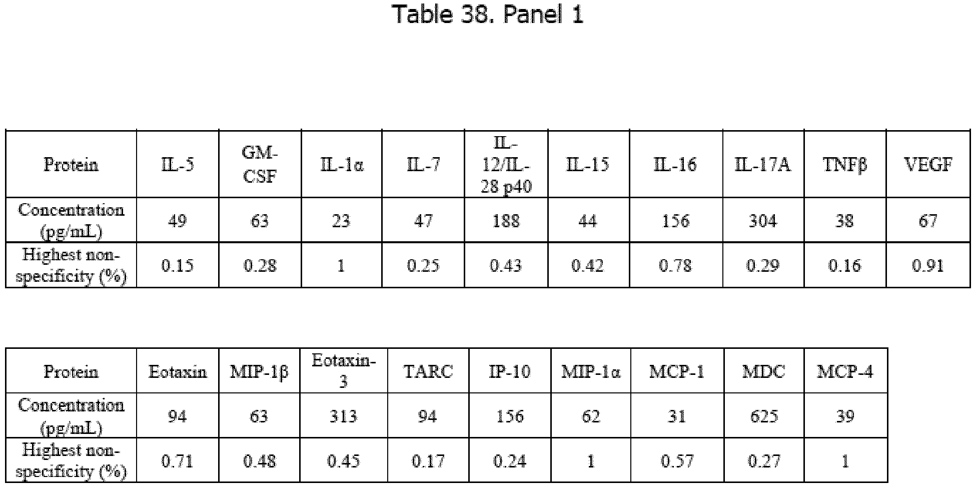

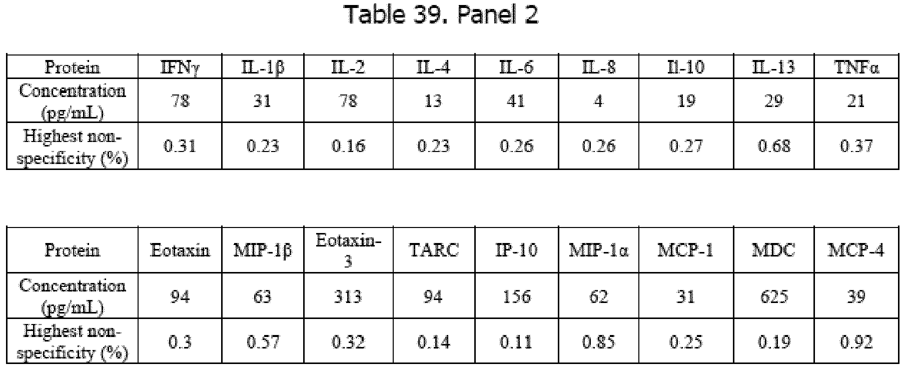

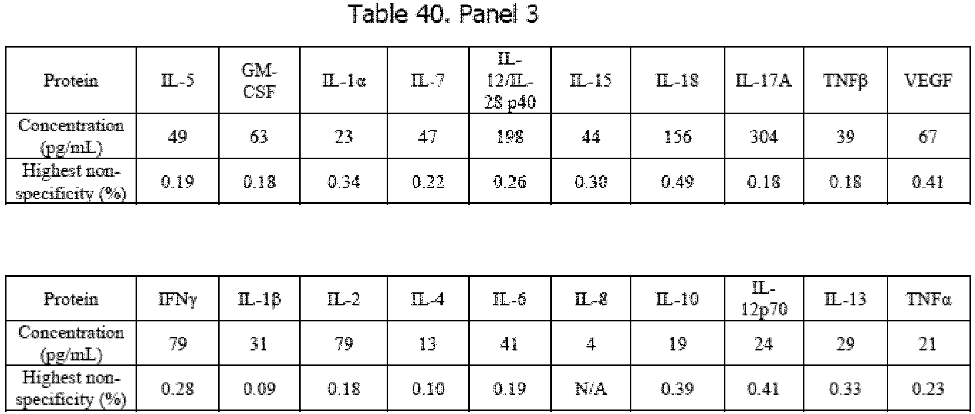

To evaluate the impact of multiplexing on assay signal, standards in the quantifiable ranges were compared between individual assays (individual calibrator and individual antibody) and multiplexed assays (blended calibrators and blended antibodies) using each kit. The calculated % signal difference between individual and multiplexed assay is shown below.

**Table 41. Panel 1**

| Assay | IFNγ | IL-1β | IL-2 | IL-4 | IL-6 | IL-8 | IL-10 | IL-12p70 | IL-13 | TNFα |
|---|---|---|---|---|---|---|---|---|---|---|
| Signal Difference (%) | 0.02 | 0.02 | 0.03 | 0.02 | 0.52 | 0.11 | 0.05 | 0.11 | 0.04 | 0.43 |

**Table 42. Panel 2**

| Assays | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|
| Signal Difference (%) | 4 | 30 | 24 | 27 | 2 | 8 | 22 | 14 | 3 | 18 |

**Table 43. Panel 3**

| Assay | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|
| Signal Difference (%) | 4 | 4 | 24 | 11 | 22 | 2 | 7 | 7 | 6 | 11 |

**Table 44. Panel 4**

| Assay | IFNγ | IL-2 | IL-4 | IL-1β | IL-5 | IL-6 | KC/GRO | IL-10 | IL-13 | TNFα |
|---|---|---|---|---|---|---|---|---|---|---|
| Signal Difference (%) | 11 | 17 | 13 | 23 | 37 | 29 | 3 | 3 | 5 | 9 |

**Table 45. Panel 5**

| Assay | IFNγ | IL-1β | IL-2 | IL-4 | IL-5 | IL-6 | KC/GRO | IL-10 | IL-12p70 | TNFα |
|---|---|---|---|---|---|---|---|---|---|---|
| Signal Difference (%) | 14 | 10 | 2 | 14 | 15 | 8 | 7 | 0 | 1 | 17 |

The kits were designed to minimize interference by receptors and other related proteins. For each panel, a multi-analyte calibrator in diluent and normal human were spiked with three different concentrations of receptors and binding partners. The recovered calibrator concentrations were compared to unspiked standards and normal serum.

All the assays in each panel were calibrated against a reference calibrator obtained from Meso Scale Discovery (Rockville, MD). The NIBSC/WHO Standards for the following human analytes were evaluated against the MSD reference calibrators. To convert sample values obtained with a panel to approximate NIBSC/WHO concentration, the calculated sample value was multiplied by the concentration ratio.

**Table 46. Panel 1**

| Analyte | NIBSC/WHO Standard | Concentration Ratio (MSD Reference : NIBSC) |
|---|---|---|
| IL-1β | 86/680 | 0.1 |
| IL-2 | 86/504 | 1.1 |
| IL-4 | 88/656 | 1.0 |
| IL-6 | 89/548 | 1.0 |
| IL-8 | 89/520 | 1.0 |
| IL-10 | 93/622 | 1.0 |
| IL-12p70 | 95/544 | 1.0 |
| IL-13 | 94/622 | 1.0 |
| TNFα | 88/186 | 1.0 |

**Table 47. Panel 2**

| Analyte | NIBSC/WHO Standard | Concentration Ratio (MSD Reference : NIBSC) |
|---|---|---|
| GM-CSF | 88/646 | 1.08 |
| IL-1α | 86/632 | 1.0 |
| IL-5 | 90/586 | 1.0 |
| IL-7 | 90/530 | 1.0 |
| IL-15 | 95/554 | 0.95 |
| IL-17A | 01/420 | 1.0 |
| TNFβ | 87/640 | 1.0 |
| VEGF | 02/286 | 1.0 |

**Table 48. Panel 3**

| Analyte | MBSC/WHO Standard | Concentration Ratio (MSD Reference: NIBSC) |
|---|---|---|
| MIP-1α | 92/518 | 1.0 |
| IL-8 | 89/520 | 1.0 |
| MCP-1 | 92/194 | 0.85 |

**Table 49. Panel 5**

| Analyte | NIBSC/WHO Standard | Concentration Ratio (MSD Reference: NIBSC) |
|---|---|---|
| IL-1β | 96/668 | 1.18 |
| IL-2 | 93/566 | 0.98 |
| IL-4 | 91/656 | 0.89 |
| IL-6 | 93/630 | 1.0 |
| TNFα | 88/532 | 1.0 |

### (a) Normal Sample Testing

Normal mouse serum (rat serum for panel 4), EDTA plasma, heparin plasma, citrate plasma, and urine samples from a commercial source were diluted 2- to 4-fold and tested with each panel. Median and range of concentrations for each sample set are displayed below. Concentrations are corrected for sample dilution.

**Table 50. Panel 1**

| Sample Type | Statistic | IFNγ | IL-1β | IL-2 | IL-4 | IL-6 | IL-8 | IL-10 | IL-12p70 | IL-13 | TNFα |
|---|---|---|---|---|---|---|---|---|---|---|---|
| Serum (N=27) | Median (pg/mL) | 3.77 | 0.0955 | 0.403 | 0.00565 | 0.167 | 9.51 | 0.0605 | 0.0102 | 0.0994 | 0.199 |
| | Range (pg/mL) | 1-14 | 0-14 | 0-3 | 0-0 | 0-27 | 1-1721 | 0-3 | 0-0 | 0-3 | 0-2 |
| | Samples in Quantitative Range | 26 | 12 | 15 | 14 | 17 | 27 | 26 | 16 | 12 | 27 |
| EDTA Plasma (N=22) | Median (pg/mL) | 3.80 | 0.0538 | 0.174 | 0.0166 | 0.174 | 0.519 | 0.167 | 0.150 | 0.0 | 0.735 |
| | Range (pg/mL) | 0-23 | 0-1 | 0-4 | 0-0 | 0-1 | 0-20 | 0-3 | 0-1 | 0-1 | 0-2 |
| | Samples in Quantitative Range | 21 | 22 | 16 | 17 | 15 | 22 | 21 | 17 | 13 | 22 |
| Heparin Plasma (N=27) | Median (pg/mL) | 2.87 | 0.0894 | 0.0510 | 0.0 | 0.114 | 60.1 | 0.0798 | 0.0473 | 0.0 | 0.456 |
| | Range (pg/mL) | 0-8 | 0-11 | 0-3 | 0-0 | 0-3 | 2-2626 | 0-3 | 0-0 | 0-3 | 0-1 |
| | Samples in Quantitative Range | 27 | 27 | 16 | 13 | 18 | 27 | 23 | 15 | 17 | 27 |
| Citrate Plasma (N=20) | Median (pg/mL) | 3.28 | 0.0627 | 0.0897 | 0.00496 | 0.190 | 2.85 | 0.115 | 0.0283 | 0.0 | 0.481 |
| | Range (pg/mL) | 1-35 | 0-0 | 0-1 | 0-0 | 0-0 | 0-112 | 0-2 | 0-0 | 0-0 | 0-3 |
| | | | | | | | | | | | |
| | Samples in Quantitative Range | 20 | 11 | 10 | 12 | 10 | 20 | 20 | 11 | 10 | 20 |
| Urine (N=5) | Median (pg/mL) | 0.399 | 0.350 | 0.0513 | 0.0168 | 0.088 | 35.185 | 0.018 | 0.009 | 0.0 | 0.0 |
| | Range (pg/mL) | 0-1 | 0-10 | 0-0 | 0-0 | 0-0 | 1-105 | 0-0 | 0-0 | 0-0 | 0-0 |
| | Samples in Quantitative Range | 5 | 5 | 5 | 5 | 5 | 5 | 5 | 5 | 5 | 5 |

**Table 51. Panel 2**

| Sample Type | Statistic | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|---|
| Serum (N=20) | Median (pg/mL) | 34.1 | 1 | ND | 1 | 53 | 1 | 60 | 5 | ND | 9 |
| | Range (pg/mL) | 11-44 | 1-62 | ND | 1-3 | 13-159 | 1-3 | 24-137 | 5-5 | ND | 2-187 |
| | Samples in Quantitative Range | 8 | 9 | 0 | 15 | 20 | 20 | 20 | 1 | 0 | 15 |
| EDTA Plasma (N=20) | Median (pg/mL) | 1.5 | 2 | 1 | 3 | 65 | 2 | 76 | 9 | ND | 95 |
| | Range (pg/mL) | 0-1 | 0-99 | 1-1 | 0-40 | 3-395 | 1-3 | 0-973 | 1-55 | ND | 18-338 |
| | Samples in Quantitative Range | 2 | 18 | 1 | 15 | 17 | 16 | 15 | 5 | 0 | 13 |
| Heparin (N=20) | Median (pg/mL) | ND | 1 | 45 | 100 | ND | 1 | 5 | 1 | ND | 9 |
| | Range (pg/mL) | ND | 1-1 | 9-148 | 35-1109 | ND | 1-57 | 1-39 | 1-2 | ND | 5-484 |
| | Samples in Quantitative Range | 0 | 2 | 19 | 19 | 0 | 7 | 17 | 19 | 0 | 17 |
| Citrate Plasma (N=20) | Median (pg/mL) | ND | ND | ND | ND | ND | ND | ND | ND | ND | ND |
| | Range (pg/mL) | ND | ND | ND | ND | ND | ND | ND | ND | ND | ND |
| | Samples in Quantitative Range | 0 | 0 | 0 | 0 | 0 | 0 | 0 | 0 | 0 | 0 |
| Urine (N=5) | Median (pg/mL) | 2 | 3 | ND | 1 | 27 | 1 | 47 | 5 | ND | 50 |
| | Range (pg/mL) | 2-2 | 2-3 | ND | 1-1 | 27-27 | 1-2 | 47-47 | 5-5 | ND | 37-85 |
| | | | | | | | | | | | |
| | Samples in Quantitative Range | 1 | 3 | 0 | 2 | 1 | 2 | 1 | 1 | 0 | 4 |

**Table 52. Panel 3**

| Sample Type | Statistic | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|---|
| Serum (N=27) | Median (pg/mL) | 41 | 46 | 6 | 28 | 65 | 9 | 547 | 107 | 1246 | 34 |
| | Range (pg/mL) | 19-145 | 7-95 | 5-9 | 5-70 | 27-261 | 7-202 | 262-665 | 76-205 | 606-3249 | 11-117 |
| | Samples in Quantitative Range | 26 | 27 | 10 | 27 | 27 | 19 | 7 | 27 | 27 | 27 |
| EDTA Plasma (N=27) | Median (pg/mL) | 117 | 63 | 13 | 79 | 197 | 11 | 769 | 79 | 1309 | 67 |
| | Range (pg/mL) | 37-795 | 8-153 | 5-115 | 13-373 | 97-676 | 7-651 | 340-2478 | 42-185 | 869-2144 | 12-582 |
| | Samples in Quantitative Range | 27 | 27 | 26 | 27 | 27 | 20 | 11 | 27 | 27 | 27 |
| Heparin Plasma (N=27) | Median (pg/mL) | 293 | 121 | 31 | 92 | 146 | 41 | 775 | 137 | 1050 | 183 |
| | Range (pg/mL) | 22-1522 | 10-301 | 5-147 | 6-957 | 91-625 | 7-2231 | 234-3281 | 69-319 | 589-1963 | 67-716 |
| | Samples in Quantitative Range | 27 | 27 | 27 | 27 | 27 | 26 | 7 | 27 | 27 | 27 |
| Citrate Plasma (N-20) | Median (pg/mL) | 191 | 51 | 12 | 51 | 77 | 9 | 964 | 135 | 994 | 64 |
| | Range (pg/mL) | 72-288 | 19-123 | 7-19 | 25-131 | 36-373 | 7-30 | 328-1869 | 76-242 | 576-1364 | 35-161 |
| | Samples in Quantitative Range | 20 | 20 | 19 | 20 | 20 | 9 | 4 | 20 | 20 | 20 |
| Urine (N=5) | Median (pg/mL) | 13 | 3 | ND | 1 | 8 | ND | 296 | 80 | ND | 13 |
| | Range (pg/mL) | 13-13 | 2-13 | ND | 1-1 | 3-64 | ND | 296-296 | 56-122 | ND | 11-15 |
| | Samples in Quantitative Range | 1 | 5 | 0 | 1 | 3 | 0 | 1 | 5 | 0 | 2 |

**Table 53. Panel 5**

| Sample Type | Statistic | IFNγ | IL-1β | IL-2 | IL-4 | IL-5 | IL-6 | KC/GRO | IL-10 | IL12p70 | TNFα |
|---|---|---|---|---|---|---|---|---|---|---|---|
| Serum (N=16) | Median (pg/mL) | 0.95 | 2.27 | 1.02 | 0.43 | 2.72 | 21.6 | 48.3 | 11.0 | 81.0 | 12.0 |
| | Range (pg/mL) | 0.34-28.7 | 1.13-3.95 | 0.55-3.98 | 0.23-1.10 | 0.58-6.52 | 5.28-110.7 | 28.7-101.8 | 5.71-45.4 | 64.8-97.1 | 8.23-34.4 |
| | Samples in Quantitative Range | 16 | 16 | 16 | 15 | 16 | 16 | 16 | 16 | 2 | 16 |
| EDTA Plasma (N=15) | Median (pg/mL) | 41.2 | 0.86 | 3.86 | 0.63 | 2.59 | 119.1 | 70.5 | 56.5 | 69.3 | 38.5 |
| | Range (pg/mL) | 18.6-262.1 | 0.46-2.40 | 2.60-5.89 | 0.48-0.70 | 1.50-2.88 | 11.0-184.6 | 54.2-96.9 | 31.5-74.7 | 50.2-170.8 | 21.3-47.0 |
| | Samples in Quantitative Range | 15 | 13 | 15 | 9 | 15 | 15 | 15 | 15 | 11 | 15 |
| Heparin Plasma (N=15) | Median (pg/mL) | 261.5 | 1.62 | 4.63 | 0.75 | 4.01 | 175.2 | 269.4 | 76.4 | 85.6 | 65.3 |
| | Range (pg/mL) | 156.4-352.4 | 0.61-2.25 | 3.35-7.36 | 0.42-1.49 | 2.26-5.72 | 28.8-354.6 | 220.1-368.6 | 63.7-104.6 | 38.0-152.0 | 35.0-76.7 |
| | Samples in Quantitative Range | 15 | 13 | 15 | 9 | 15 | 15 | 15 | 15 | 8 | 15 |
| Citrate Plasma (N=16) | Median (pg/mL) | 7.04 | 1.01 | 3.09 | 0.73 | 3.37 | 41.9 | 65.3 | 30.7 | 71.2 | 42.8 |
| | Range (pg/mL) | 0.31-121.8 | 0.45-2.02 | 0.65-5.03 | 0.39-1.47 | 1.72-8.24 | 6.84-74.2 | 34.9-172.0 | 5.30-68.2 | 50.4-107.4 | 5.45-58.8 |
| | Samples in Quantitative Range | 16 | 16 | 15 | 16 | 16 | 16 | 16 | 16 | 15 | 16 |
| Urine (N=10) | Median (pg/mL) | 0.32 | 0.57 | 0.49 | 0.43 | ND | ND | 2.31 | 1.36 | 101.6 | 0.63 |
| | Range (pg/mL) | 0.09-0.66 | 0.35-1.34 | 0.49-0.65 | 0.43-0.62 | ND | ND | 1.91-2.84 | 0.98-1.53 | 67.3-125.1 | 0.48-3.90 |
| | Samples in Quantitative Range | 7 | 6 | 3 | 9 | 0 | 0 | 10 | 4 | 9 | 8 |

### (b) Stimulated Samples

Panel 1: Freshly collected normal human whole blood was incubated with LPS and simultaneously incubated with peptidoglycan (PG) and Zymosan (ZY) for different time periods and plasma was then isolated. These samples were then tested with panel 1. The dilution adjusted concentrations for each stimulation model is displayed below.

**Table 54. Panel 1**

| Stimulant | Incubation time (hr) | IFNγ | IL-1β | IL-2 | IL-4 | IL-6 | IL-8 | IL-10 | IL-12p70 | IL-13 | TNFα |
|---|---|---|---|---|---|---|---|---|---|---|---|
| Control | 0 | 11.8 | 0.22 | 0.68 | 0.09 | 0.63 | 6.08 | 0.37 | 0.37 | ND | 2.57 |
| Control | 6 | 9.1 | 4.53 | 0.31 | 0.1 | 1.09 | 40.01 | 0.37 | 0.45 | 0.61 | 3.16 |
| LPS | 3 | 34.4 | 1028 | ND | 4.84 | 8028 | 5635 | 8.58 | 2.99 | 5.14 | 7962.8 |
| | 12 | 458.6 | 9153 | ND | 8.11 | 22053 | 9778 | 115.8 | 4.14 | ND | 9150 |
| PILA 100 ng | 6 | 8.04 | 14.51 | ND | ND | ND | 301.6 | 0.63 | 0.21 | ND | 9.30 |
| PILA 1 ug | 6 | 14.7 | 164.5 | ND | 0.13 | 366.9 | 1978.8 | 10.4 | 0.23 | ND | 261.18 |

Panel 2: Freshly collected normal human whole blood was incubated at 37°C with LPS and PHA for different time periods and plasma was isolated. The samples were tested with panel 2.

**Table 55. Panel 2**

| Stimulant | Incubation time (hr) | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|---|
| Control | 0 | ND | ND | ND | ND | 96 | 2 | 73 | ND | ND | ND |
| Control | 6 | ND | 12 | ND | 4 | 55 | 2 | 129 | ND | ND | 44 |
| LPS | 3 | ND | ND | ND | ND | 96 | 2 | 73 | ND | ND | ND |
| | 12 | ND | 19 | ND | 3 | 6427 | 2 | 161 | ND | 1 | 3 |
| PILA 100 ng | 6 | ND | 10 | ND | 4 | 75 | 2 | 130 | ND | ND | 33 |
| PILA 1 ug | 6 | ND | 16 | ND | 4 | 343 | ND | 794 | ND | ND | 2096 |

Panel 3: Freshly collected normal human whole blood was incubated at 37 °C with LPS for different time period and plasma was isolated. The samples were tested with panel 3.

**Table 56. Panel 3**

| Stimulant | Incubation time (hr) | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|---|
| Control | 0 | 184 | 51 | 11 | 81 | 239 | 10 | ND | 114 | 1128 | 62 |
| LPS | 3 | 158 | >4000 | 5 | 134 | >10000 | >3960 | 3468 | 412 | 1891 | 89 |
| | 12 | 91 | >4000 | 10 | 161 | >10000 | >3930 | 2764 | 250 | 1210 | 114 |

Panel 5: Freshly collected normal pooled mouse whole blood was incubated with LPS and simultaneously incubated with peptidoglycan (PG) and Zymosan (ZY) for different time periods and plasma isolated. Samples were run on panel 5.

**Table 57. Panel 5**

| Stimulant | Incubation time (hr) | IFNγ | IL-1β | IL-2 | IL-4 | IL-5 | IL-6 | KC/GRO | IL-10 | IL-12p70 | TNFα |
|---|---|---|---|---|---|---|---|---|---|---|---|
| Control | 0 | 11.76 | 1.02 | 2.74 | 0.56 | 3.20 | 125.40 | 96.75 | 24.82 | 56.17 | 35.40 |
| LPS | 3 | 6.16 | 7.19 | 1.65 | 0.73 | 6.73 | 1510.01 | 86.73 | 57.20 | 115.60 | 266.37 |
| | 12 | 11.73 | 89.06 | 1.63 | 16.90 | 6.92 | 3362.96 | 128.59 | 47.75 | 138.06 | 396.69 |
| None (Control) | 3 | 6.66 | 2.59 | 2.58 | 4.60 | 2.49 | 400.40 | 89.27 | 31.55 | 82.99 | 30.69 |
| PG/XY | 3 | 14.05 | 87.45 | 2.65 | 1.46 | 2.93 | 703.30 | 213.05 | 42.27 | 103.34 | 111.58 |
| None (Control) | 12 | 15.01 | 4.67 | 2.48 | 6.23 | 2.83 | 236.86 | 50.85 | 15.51 | 94.03 | 21.45 |
| PG/XY | 12 | 20.78 | 156.90 | 3.01 | 2.02 | 3.65 | 1500.23 | 159.05 | 59.44 | 90.93 | 723.53 |

For panels 1-3, freshly isolated PBMC from normal whole blood was stimulated with LPS, PHA, PWM, Con A, and co-stimulated with CD3 and CD28 antibodies. The samples were then tested with panels 1-3. The dilution adjusted concentrations in pg/mL for each stimulation model is displayed.

**Table 58. Panel 1**

| Stimulant | Incubation time (hr) | IFNγ | IL-1β | IL-2 | IL-4 | IL-6 | IL-8 | IL-10 | IL-12p70 | IL-13 | TNFα |
|---|---|---|---|---|---|---|---|---|---|---|---|
| Control | 24 | 2.56 | 16.56 | 0.49 | ND | ND | 4043 | 0.8 | 0.53 | 7.6 | 1.1 |
| PNA 5 ug/mL | 24 | 13829 | 222 | 1614 | 20.28 | 9052 | >1000 | 136.3 | 13.05 | 277.1 | 365.6 |
| LPS 10ug/mL | 24 | 1494 | >1000 | ND | ND | 27701 | >1000 | 748 | 9.8 | 228.5 | >660 |
| PKM 50ug/mL | 24 | 9468 | 5974 | 995 | 5.09 | 29858 | 205183 | 1123 | 3.96 | 5.5 | 1276 |
| a-CD3+CD28 (5+5 ug/mL) | 24 | 1747 | 4.11 | 571.2 | 11.03 | ND | 8017 | 42.9 | ND | 35 | 11.35 |
| Con A 20 ug/mL | 24 | 24658 | 116.5 | 3589 | 11.4 | 236.9 | 46528 | 146 | 24.04 | 45.08 | 263.5 |

**Table 59. Panel 2**

| Stimulant | Incubation time (hr) | GM-CSF | IL-1α | IL-5 | IL-7 | IL-12/IL-23 p40 | IL-15 | IL-16 | IL-17A | INFβ | VEGF |
|---|---|---|---|---|---|---|---|---|---|---|---|
| Control | 24 | ND | ND | ND | 7 | 14 | ND | 1270 | ND | ND | 1243 |
| PNA 5 ug/mL | 24 | 178 | 51 | 291 | 14 | 338 | ND | 960 | 1677 | 9 | 1147 |
| LPS 10ug/mL | 24 | 17 | 940 | 2 | 11 | 343 | ND | 794 | ND | ND | 2096 |
| PKM 50ug/mL | 24 | 66 | 811 | 31 | 9 | 526 | ND | 782 | 658 | 2 | 2194 |
| a-CD3+CD28 (5+5 ug/mL) | 24 | 78 | | 339 | 10 | 132 | ND | 1046 | 321 | 27 | 804 |
| Con A 20 ug/mL | 24 | 212 | 272 | 194 | 11 | 1390 | ND | 1252 | 816 | 53 | 1299 |

**Table 60. Panel 3**

| Stimulant | Incubation time (hr) | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|---|
| Control | 6 | 140 | 181 | 33 | 132 | 312 | 41 | ND | 80 | 1157 | 47 |
| Control | 24 | 127 | 76 | 31 | 199 | 2322 | 34 | 3742 | 141 | 769 | 59 |
| PNA (100 mg/mL) | 6 | 197 | 1753 | 31 | 147 | 372 | 117 | NaN | 71 | 942 | 55 |
| PNA (1 ug/mL) | 6 | 149 | >4100 | 21 | 141 | 1653 | 1853 | NaN | 443 | 940 | 55 |
| PNA (5 ug/mL) | 24 | 130 | 19163 | NaN | 683 | 107930 | 10374 | 192588 | 80059 | 1986 | 278 |
| LPS (10 ug/mL) | 24 | 84 | 59243 | NaN | 258 | 3036 | 48161 | 182842 | 355 | 515 | 149 |
| PKM (50 ug/mL) | 24 | 131 | 59467 | NaN | 232 | 4169 | 43588 | 195681 | 465 | 560 | 123 |
| CD3 + CD28 (5 ug/mL each) | 24 | 143 | 1368 | NaN | 1250 | 75234 | 534 | 4646 | 6844 | 2643 | 507 |
| Con A 20 ug/mL | 24 | 230 | 4347 | NaN | 1307 | 105145 | 700 | 35480 | 43377 | 3889 | 320 |

For panels 1-3, human acute monocyte leukemia cell line (THP-1 cell line) was stimulated with LPS for six and 16 hours. The supernates were then isolated and tested with panels 1-3. The dilution adjusted concentrations in pg/mL for each sample is displayed below.

**Table 61. Panel 1**

| Stimulant | Incubation Time (hr) | IFNγ | IL-1β | IL-2 | IL-4 | IL-6 | IL-8 | IL-10 | IL-12p70 | IL-13 | TNFα |
|---|---|---|---|---|---|---|---|---|---|---|---|
| Control | 0 | 1.09 | 20.48 | 0.345 | 0.07 | 0.19 | 449.8 | 0.44 | 0.38 | 1.41 | 9.91 |
| LPS | 6 | 1.1 | 645.5 | 11.5 | ND | ND | 61066 | 97.2 | 11.12 | 0.87 | 12472 |
| | 16 | 0.67 | 423 | ND | ND | ND | 69638 | 15.5 | ND | 1.06 | 2915 |

**Table 62. Panel 2**

| Stimulant | Incubation Time (hr) | GM-CSF | IL-1α | IL-5 | IL-7 | IL-12/IL-23 p40 | IL-15 | IL-16 | IL-17A | TNFβ | VEGF |
|---|---|---|---|---|---|---|---|---|---|---|---|
| Control | 0 | ND | ND | ND | ND | ND | ND | 205 | ND | ND | 1995 |
| LPS | 6 | ND | 47 | ND | ND | 56 | ND | 421 | ND | ND | 276 |
| | 16 | ND | 22 | ND | ND | 234 | ND | 552 | ND | ND | >1070 |

**Table 63. Panel 3**

| Stimulant | Incubation Time (hr) | Eotaxin | MIP-1β | Eotaxin-3 | TARC | IP-10 | MIP-1α | IL-8 | MCP-1 | MDC | MCP-4 |
|---|---|---|---|---|---|---|---|---|---|---|---|
| Control | 0 | 26 | 983 | 12 | ND | 96 | 70 | 1132 | 205 | 148 | ND |
| | | | | | | | | | | | |
| LPS | 6 | ND | >4000 | ND | ND | 324 | >3960 | 45546 | 577 | 14998 | 57 |
| | 16 | ND | >4000 | ND | 20 | 687 | 2759 | 52262 | 1290 | >400O0 | 332 |

For panel 5, a mouse monocyte macrophage cell line (J774A.1) and a mouse leukemic monocyte macrophage cell line (RAW 264.7) were stimulated with different stimulants. The J774A.1 cell line stimulation was for four house while the RAW cell line stimulation was for six hours. The lysates were collected and run on panel 5. The concentrations are listed in pg/mL and normalized for 50 ug of lysate per well.

**Table 64. Panel 5**

| Cell Line | Stimulant | IFNγ | IL-1β | IL-2 | IL-4 | IL-5 | IL-6 | KC/GRO | IF-1 0 | IL-12p70 | INFα |
|---|---|---|---|---|---|---|---|---|---|---|---|
| J774A.1 | None | ND | 1.9 | ND | ND | ND | 17 | ND | 34 | ND | 812 |
| J774A.1 | 5 ug/mL LPS | ND | 8948 | 3.9 | ND | ND | 62529 | 107 | 320 | ND | >1000O |
| J774A.1 | 5 ug/mL PWM | ND | 10674 | 2.8 | ND | ND | 47527 | 111 | 209 | ND | >10000 |
| J774A.1 | 1 ng/mL LPS | ND | 304 | ND | ND | ND | 407 | 0.74 | 87 | ND | 364 |
| J774A.1 | 100 ng/mL PWM | ND | 150 | ND | ND | ND | 57 | ND | 84 | ND | 264 |
| RAN 264.7 | 100 ng/mL LPS | ND | 41450 | 1.8 | 0.16 | ND | 8129 | 12 | 1057 | ND | >10000 |

The following calibrator blends were used in each panel as follows:

**Table 65. Panel 1**

| Calibrator | Sequence | Expression System |
|---|---|---|
| IFNγ | Gln24-Gln166 | E. coli |
| IL-1β | Ala117-Ser269 | E coli |
| IL-2 | Ala21-Thr153 | E. coli |
| IL-4 | His25-Ser153 | E coli |
| IL-6 | Pro29-Met212 | E. coli |
| IL-8 | Ser28-Ser99 | E. coli |
| IL-10 | Ser19-Asn178 | Sf21 insect cells |
| IL-12p70 | IL-12p40 (Ile23-Ser328) | Sf21 insect cells |
| | IL-12p35 (Arg23-Ser219) | |
| IL-13 | Gly21-Asn132 | E. coli |
| TNFα | Val77 -Leu233 | E. coli |

**Table 66. Panel 2**

| Calibrator | Sequence | Expression System |
|---|---|---|
| GM-CSF | Ala18-Glu144 | E. coli |
| IL-1α | Ser113-Ala271 | E. coli |
| IL-5 | Ile20-Ser134 | Sf21 insect cells |
| IL-7 | Asp26-His177 | E. coli |
| IL-12/IL-23 p40 | Ile23-Ser328 | Sf21 insect cells |
| IL-15 | Asn49-Ser162 | E. coli |
| IL-16 | Pro2-Ser130 | E. coli |
| IL-17A | Ile20-Ala155 | E. coli |
| TNFβ | Len35-Leu205 | E. coli |
| | | |
| VEGF | Ala27-Arg191 | Sf21 insect cells |

**Table 67. Panel 3**

| Calibrator | Sequence | Expression System |
|---|---|---|
| Eotaxin | Gly24-Pro97 | E. coli |
| MIP-1β | Ala24-Asn92 | E. coli |
| Eotaxin-3 | Thr24-Leu94 | E. coli |
| TARC | Ala24-Ser94 | E. coli |
| IP-10 | Val22-Pro98 | E. coli |
| MIP-1α | Ala27-Ala92 | E. coli |
| IL-8 | Ser28-Ser99 | E. coli |
| MCP-1 | Gln24-Thr99 | E. coli |
| MDC | Gly25-Gln93 | E. coli |
| MCP-4 | Gln24-Thr98 | E. coli |

**Table 68. Panel 4**

| Calibrator | Sequence | Expression System |
|---|---|---|
| IFNγ | Glu23-Cys156 | E. coli |
| IL-2 | Ala21-Gln155 | E. coli |
| IL-4 | Cys25-Ser147 | E. coli |
| IL-1β | Val117-Ser268 | E. coii |
| IL-5 | Met20-Val132 | E. coli |
| IL-6 | Phe25-Trr211 | E. coli |
| KC/GRO | Ala25-Lys96 | E. coli |
| IL-10 | Ser19-Asa178 | E. coli |
| IL-13 | Thr19-His131 | E. coli |
| INFα | Leu80-Leu235 | E. coli |

**Table 69. Panel 5**

| Calibrator | Sequence | Expression System |
|---|---|---|
| IFNγ | His23-Cys155 | E. coli |
| IL-1β | Val118-Ser269 | E. coli |
| IL-2 | Ala21-Gln169 | E. coli |
| IL-4 | His23-Ser140 | E. coli |
| IL-5 | Met21-Gly133 | Sf21 insect cells |
| IL-6 | Phe25-Thr211 | E. coli |
| KC/GRO | Arg20-Lys96 | E. coli |
| IL-10 | Ser19-Ser178 | E. coli |
| IL-12p70 | Met23-Ser335 (mouse IL-12p40) & Arg23-Ala215 (mouse IL-1235) | Sf21 insect cells |
| TNFα | Leu80-Leu235 | E. coli |

The following antibodies, capture and detection, were used in each panel as follows:

**Table 70. Panel 1**

| | Source Species | |
|---|---|---|
| Analyte | MSD Capture Antibody | MSD Detection Antibody |
| IFNγ | Mouse Monoclonal | Mouse Monoclonal |
| IL-1β | Mouse Monoclonal | Goat Polyclonal |
| IL-2 | Mouse Monoclonal | Mouse Monoclonal |
| IL-4 | Mouse Monoclonal | Mouse Monoclonal |
| IL-6 | Mouse Monoclonal | Goat Polyclonal |
| IL-8 | Mouse Monoclonal | Goat Polyclonal |
| IL-10 | Mouse Monoclonal | Mouse Monoclonal |
| IL-12p70 | Mouse Monoclonal | Mouse Monoclonal |
| IL-13 | Rat Monoclonal | Mouse Monoclonal |
| TNFα | Mouse Monoclonal | Goat Polyclonal |

**Table 71. Panel 2**

| | Source Species | |
|---|---|---|
| Analyte | MSD Capture Antibody | MSD Detection Antibody |
| GM-CSF | Mouse Monoclonal | Rat Monoclonal |
| IL-1α | Mouse Monoclonal | Goat Polyclonal |
| IL-5 | Mouse Monoclonal | Mouse Monoclonal |
| IL-7 | Mouse Monoclonal | Goat Monoclonal |
| IL-12/IL-23 p40 | Mouse Monoclonal | Mouse Polyclonal |
| IL-15 | Mouse Monoclonal | Mouse Polyclonal |
| IL-16 | Mouse Monoclonal | Goat Monoclonal |
| IL-17A | Mouse Monoclonal | Goat Monoclonal |
| TNFβ | Mouse Monoclonal | Mouse Monoclonal Mouse Monoclonal |
| VEGF | Mouse Monoclonal | Mouse Polyclonal |

**Table 72. Panel 3**

| | Source Species | |
|---|---|---|
| Analyte | MSD Capture Antibody | MSD Detection Antibody |
| Eotaxin | Mouse Monoclonal | Mouse Monoclonal |
| MP-1β | Mouse Monoclonal | Mouse Monoclonal |
| Eotaxin-3 | Mouse Monoclonal | Mouse Monoclonal |
| TARC | Mouse Monoclonal | Mouse Monoclonal |
| IP-10 | Mouse Monoclonal | Mouse Monoclonal |
| MP-1α | Mouse Monoclonal | Mouse Monoclonal |
| IL-8 | Mouse Monoclonal | Goat Monoclonal |
| MCP-1 | Mouse Monoclonal | Mouse Monoclonal |
| MDC | Mouse Monoclonal | Mouse Monoclonal |
| MCP-4 | Mouse Monoclonal | Mouse Monoclonal |

**Table 73. Panel 4**

| | Source Species | |
|---|---|---|
| Analyte | MSD Capture Antibody | MSD Detection Antibody |
| IFNγ | Mouse Monoclonal | Goat Monoclonal |
| IL-2 | Mouse Monoclonal | Goat Monoclonal |
| IL-4 | Mouse Monoclonal | Goat Monoclonal |
| IL-1β | Mouse Monoclonal | Goat Monoclonal |
| IL-5 | Rat Monoclonal | Rat Monoclonal |
| IL-6 | Mouse Monoclonal | Goat Monoclonal |
| KC/GRO | Mouse Monoclonal | Goat Monoclonal |
| IL-10 | Mouse Monoclonal | Goat Monoclonal |
| IL-13 | Mouse Monoclonal | Goat Monoclonal |
| INFα | Hamster Monoclonal | Goat Monoclonal |

**Table 74. Panel 5**

| | Source Species | |
|---|---|---|
| Analyte | MSD Capture Antibody | MSD Detection Antibody |
| IFNγ | Rat Monoclonal | Rat Monoclonal |
| IL-1β | Mouse Monoclonal | Goat Monoclonal |
| IL-2 | Rat Monoclonal | Rat Monoclonal |
| IL-4 | Rat Monoclonal | Rat Monoclonal |
| IL-5 | Rat Monoclonal | Rat Monoclonal |
| IL-6 | Rat Monoclonal | Goat Monoclonal |
| KC/GRO | Rat Monoclonal | Goat Monoclonal |
| IL-10 | Rat Monoclonal | Goat Monoclonal |
| IL-12p70 | Rat Monoclonal | Rat Monoclonal |
| INFα | Hamster Monoclonal | Goat Monoclonal |

Various publications and test methods are cited herein, the disclosures of which are incorporated herein by reference in their entireties, In cases where the present specification and a document incorporated by reference and/or referred to herein include conflicting disclosure, and/or inconsistent use of terminology, and/or the incorporated/referenced documents use or define terms differently than they are used or defined in the present specification, the present specification shall control.

### References

1. Kause ML, et al. Assessing immune function by profiling cytokine release from stimulated blood leukocytes and the risk of infection in rheumatoid arthritis. Clin. Immunol. 2011; 141(1): 67-72.
2. Holmes C, et al. Proinflammatory cytokines, sickness behavior, and Alzheimer disease. Neurology 2011; 77: 212-8.
3. Desai D, et al. Cytokines and cytokine-specific therapy in asthma. Ad. Clin. Chem. 2012; 57: 57-97.
4. Gui T, et al. Diverse roles of macrophages in atherosclerosis: from inflammatory biology to biomarker discovery. 2012 Apr 11; 693083.
5. Islam SA, et al. T cell homing to epithelial barriers in allergic disease. Nat. Med. 2012 May 4; 18 (5): 705-15.
6. Su DL, et al. Roles of pro- and anti-inflammatory cytokines in the pathogenesis of SLE. Biomed. Biotechnol. 2012; 347141
7. Lukens JR, et al. Inflammasome activation in obesity-related inflammatory disease and autoimmunity. Discov. Med. 2011 Jul; 12 (62): 65-74.
8. Laoui D, et al. Tumor-associated macrophages in breast cancer: distinct subsets, distinct functions. 2011; 55 (7-9): 861-7.
9. Hallberg L, et al. Exercise-induced release of cytokines in patients with major depressive disorder. J. Affect. Disord. 2010; 126(1): 262-267.
10. Oreja-Guevara C, et al. TH1/TH2 Cytokine profile in relapsing-remitting multiple sclerosis patients treated with Glatiramer acetate or Natalizumab. BMC Neurol. 2012 Sep. 18; 12(1): 95.
11. Svensson J, et al. Few differences in cytokines between patients newly diagnosed with type 1 diabetes and their healthy siblings. Hum. Immunol. 2012 Aug 17; S0198-8859 (12): 00502-2.
12. Yehuda H, et al. Isothiocyanates inhibit psoriasis-related proinflammatory factors in human skin. Inflamm. Res. 2012 Jul.; 61 (7): 735-42.
13. Gologan S, et al. Inflammatory gene expression profiles in Crohn's disease and ulcerative colitis: A comparative analysis using a reverse transcriptase multiplex ligation-dependent probe amplification protocol. J. Crohns Colitis. 2012 Sep 24; S1873-9946 (12)00393-5.
14. Kwan W, et al. Bone marrow transplantation confers modest benefits in mouse models of Huntington's disease. J. Neurosci. 2012; 32 (1): 133-42.
15. Crotta S, et al. Hepatitis C virions subvert natural killer cell activation to generate a cytokine environment permissive for infection. J. Hepatol. 2010; 52(2): 183-90.
16. Liu X, et al. Age-dependent neuroinflammatory responses and deficits in long-term potentiation in the hippocampus during systemic inflammation. Neuroscience. 2012 Aug 2; 216: 133-42.
17. Moon MH, et al. Sphingosine-1-phosphate inhibits interleukin-1b-induced inflammation in human articular chondrocytes. Int. J. Mol. Med. 2012 Sep 19; 1135.
18. Mihai G, et al. Circulating cytokines as mediators of fever. Clin. Infect. Dis. 2000; 31: s178-s184.
19. Liao W, et al. IL-2 family cytokines: new insights into the complex roles of IL-2 as a broad regulator of T helper cell differentiation. Curr. Opin. Immunol. 2011 Oct; 23(5): 598-604.
20. Aberg JA, Aging, inflammation, and HIV infection. Top Antivir. Med. 2012 Aug; 20(3): 101-5.
21. Sharma M, et al. Enhanced pro-inflammatory chemokine/cytokine response triggered by pathogenic Entamoeba histolytica: basis of invasive disease. 2005 Dec; 131 (pt. 6): 783-96.
22. Jacysyn JF, et al. IL-4 from Th2-type cells suppresses induction of delayed-type hypersensitivity elicited shortly after immunization. Immunol. Cell Biol. 2003 Dec; 81(6): 424-30.
23. Poon AH, et al. Pathogenesis of severe asthma. Clin. Exp. Allergy. 2012 May; 42(5): 625-37.
24. Deng B, et al. Cytokine and chemokine levels in patients with sever fever with thrombocytopenia syndrome virs. PLoS One. 2012; 7(7): e41365.
25. Zupan J, et al. J. Biomed. Sci. The relationship between osteoclastogenic and anti-osteoclastogenic pro-inflammatory cytokines differs in human osteoporotic and osteoarthritic bone tissues. J. Biomed. Sci. 2012 Mar 1; 19: 28.
26. O'Donoghue RJ, et al. Genetic partitioning of interleukin-6 signaling in mice dissociates Stat3 from Smad3-mediated lung fibrosis. EMBO Mol. Med. 2012 Sep; 4(9): 939-51.
27. Goral V, et al. The relation between pathogenesis of liver cirrhosis, hepatic encephalopathy and serum cytokine levels: what is the role of tumor necrosis factor a? Hepatogastoenterology. 2011 May-Jun; 58(107-108): 943-8.
28. Smith PD, et al. The evolution of chemokine release supports a bimodal mechanism of spinal cord ischemia and reperfusion injury. Circulation. 2012 Sep 11; 126 (11 Suppl 1): S110-7.
29. Tinkle SS, et al. Beryllium-stimulated release of tumor necrosis factor-alpha, interleukin-6, and their soluble receptors in chronic beryllium disease. J. Respir. Crit. Care Med. 1997 Dec; 156(6): 1884-91.
30. Aoun E, et al. Diagnostic accuracy of interleukin-6 and interleukin-8 in predicting severe acute pancreatitis: a meta-analysis. Pancreatolody. 2009 Jan; 9(6): 777-85.
31. Bliss SK, et al. IL-10 prevents liver necrosis during murine infection with Trichinella spiralis. J. Immunol. 2003; 171: 3142-3147.
32. Weijer S, et al. Endogenous inerleukin-12 improves the early antimicrobial host response to murine Escherichia coli peritonitis. Shock. 2005; 23: 54-8.
33. Middleton MK, et al. 12/15-lipoxygenase-dependent myeloid production of interleukin-12 is essential for resistance to chronic toxoplasmosis. Infect. Immunol. 2009; 77: 5690-700.
34. Ying X, et al. Association of interleukin-13 SNP rs1800925 with allergic rhinitis risk: a meta-analysis based on 1,411 cases and 3169 controls. Gene. 2012 Sep; 506(1): 179-83.
35. Walczak A, et al. The IL-8 and IL-13 gene polymorphisms in inflammatory bowel disease and colorectal cancer. DNA Cell Biol. 2012 Aug; 31(8); 1431-8.
36. Hamishehkar H, et al. Pro-inflammatory cytokine profile of critically ill septic patients following therapeutic plasma exchange. Transfs. Apher. Sci. 2012 Sep 11; S1473-0502(12)00205-4.
37. McClellan JL, et al. Intestinal inflammatory response in relation to turmorigenesis in the Apc(Min/+) mouse. Cytokine. 2012; 57: 113-9.
38. Lane BR, et al. TNF-alpha inhibits HIV-1 replication in peripheral blood monocytes and alveolar macrophages by inducing the production of RANTES and decreasing C-C chemokine receptor 5 (CCR5) expression. J. Immunol. 1999; 163: 3653-61.
39. Bowen RA, et al. Impact of blood collection devices on clinical chemistry assays. Clin. Biochem. 2010 Jan; 43(1-2): 4-25.
40. Zhou H, et al. Collection, storage, preservation, and normalization of human urinary exosomes for biomarker discovery. Kidney. 2006; 69: 1471-76.
41. Thomas CE, et al. Urine collection and processing for protein biomarker discovery and quantification. Cancer Epidemiol Biomarkers & Prevention. 2010; 19: 953-59.
42. Schoonenboom NS, et al. Effects of processing and storage conditions on amyloid beta (1-42) and tau concentrations in cerebrospinal fluid: implications for use in clinical practice. Clin Chem. 2005; 51: 189-95.
43. Girgrah N, et al. Purification and characterization of the P-80 glycoprotein from human brain. Biochem J. 1988; 256: 351-56.

The present invention is further defined by the following items:
1. A kit for the analysis of a cytokine panel comprising
   (a) a multi-well assay plate selected from:
      (i) a multi-well assay plate comprising a plurality of wells, each well comprising ten discrete binding domains to which capture antibodies to the human analytes are bound: IFN-gamma, IL-1beta, IL-2, IL-4, IL-6, IL-8, IL-10, IL-12p70, IL-13, TNFalpha;
      (ii) a multi-well assay plate comprising a plurality of wells, each well comprising ten discrete binding domains to which capture antibodies to the following human analytes are bound: GM-CSF, IL-1alpha, IL-5, IL-7, IL-12/IL-23 p40, IL-15, IL-16, IL-17A, TNF-beta, VEGF-A;
      (iii) a multi-well assay plate comprising a plurality of wells, each well comprising ten discrete binding domains to which capture antibodies to the following human analytes are bound: Eotaxin, MIP-1 alpha, Eotaxin-3, TARC, IP-10, MIP-1 beta, IL-8, MCP-1, MDC, MCP-4;
      (iv) a multi-well assay plate comprising a plurality of wells, each well comprising ten discrete binding domains to which capture antibodies to the following rat analytes are bound: IFN-gamma, IL-2, IL-4, IL-1 beta, IL-5, IL-6, KC/GRO, IL-10, IL-13, TNF-alpha; or
      (v) a multi-well assay plate comprising a plurality of wells, each well comprising ten discrete binding domains to which capture antibodies to the following mouse analytes are bound: IFN-gamma, IL-1-beta, IL-2, IL-4, IL-5, IL-6, KC/GRO, IL-10, IL-12p70, TNF-alpha;
   (b) in one or more vials, containers, or compartments, a set of labeled detection antibodies specific for said human analytes; and
   (c) in one or more vials, containers, or compartments, a set of calibrator proteins.
2. The kit of item 1 wherein said kit further comprises one or more diluents.
3. The kit of item 1 wherein said detection antibodies are labeled with an electrochemiluminescent (ECL) label.
4. The kit of item 3 wherein said kit further comprises an ECL read buffer.
5. The kit of item 3 wherein said discrete binding domains are positioned on an electrode within said well.
6. The kit of item 1 wherein said set of calibrator proteins comprise a lyophilized blend of proteins.
7. The kit of item 1 wherein said set of calibrator proteins comprise a liquid formulation of calibrator proteins.
8. A 10-spot 96-well multi-well plate, wherein each plate comprises a plate top, a plate bottom, and each well comprises a spot pattern, wherein the plate meets the following specifications: (a) a length range of 3.8904-3.9004 inches; (b) a width range of 2.4736-2.4836 inches; and (c) well to well spacing of 0.3513-0.3573 inches.
9. A lot of plates of item 8.
10. A kit comprising a plate of item 8, wherein said spot pattern comprises ten discrete binding domains to which capture antibodies to one of the following sets of analytes are bound:
   (a) human analytes: IFN-gamma, IL-1beta, IL-2, IL-4, IL-6, IL-8, IL-10, IL-12p70, IL-13, TNFalpha;
   (b) human analytes: GM-CSF, IL-1alpha, IL-5, IL-7, IL-12/IL-23 p40, IL-15, IL-16, IL-17A, TNF-beta, VEGF-A;
   (c) human analytes: Eotaxin, MIP-1 alpha, Eotaxin-3, TARC, IP-10, MIP-1 beta, IL-8, MCP-1, MDC, MCP-4;
   (d) rat analytes: IFN-gamma, IL-2, IL-4, IL-1 beta, IL-5, IL-6, KC/GRO, IL-10, IL-13, TNF-alpha;
   (e) mouse analytes: IFN-gamma, IL-1-beta, IL-2, IL-4, IL-5, IL-6, KC/GRO, IL-10, IL-12p70, TNF-alpha;
   said kit further comprising (i) in one or more vials, containers, or compartments, a set of labeled detection antibodies specific for said human analytes; and (ii) in one or more vials, containers, or compartments, a set of calibrator proteins.
11. A plate of item 8, wherein said plate exceeds said specifications.
12. A plate of item 8, wherein (a) and (b) are measured from a center of a spot pattern in a first well, A1, to a center of a spot pattern of an outermost well, A12, of said plate.
13. A 10-spot 96-well multi-well plate, wherein each plate comprises a plate top, a plate bottom, an x- and y-axis of the plate top and bottom, and each well comprises a spot pattern, wherein the plate meets the following specifications: Δx ≤ 0.2 mm, Δy ≤ 0.2 mm, and α ≤ 0.1°, wherein (a) Δx is the difference between a center of the spot pattern and a center of a well along the x axis of the plate; (b) Δy is the difference between the center of a spot pattern and a center of the well along the y axis of the plate; and (c) α is a counter-clockwise angle between the x axis of the plate bottom and the x axis of the plate top.
14. A lot of plates of item 13.
15. A kit comprising a plate of item 13, wherein said spot pattern comprises ten discrete binding domains to which capture antibodies to one of the following sets of analytes are bound:
   (a) human analytes: IFN-gamma, IL-1beta, IL-2, IL-4, IL-6, IL-8, IL-10, IL-12p70, IL-13, TNFalpha;
   (b) human analytes: GM-CSF, IL-1alpha, IL-5, IL-7, IL-12/IL-23 p40, IL-15, IL-16, IL-17A, TNF-beta, VEGF-A;
   (c) human analytes: Eotaxin, MIP-1 alpha, Eotaxin-3, TARC, IP-10, MIP-1 beta, IL-8, MCP-1, MDC, MCP-4;
   (d) rat analytes: IFN-gamma, IL-2, IL-4, IL-1 beta, IL-5, IL-6, KC/GRO, IL-10, IL-13, TNF-alpha;
   (e) mouse analytes: IFN-gamma, IL-1-beta, IL-2, IL-4, IL-5, IL-6, KC/GRO, IL-10, IL-12p70, TNF-alpha;
   said kit further comprising (i) in one or more vials, containers, or compartments, a set of labeled detection antibodies specific for said human analytes; and (ii) in one or more vials, containers, or compartments, a set of calibrator proteins.
16. A plate of item 13, wherein said plate exceeds said specifications.
17. A kit for the analysis of two or more cytokine panels comprising:
   (a) two or more multi-well assay plates each comprising a plurality of wells, each well comprising ten discrete binding domains to which capture antibodies to a set of analytes are bound, wherein said set of analytes is selected from the group consisting of:
      (i) human analytes: IFN-gamma, IL-1beta, IL-2, IL-4, IL-6, IL-8, IL-10, IL-12p70, IL-13, and TNFalpha;
      (ii) human analytes: GM-CSF, IL-1alpha, IL-5, IL-7, IL-12/IL-23 p40, IL-15, IL-16, IL-17A, TNF-beta, and VEGF-A;
      (iii) human analytes: Eotaxin, MIP-1 alpha, Eotaxin-3, TARC, IP-10, MIP-1 beta, IL-8, MCP-1, MDC, and MCP-4;
      (iv) rat analytes: IFN-gamma, IL-2, IL-4, IL-1 beta, IL-5, IL-6, KC/GRO, IL-10, IL-13, and TNF-alpha; or
      (v) mouse analytes: IFN-gamma, IL-1-beta, IL-2, IL-4, IL-5, IL-6, KC/GRO, IL-10, IL-12p70, and TNF-alpha;
   (b) in one or more vials, containers, or compartments, a set of labeled detection antibodies specific for said analytes; and
   (c) in one or more vials, containers, or compartments, a set of calibrator proteins.
18. The kit of item 17 wherein said detection antibodies are labeled with an electrochemiluminescent (ECL) label.
19. The kit of item 18 wherein said discrete binding domains are positioned on an electrode within said well.
20. The kit of item 18 wherein said capture antibodies and/or detection antibodies have been subjected to an analytical testing method selected from the group consisting of CIEF, SEC-MALS, DLS, denaturing/non-denaturing gels, and Experion.
21. A method of manufacturing a lot of kits used in the analysis of a cytokine panel, wherein said kit comprises qualified detection and capture antibodies specific for one of the following sets of analytes:
   (i) human analytes; IFN-gamma, IL-1beta, IL-2, IL-4, IL-6, IL-8, IL-10, IL-12p70, IL-13, and TNFalpha;
   (ii) human analytes: GM-CSF, IL-1alpha, IL-5, IL-7, IL-12/IL-23 p40, IL-15, IL-16, IL-17A, TNF-beta, and VEGF-A;
   (iii) human analytes: Eotaxin, MIP-1 alpha, Eotaxin-3, TARC, IP-10, MIP-1 beta, IL-8, MCP-1, MDC, and MCP-4;
   (iv) rat analytes: IFN-gamma, IL-2, IL-4, IL-1 beta, IL-5, IL-6, KC/GRO, IL-10, IL-13, and TNF-alpha; or
   (v) mouse analytes: IFN-gamma, IL-1-beta, IL-2, IL-4, IL-5, IL-6, KC/GRO, IL-10, IL-12p70, and TNF-alpha;
   said method comprising the steps of subjecting a subset of kits in said lot to plate coating uniformity testing and passing said lot based on results of said uniformity testing.
22. The method of item 21 wherein said lot meets a specification selected from the group consisting of: (a) average intraplate CV of ≤ 10%; (b) maximum intraplate CV of ≤ 13%; (c) average uniformity metric of ≤ 25%; (d) maximum uniformity metric of ≤ 37%; (e) CV of intraplate averages of ≤ 18%; (f) lower signal boundary of > 1500; and (g) upper signal boundary of < 10⁶.
23. The method of item 21 wherein said lot meets the following specifications: (a) average intraplate CV of ≤ 10%; (b) maximum intraplate CV of ≤ 13%; (c) average uniformity metric of ≤ 25%; (d) maximum uniformity metric of ≤ 37%; (e) CV of intraplate averages of ≤ 18%; (f) lower signal boundary of > 1500; and (g) upper signal boundary of < 10⁶.
24. A method of manufacturing a kit used in the analysis of a cytokine panel, wherein said kit comprises qualified detection and capture antibodies specific for one of the following sets of analytes:
   (i) human analytes: IFN-gamma, IL-1beta, IL-2, IL-4, IL-6, IL-8, IL-10, IL-12p70, IL-13, and TNFalpha;
   (ii) human analytes: GM-CSF, IL-1alpha, IL-5, IL-7, IL-12/IL-23 p40, IL-15, IL-16, IL-17A, TNF-beta, and VEGF-A;
   (iii) human analytes: Eotaxin, MIP-1 alpha, Eotaxin-3, TARC, IP-10, MIP-1 beta, IL-8, MCP-1, MDC, and MCP-4;
   (iv) rat analytes: IFN-gamma, IL-2, IL-4, IL-1 beta, IL-5, IL-6, KC/GRO, IL-10, IL-13, and TNF-alpha; or
   (v) mouse analytes: IFN-gamma, IL-1-beta, IL-2, IL-4, IL-5, IL-6, KC/GRO, IL-10, IL-12p70, and TNF-alpha;
   said method comprising the steps of:
   (a) subjecting a preliminary set of detection antibodies specific for said mouse analytes to CIEF, DLS, and Experion;
   (b) selecting qualified detection antibodies from said preliminary set of detection antibodies based on said CIEF, DLS, and Experion testing;
   (c) subjecting a preliminary set of capture antibodies specific for said mouse analytes to CIEF, DLS, and Experion; and
   (b) selecting qualified capture antibodies from said preliminary set of capture antibodies based on said CIEF, DLS, and Experion testing.
25. The method of item 24, wherein said method further comprises subjecting said preliminary set of detection antibodies to an additional analytical method selected from the group consisting of denaturing SDS-PAGE, non-denaturing SDS-PAGE, SEC-MALS, and combinations thereof.
26. The method of item 24, wherein said method further comprises subjecting said preliminary set of detection antibodies to an additional analytical method consisting of denaturing SDS-PAGE, non-denaturing SDS-PAGE, and SEC-MALS.
27. The method of item 24, wherein said method further comprises subjecting said preliminary set of capture antibodies to an additional analytical method selected from the group consisting of denaturing SDS-PAGE, non-denaturing SDS-PAGE, SEC-MALS, and combinations thereof.
28. The method of item 24, wherein said method further comprises subjecting said preliminary set of capture antibodies to an additional analytical method consisting of denaturing SDS-PAGE, non-denaturing SDS-PAGE, and SEC-MALS.
29. The method of item 24 wherein said method further comprises subjecting each of said preliminary set of detection and capture antibodies to an additional analytical method consisting of denaturing SDS-PAGE, non-denaturing SDS-PAGE, and SEC-MALS.

## Claims

1. A lot of kits for the analysis of a cytokine panel, each of the kits comprising:
(a) a multi-well assay plate comprising:
a plurality of wells, each well comprising ten discrete binding domains to which capture antibodies to the human analytes are bound: IFN-gamma, IL-1beta, IL-2, IL-4, IL-6, IL-8, IL-10, IL-12p70, IL-13, TNFalpha; and
(b) in one or more vials, containers, or compartments, a set of labeled detection antibodies specific for the human analytes,
wherein a subset of plates of the lot meets the following specifications: (a) average intraplate Coefficient of Variability (CV) of ≤ 10%; (b) maximum intraplate CV of ≤ 13%; (c) average uniformity metric of ≤ 25%; (d) maximum uniformity metric of ≤ 37%; (e) CV of intraplate averages of ≤ 18%; (f) lower signal boundary of ≥ 1500; and (g) upper signal boundary of ≤ 10⁶.

2. The lot of kits of claim 1, wherein each labeled detection antibody in the set of labeled detection antibodies is labeled with an electrochemiluminescent (ECL) label.

3. The lot of kits of claims 1 or 2, wherein the kit further comprises an ECL read buffer.

4. The lot of kits of any of claims 1-3, wherein the discrete binding domains are positioned on an electrode within the well.

5. The lot of kits of any of claims 1-4, wherein the kit further comprises in one or more vials, containers, or compartments, a set of calibrator proteins.

6. The lot of kits of any of claims 1-5, wherein the set of calibrator proteins comprise a lyophilized blend of proteins.

7. The lot of kits of any of claims 1-6, wherein the multi-well assay plate is a 10-spot 96-well multi-well plate, wherein each plate comprises a plate top, a plate bottom, and each well comprises a spot pattern, wherein the plate meets the following specifications: (a) a length range of 3.8904-3.9004 inches; (b) a width range of 2.4736-2.4836 inches; and (c) well to well spacing of 0.3513-0.3573 inches.

8. The lot of kits of any of claims 1-7, wherein the multi-well assay plate is a 10-spot 96-well multi-well plate, wherein each plate comprises a plate top, a plate bottom, an x- and y-axis of the plate top and bottom, and each well comprises a spot pattern, wherein the plate meets the following specifications: Δx < 0.2 mm, Δy < 0.2 mm, and α < 0.1°, wherein (a) Δx is the difference between a center of the spot pattern and a center of a well along the x axis of the plate; (b) Δy is the difference between the center of a spot pattern and a center of the well along the y axis of the plate; and (c) α is a counter-clockwise angle between the x axis of the plate bottom and the x axis of the plate top.

9. A method of manufacturing a lot of kits used in the analysis of a cytokine panel, wherein each kit of the lot comprises qualified detection and capture antibodies specific for the following human analytes: IFN-gamma, IL-1beta, IL-2, IL-4, IL-6, IL-8, IL-10, IL-12p70, IL-13, and TNFalpha,
wherein each kit comprises a multi-well assay plate and each well of the plate comprises ten discrete binding domains to which capture antibodies to the analytes are bound;
the method comprising subjecting a subset of kits in the lot to plate coating uniformity testing and passing the lot based on results of the uniformity testing, and
wherein the subset meets the following specifications: (a) average intraplate CV of ≤ 10%; (b) maximum intraplate CV of ≤ 13%; (c) average uniformity metric of ≤ 25%; (d) maximum uniformity metric of ≤ 37%; (e) CV of intraplate averages of ≤ 18%; (f) lower signal boundary of ≥ 1500; and (g) upper signal boundary of ≤ 10⁶.

10. The lot of any of claims 1-8 or the method of claim 9, wherein the lower limit of a detection range for each of the analytes is 0.10-0.37 pg/mL for IFN-gamma, 0.02-0.08 pg/mL for IL-1beta, 0.07-0.13 pg/mL for IL-2, 0.02-0.04 pg/mL for IL-4, 0.46-1.0 pg/mL for IL-6, 0.01-0.01 pg/mL for IL-8, 0.02-0.05 pg/mL for IL-10, 0.09-0.19 pg/mL for IL-12p70, 0.21-0.43 pg/mL for IL-13, and 0.05-0.10 pg/mL for TNFalpha.

11. The method of claim 9, wherein each antibody in the set of detection antibodies is labeled with an electrochemiluminescent (ECL) label.

12. The method of claim 9 or 11, wherein the kit further comprises an ECL read buffer.

13. The method of any of claims 9 and 11-12, wherein the discrete binding domains are positioned on an electrode within a well.

14. The method of any of claims 9 and 11-13, further comprising a set of calibrator proteins, which comprise a lyophilized blend of proteins.

15. The method of any of claims 9 and 11-14, wherein the plate is a 10-spot 96-well multi-well plate, wherein each plate comprises a plate top, a plate bottom, and each well comprises a spot pattern, wherein the plate meets the following specifications: (a) a length range of 3.8904-3.9004 inches; (b) a width range of 2.4736-2.4836 inches; and (c) well to well spacing of 0.3513-0.3573 inches.

16. The method of any of claims 9 and 11-15, wherein the plate is a 10-spot 96-well multi-well plate, wherein each plate comprises a plate top, a plate bottom, an x- and y-axis of the plate top and bottom, and each well comprises a spot pattern, wherein the plate meets the following specifications: Δx ≤ 0.2 mm, Δy ≤ 0.2 mm, and α ≤ 0.1°, wherein (a) Δx is the difference between a center of the spot pattern and a center of a well along the x axis of the plate; (b) Δy is the difference between the center of a spot pattern and a center of the well along the y axis of the plate; and (c) α is a counter-clockwise angle between the x axis of the plate bottom and the x axis of the plate top.

17. The lot of kits of claim 1, wherein the subset of plates further meets a precision specification of a concentration CV of less than 20% for controls on both intra- and inter-day runs.

18. The method of claim 9, wherein the subset of plates further meets a precision specification of a concentration CV of less than 20% for controls on both intra- and inter-day runs.

19. The method of claim 9, further comprising:
(a) subjecting a preliminary set of detection antibodies specific for the analytes to CIEF, DLS, and electrophoresis testing;
(b) selecting qualified detection antibodies from the preliminary set of detection antibodies based on the CIEF, DLS, and electrophoresis testing;
(c) subjecting a preliminary set of capture antibodies specific for the analytes to CIEF, DLS, and electrophoresis testing; and
(b) selecting qualified capture antibodies from the preliminary set of capture antibodies based on the CIEF, DLS, and automated electrophoresis testing

20. The method of claim 19, further comprising subjecting the preliminary set of detection antibodies to an additional analytical method selected from the group consisting of denaturing SDS-PAGE, non-denaturing SDS-PAGE, SEC-MALS, and combinations thereof.
